(19) Europäisches Patentamt
European Patent Office
Office européen des brevets

(11) EP 0 894 089 B1

(12) EUROPEAN PATENT SPECIFICATION

(45) Date of publication and mention of the grant of the patent:
**23.10.2002 Bulletin 2002/43**

(21) Application number: **97922610.7**

(22) Date of filing: **02.05.1997**

(51) Int Cl.[7]: **C07D 417/14**, A61K 31/425, C07D 417/12 // (C07D417/14, 277:00, 311:00, 207:00)

(86) International application number:
**PCT/US97/07416**

(87) International publication number:
**WO 97/041121 (06.11.1997 Gazette 1997/47)**

# (54) NOVEL HETEROCYCLIC COMPOUNDS HAVING ANTIDIABETIC, HYPOLIPIDAEMIC, ANTIHYPERTENSIVE PROPERTIES, PROCESS FOR THEIR PREPARATION AND PHARMACEUTICAL COMPOSITIONS CONTAINING THEM

NEUE HETEROZYKLISCHE VERBINDUNGEN MIT ANTIDIABETISCHEN, HYPOLIPIDDAEMISCHEN UND BLUTDRUCKSENKENDEN EIGENSCHAFTEN, VERFAHREN ZU IHRER HERSTELLUNG UND PHARMAZEUTISCHE ZUSAMMENSETZUNGEN DIE DIESE ENTHALTEN

NOUVEAUX COMPOSES HETEROCYCLIQUES PRESENTANT DES PROPRIETES ANTIDIABETIQUES, HYPOLIPIDEMIANTES, ANTIHYPERTENSIVES, LEUR PROCEDE DE PREPARATION ET COMPOSITIONS PHARMACEUTIQUES LES CONTENANT

(84) Designated Contracting States:
**CH DE FR GB LI SE**

(43) Date of publication of application:
**03.02.1999 Bulletin 1999/05**

(73) Proprietor: **Dr. Reddy's Laboratories Ltd.
Hyderabad 500 016 (IN)**

(72) Inventors:
- **LOHRAY, Vidya, Bhushan
Hyderabad-500033, Andhra Pradesh (IN)**
- **LOHRAY, Braj, Bhushan
Hyderabad-500033, Andhra Pradesh (IN)**
- **BAJJI, Ashok, Channaveerappa
Hyderabad-500050, Andhra Pradesh (IN)**
- **ALLA, Sekar, Reddy
Hyderabad-500050, Andhra Pradesh (IN)**
- **RAMANUJAM, Rajagopalan, 28, Vishaal Towers
Hyderabad-500873, Andhra Pradesh (IN)**
- **CHAKRABARTI, Ranjan
Hyderabad-500038, Andhra Pradeash (IN)**

(74) Representative:
**Ebner von Eschenbach, Jennifer et al
Ladas & Parry,
Dachauerstrasse 37
80335 München (DE)**

(56) References cited:
**EP-A- 0 528 734**
**EP-A- 0 645 387**
**EP-A- 0 676 398**
**EP-A- 0 745 600**
**US-A- 5 037 842**

- **ARZNEIMITTEL FORSCHUNG/DRUG DESIGN, vol. 45, no. II, 1995, pages 1176-1181, XP002037304 G. DE NANTEUIL ET AL.: "Euglygaemic and Biological Activities of Novel Thiazolidine-2,4-dione Derivatives"**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 894 089 B1

## Description

The present invention relates to novel antidiabetic compounds, their tautomeric forms, their derivatives, their stereoisomers, their polymorphs, their pharmaceutically acceptable salts, their pharmaceutically acceptable solvates and pharmaceutically acceptable compositions containing them. This invention particularly relates to novel azolidinedione derivatives of the general formula (I), their tautomeric forms, their stereoisomers, their polymorphs and their pharmaceutically acceptable salts, pharmaceutically acceptable solvates and pharmaceutical compositions containing them.

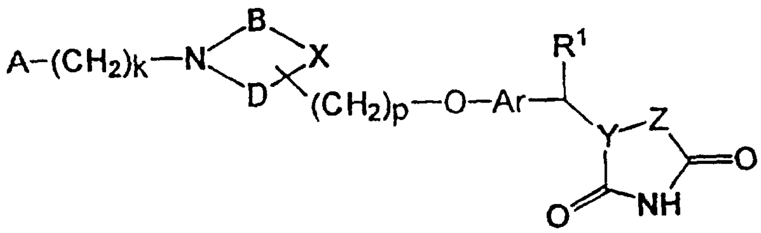

(I)

The present invention also relates to a process for the preparation of the above said novel, azolidinedione derivatives, their tautomeric forms, their stereoisomers, their polymorphs, their pharmaceutically acceptable salts, pharma-ceutically acceptable solvates, novel intermediates and pharmaceutical compositions containing them.

The azolidinedione derivatives of the general formula (I) defined above of the present invention are useful for the treatment and / or prophylaxis of diseases or conditions in which insulin resistance is the underlying pathophysiological mechanism. Examples of these diseases and conditions are type II diabetes, impaired glucose tolerance, dyslipidaemia, hypertension, coronary heart disease and other cardiovascular disorders including atherosclerosis. The azolidinedione derivatives of the formula (I) are useful for the treatment of insulin resistance associated with obesity and psoriasis. The azolidinedione derivatives of the formula (I) can also be used to treat diabetic complications and can be used for treatment and / or prophylaxis of other diseases and conditions such as polycystic ovarian syndrome (PCOS), certain renal diseases including diabetic nephropathy, glomerulonephritis, glomerular sclerosis, nephrotic syndrome, hypertensive nephrosclerosis, end-stage renal diseases and microalbuminuria as well as certain eating disorders, as aldose reductase inhibitors and for improving cognitive functions in dementia.

## BACKGROUND OF THE INVENTION

Insulin resistance is the diminished ability of insulin to exert its biological action across a broad range of concentrations. In insulin resistance, the body secretes abnormally high amounts of insulin to compensate for this defect; failing which, the plasma glucose concentration inevitably rises and develops into diabetes. Among the developed countries, diabetes mellitus is a common problem and is associated with a variety of abnormalities including obesity, hypertension, hyperlipidemia (J. Clin. Invest., (1985) 75: 809-817; N. Engl. J. Med. (1987) 317: 350-357; J. Clin. Endocrinol. Metab., (1988) 66: 580-583; J. Clin. Invest., (1975) 68: 957-969) and other renal complications (See Patent Application No. WO 95/21608). It is now increasingly being recognized that insulin resistance and relative hyperinsulinemia have a contributory role in obesity, hypertension, atherosclerosis and type 2 diabetes mellitus. The association of insulin resistance with obesity, hypertension and angina has been described as a syndrome having insulin resistance as the central pathogenic link-Syndrome-X. In addition, polycystic ovarian syndrome (Patent Application No. WO 95/07697), psoriasis (Patent Application No. WO 95/35108), dementia (Behavioral Brain Research (1996) 75: 1-11) etc. may also have insulin resistance as a central pathogenic feature.

A number of molecular defects have been associated with insulin resistance. These include reduced expression of insulin receptors on the plasma membrane of insulin responsive cells and alterations in the signal transduction pathways that become activated after insulin binds to its receptor including glucose transport and glycogen synthesis.

Since defective insulin action is thought to be more important than failure of insulin secretion in the development of non-insulin dependent diabetes mellitus and other related complications, this raises doubts about the intrinsic suitability of antidiabetic treatment that is based entirely upon stimulation of insulin release. Recently, Takeda has developed a new class of compounds which are the derivatives of 5-(4-alkoxybenzyl)-2,4-thiazolidinediones of the formula (II) (Ref. *Chem. Pharm. Bull.* 1982, 30, 3580-3600). In the formula (II), V represents substituted or unsubstituted divalent aromatic group and U represents various groups which have been reported in various patent documents.

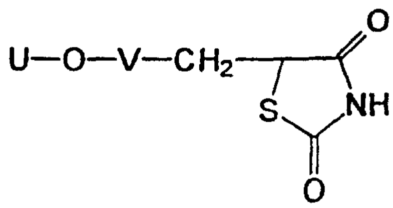

(II)

By way of examples, U may represent the following groups:

(i) a group of the formula (IIa) where $R^1$ is hydrogen or hydrocarbon residue or heterocyclic residue which may each be substituted, $R^2$ is hydrogen or a lower alkyl which may be substituted by hydroxy group, X is an oxygen or sulphur atom, Z is a hydroxylated methylene or a carbonyl, m is 0 or 1, n is an integer of 1-3. These compounds have been disclosed in the European Patent Application No. 0 177 353

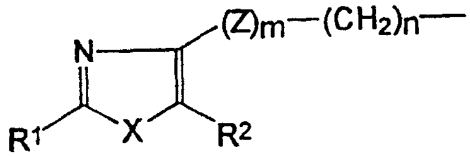

(IIa)

An example of these compounds is shown in formula (IIb)

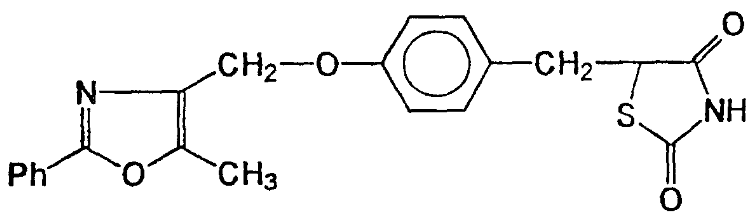

(IIb)

(ii) a group of the formula (IIc) wherein $R^1$ and $R^2$ are the same or different and each represents hydrogen or $C_1$-$C_5$ alkyl, $R^3$ represents hydrogen, acyl group, a ($C_1$-$C_6$) alkoxycarbonyl group or aralkyloxycarbonyl group, $R^4$- $R^5$ are same or different and each represent hydrogen, $C_1$-$C_5$ alkyl or $C_1$-$C_5$ alkoxy or $R^4$, $R^5$ together represent $C_1$-$C_4$ alkenedioxy group, n is 1, 2, or 3, W represents $CH_2$, CO, $CHOR^6$ group in which $R^6$ represents any one of the items or groups defined for $R^3$ and may be the same or different from $R^3$. These compounds are disclosed in the European Patent Application No. 0 139 421.

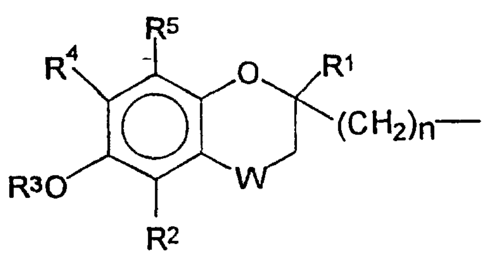

(IIc)

An example of these compounds is shown in (lid)

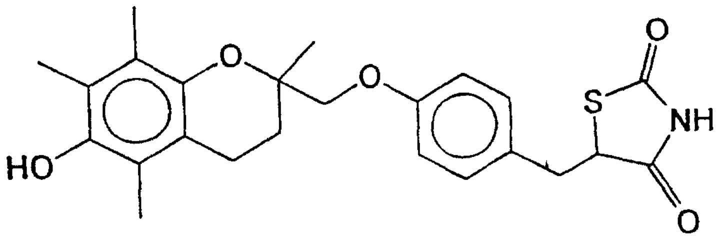 (IId)

iii) A group of formula (lie) where $A^1$ represents substituted or unsubstituted aromatic heterocyclic group, $R^1$ represents a hydrogen atom, alkyl group, acyl group, an aralkyl group wherein the aryl moiety may be substituted or unsubstituted, or a substituted or unsubstituted aryl group, n represents an integer in the range from 2 to 6. These compounds are disclosed in European Patent No. 0 306 228.

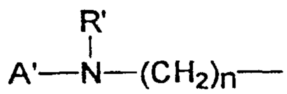 (IIe)

An example of this compound is shown in formula (IIf)

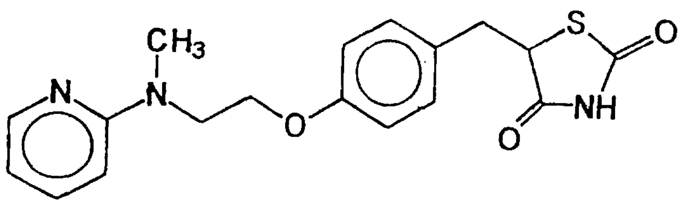 (IIf)

iv) A group of formula (IIg) where Y represents N or $CR^5$, $R^1$, $R^2$, $R^3$, $R^4$ and $R^5$ represents hydrogen, halogen, alkyl and the like and $R^6$ represents hydrogen, alkyl, aryl and the like, n represents an integer of 0 to 3. These compounds are disclosed in European Patent Application No. 0 604 983.

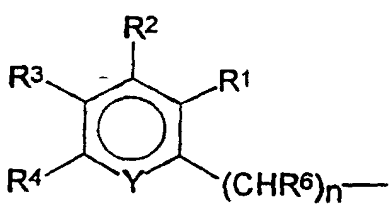 (IIg)

An example of this compound is shown in formula (IIh)

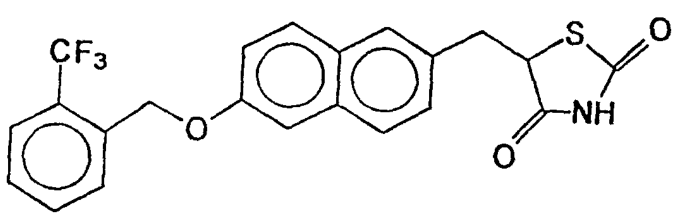 (IIh)

v) A group of formula (IIi a-d) where $R^1$ represents hydrogen atom, halogen, linear or branched $(C_1-C_6)$alkyl, $(C_1-C_6)$alkoxy, trifluoromethyl or cyano groups and X represents S, O or NR where R = H or $(C_1-C_6)$alkyl group. These compounds are disclosed in European Patent Application No. 0 528 734.

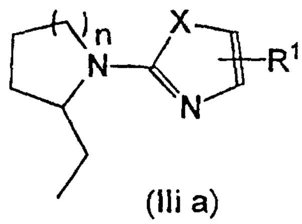

(IIi a)

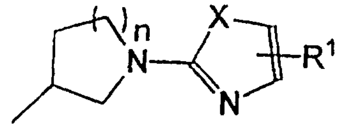

(IIi b)

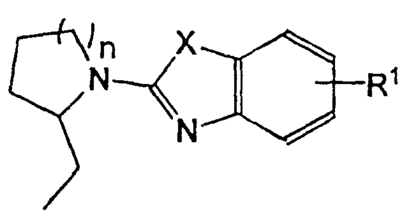

(IIi c)

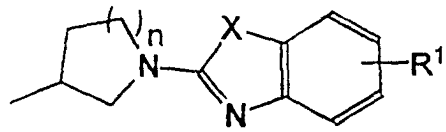

(IIi d)

An example of this class of compound is shown in formula (IIj)

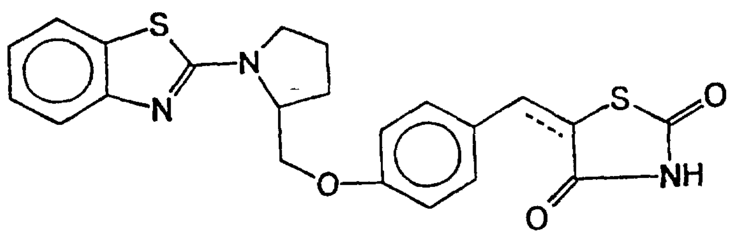

(IIj)

Still another class of antihyperglycemic agents are 5-substituted oxazolidine-2,4-diones and 2-substituted-1,2,4-oxadiazolidine-3,5-diones which can be represented in the formula (IIk),

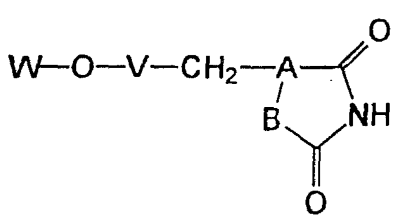

(IIk)

where V represents substituted or unsubstituted divalent aryl or hetero aryl group, W represents various groups which have been reported in various patent documents, A represents nitrogen atom or a CH group and B is an oxygen atom.

By way of examples, W may represent the following groups:

vi) a group of formula (II 1), where R is $(C_1-C_6)$ alkyl groups, cycloalkyl group, furyl, thienyl, substituted or unsubstituted phenyl group, X is hydrogen, methyl, methoxy, chloro or fluoro.

These compounds have been disclosed in the U.S. Patent No. 5 037 842.

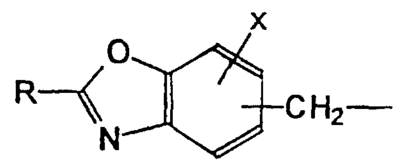

(II l)

An example of these compounds is shown in formula (IIm).

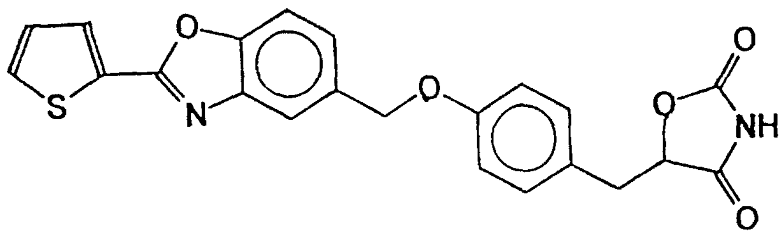

(IIm)

(vii) A group of formula (IIn) wherein $A^1$ represents a substituted or unsubstituted aromatic heterocyclyl group; $R^1$ represents a hydrogen atom, an alkyl group, an acyl group, an aralkyl group, wherein the aryl moiety may be substituted or unsubstituted or a substituted or unsubstituted aryl group, n represents an integer in the range of from 2 to 6. These compounds have been disclosed in the patent application No. WO 92/02520.

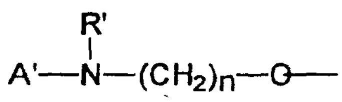

(IIn)

An example of these compounds is shown in formula (IIo)

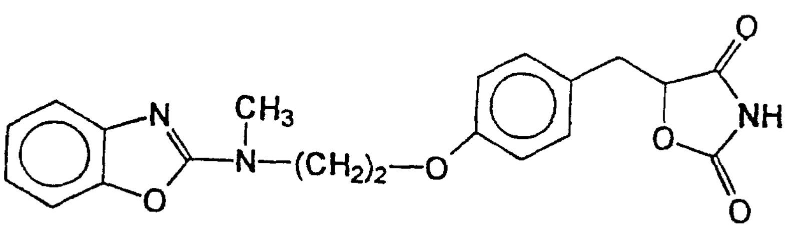

(IIo)

(viii) A group of formulae (IIp) and (IIq), where $R^1$ is hydrogen, $(C_1\text{-}C_8)$alkyl, $(C_1\text{-}C_8)$alkoxy, trifluoroalkoxy, halogen or trifluoromethyl group, $R^2$ is hydrogen or methyl and X is oxygen or sulfur. These compounds have been described in U.S. Patent No. 5 480 486.

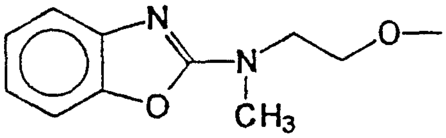

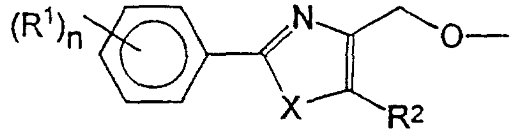

(IIp) (IIq)

An example of these compounds is shown in formula (IIr)

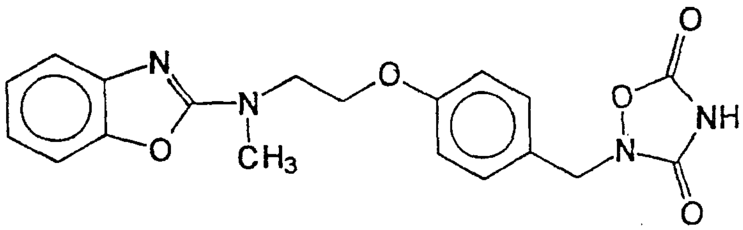

(IIr)

(ix) a group of formula (IIs)

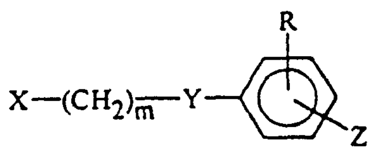

(IIs)

wherein:

X represents an indolyl, indolinyl, azaindolyl, azaindolinyl, imidazopyridyl or imidazopyrimidinyl group which is unsubstituted or is substituted;
Y represents an oxygen atom or a sulphur atom;
Z represents a group of formula (i), (ii), (iii), (iv) or (v):

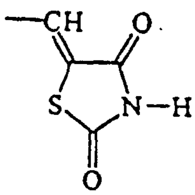

(i)

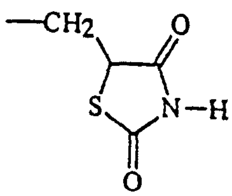

(ii)

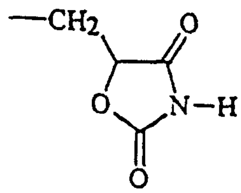

(iii)

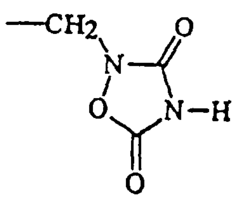

(iv)

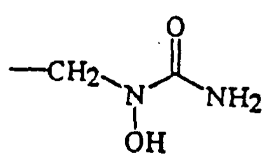

(v)

R represents a hydrogen atom, an alkyl group having from 1 to 4 carbon atoms, an alkoxy group having from 1 to 4 carbon atoms, a halogen atom, a hydroxy group, a nitro group, an aralkyl group in which an alkyl group having from 1 to 5 carbon atoms is substituted by an aryl group having from 6 to 10 ring carbon atoms, or a group of formula $-NR^aR^b$,

wherein $R^a$ and $R^b$ are the same or different and each represents a hydrogen atom, an alkyl group having from 1 to 8 carbon atoms, an aralkyl group in which an alkyl group having from 1 to 5 carbon atoms is substituted by an aryl group having from 6 to 10 ring carbon atoms, an aryl group having from 6 to 10 ring carbon atoms, an aliphatic carboxylic acyl group having from 1 to 11 carbon atoms, an aliphatic carboxylic acyl group which has from 2 to 6 carbon atoms and which is substituted by an aryl group having from 6 to 10 rings.carbon atoms, or an

aromatic carboxylic acyl group in which the aryl part has from 6 to 10 ring carbon atoms, m is an integer of from 1 to 5.

These compounds have been described in EP O 676 398A2.

(x) a group of formula (IIt)

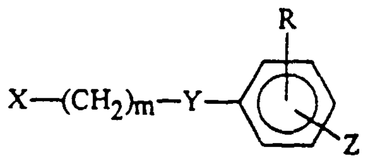

(IIt)

in which:

X represents a benzimidazole group which is unsubstituted or is substituted;
Y represents an oxygen atom or a sulphur atom;
Z represents a group of formula (i), (ii), (iii), (iv) or (v):

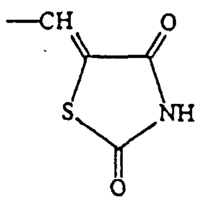

(i)

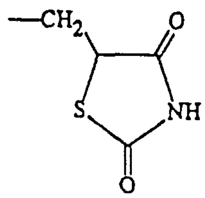

(ii)

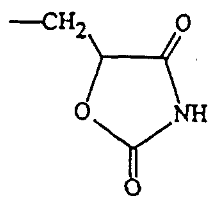

(iii)

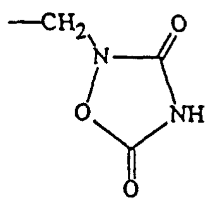

(iv)

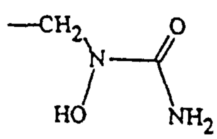

(v)

R represents:

a hydrogen atom;
an alkyl group having from 1 to 4 carbon atoms;
an alkoxy group having from 1 to 4 carbon atoms;
a halogen atom;
a hydroxy group;
a nitro group;
a group of formula $-NR^aR^b$,

in which $R^a$ and $R^b$ are the same or different and each represents a hydrogen atom, an alkyl group having from 1 to 8 carbon atoms, an aralkyl group in which an alkyl group having from 1 to 5 carbon atoms is substituted by a carbocyclic aryl group having from 6 to 10 carbon atoms: a carbocyclic aryl group having from 6 to 10 carbon atoms: an aliphatic acyl group having from 1 to 11 carbon atoms: an arylaliphatic acyl group in which an aliphatic acyl group having from 2 to 6 carbon atoms is substituted by at least one carbocyclic aryl group having from 6 to 10 carbon atoms: or an aromatic acyl group having from 7 to 11 carbon atoms: or an aralkyl group in which an alkyl group having from 1 to 5 carbon atoms is substituted by a carbocyclic aryl group having from 6 to 10 carbon atoms; and m represents an integer from 1 to 5.

These compounds have been described in EP O 745 600A1.

(xi) a group of formula (IIu)

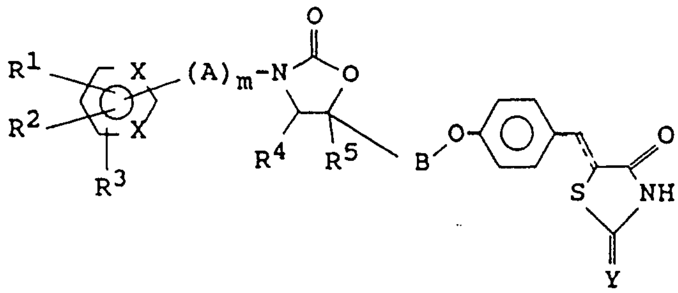

wherein $R^1$, $R^2$ and $R^3$ may be the same or different and individually represent a hydrogen atom, a halogen atom, a lower alkyl group or lower alkoxyl group which may be substituted by one or more halogen atom(s), a hydroxyl group, a nitro group, an amino group, a lower acylamino group, a mono- or di-lower alkylamino group, a carboxyl group, a lower alkoxycarbonyl group, a cyano group, a 2-oxazolyl group, a thiazolidine-2,4-dion-5-ylidene-methyl group or a thiazolidine-2,4-dion-5-ylmethyl group and $R^1$ and $R^2$ may be coupled together to form an alkylene chain $-(CH_2)_p-$ wherein p stands for 3, 4 or 5 or an alkylenedioxy chain $-O-(CH_2)_qO-$ wherein q stands for 1, 2 or 3, thereby forming a ring; $R^4$ and $R^5$ may be the same or different and individually represent a hydrogen atom or a lower alkyl group; X represents a carbon atom or nitrogen atom; Y represents an oxygen atom or an imino group; A and B individually represent a lower alkylene group; m stands for 0 or 1; and the dashed line indicates the presence or absence of a double bond; or a salt thereof.

These compounds have been described in EP O 645 387A1.

## SUMMARY OF THE INVENTION

With an objective of developing new compounds for the treatment of type II diabetes [non-insulin-dependent-diabetes mellitus (NIDDM)] which could be more potent at relatively lower doses and having better efficacy with lower toxicity, we focused our research efforts in a direction of incorporating safety and to have better efficacy, which has resulted in the development of novel azolidinedione derivatives having the general formula (I) as defined above.

The main objective of the present invention is therefore, to provide novel azolidinedione derivatives, their tautomeric forms, their stereoisomers, their polymorphs, their pharmaceutically, acceptable salts, their pharmaceutically acceptable solvates and pharmaceutical compositions containing them, or their mixtures.

Another objective of the present invention is to provide novel azolidinedione derivatives, their tautomeric forms, their stereoisomers, their polymorphs, their pharmaceutically acceptable salts, their pharmaceutically acceptable solvates and pharmaceutical compositions containing them or their mixtures having enhanced activities, no toxic effect or reduced toxic effect.

Yet another objective of the present invention is to produce a process for the preparation of novel azolidinediones of the formula (I) as defined above, their tautomeric forms, their stereoisomers, their polymorphs, their pharmaceutically acceptable salts and their pharmaceutically acceptable solvates.

Still another objective of the present invention is to provide pharmaceutical compositions containing compounds of the general formula (I), their tautomers, their stereoisomers, their polymorphs, their salts, solvates or their mixtures in combination with suitable carriers, solvents, diluents and other media normally employed in preparing such compositions.

Yet another objective of the present invention is to provide a process for the preparation of the novel intermediate of the formula (III)

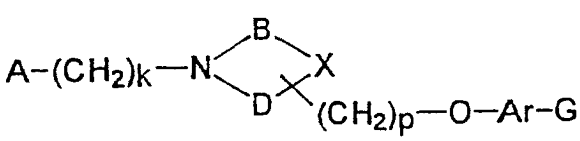

(III)

where G represents -CHO, $-NO_2$; $-NH_2$, -CH=NHOH, $-CH_2NHOH$, $-CH_2N(OH)CONH_2$ or $-CH_2CH(J)-COOR$, where J represents hydroxy group, or halogen atom such as chlorine, bromine or iodine and R represents H or lower alkyl group such as methyl, ethyl or propyl. A, B, D, X, k, p and Ar are as defined in formula (I).

## DETAILED DESCRIPTION OF THE INVENTION

Azolidinedione derivatives of the present invention have the general formula (I)

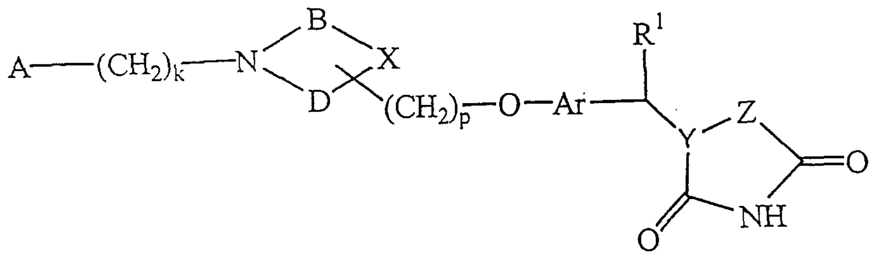

In the above formula (I), A represents dihydrobenzofuranyl, benzofuranyl , dihydrobenzopyranyl, or indolyl, wherein the group represented by A may be substituted by hydroxy, $(C_1-C_6)$alkyl or $(C_1-C_6)$alkoxy, which alkoxy group may be itself substituted by aryl; B represents a linking group and contains 1-4 carbon atoms, bonds; D represents a bond or D contains 1-4 carbons atoms, X represents either a $CH_2$ group, NH or sulfur; Ar represents phenylene, or naphthylene, $R^1$ represents hydrogen atom, carbon atoms or forms a bond together with the adjacent group Y. Y represents a nitrogen atom or a group $CR^2$ where or alkyl $R^2$ forms a bond together with $R^1$; Z represents an oxygen atom or a sulfur atom when Y is $CR^2$ and Z represents an oxygen atom when Y is a nitrogen atom; k is an integer ranging from 1-4-and p is an integer ranging from 0 to 1 When p is 0 (zero) $(CH_2)_P$ represents a bond.

Suitable linking group between N and X represented by B may contain 1-4 carbon atoms, 1-2 being preferred and suitable linking group between N and X represented by D may represent either a bond or contain 1-4 carbon atoms, 1-2 being preferred. The compounds according to formula (I) always have a linking group B and a linking group D. The linking group D having no carbon atom means that the linking group D represents a bond.

Suitable X includes $CH_2$, NH or S, preferably $CH_2$, or NH. Preferred ring structures comprise a nitrogen atom, linking groups represented by B and D, and X are pyrrolidinyl, piperidinyl, piperazinyl, aziridinyl and morpholinyl groups.

It is more preferred that the ring structure comprise a nitrogen atom, linking groups represented by B and D, and X and is a pyrrolidinyl group.

It is preferred that $R^1$ and $R^2$ represent hydrogen atom or $R^1$ and $R^2$ together represent a bond.

Suitable Z group includes a hetero atom selected from 0 or S, with the provision that when Y is $CR^2$, Z is selected from sulfur or oxygen, and when Y is nitrogen, Z represents oxygen.

Suitable ring structure comprising Y and Z include 2,4-dioxooxazolidin-5-yl; 2,4-dioxothiazolidin-5-yl; and 3,5-dioxo-1,2,4-oxadiazolidin-2-yl groups.
Preferred ring structures comprising Y and Z include 2,4-dioxooxazolidin-5-yl and 2,4-dioxothiazolidin-5-yl groups.

It is more preferred that the ring structure comprising Y and Z is a 2,4-dioxothiazolidin-5-yl group.

Suitable k is an integer ranging from 1 to 4, especially 1 to 2 and suitable p is an integer ranging from 0 to 4. It is preferred that p is 1 or 0. When p is zero $(CH_2)p$ represents a bond; the ring structure comprising N, X and the linking groups B and D is directly linked to the oxygen atom.

Pharmaceutically acceptable salts forming part of this invention include salts of the azolidinedione moiety such as alkali metal salts like Li, Na, and K salts, alkaline earth metal salts like Ca and Mg salts, salts of organic bases such as lysine, arginine, guanidine, diethanolamine, choline and the like, ammonium or substituted ammonium salts, salts of carboxy group wherever appropriate, such as aluminum, alkali metal salts, alkaline earth metal salts, ammonium or substituted ammonium salts. Salts may include acid addition salts which are, sulphates, nitrates, phosphates, perchlorates, borates, hydrohalides, acetates, tartrates, maleates, citrates, succinates, palmoates, methanesulphonates, benzoates, salicylates, hydroxynaphthoates, benzenesulfonates, ascorbates, glycerophosphates, ketoglutarates and the like. Pharmaceutically acceptable solvates may be hydrates or comprise other solvents of crystallization such as alcohols.

Particularly useful compounds according to the invention include:

5-[4-[N-(6-benzyloxy-2,5,7,8-tetramethylchroman-2-ylmethyl)-(2S)-pyrrolidine-2-methoxyl]phenyl methylene] thiazolidine-2,4-dione and its salts;

5-[4-[N-(6-benzyloxy-2,5,7,8-tetramethylchroman-2-ylmethyl)-(2R)-pyrrolidine-2-methoxyl]phenyl methylene] thiazolidine-2,4-dione, and its salts;
5-[4-[N-(6-benzyloxy-2,5,7,8-tetramethylchroman-2-ylmethyl)-(2S)-pyrrolidine-2-methoxy]phenyl methyl]thiazolidine-2,4-dione;
5-[4-[N-(6-benzyloxy-2,5,7,8-tetramethylchroman-2-ylmethyl)-(2R)-pyrrolidine-2-methoxy]phenyl methyl]thiazolidine-2,4-dione and its salts;
5-[4-[N-(6-benzyloxy-2,5,7,8-tetramethylchroman-2-ylmethyl)-(2S)-pyrrolidine-2-methoxy]phenyl methyl]thiazolidine-2,4-dione sodium salt;
5-[4-[N-(6-benzyloxy-2,5,7,8-tetramethylchroman-2-ylmethyl)-(2S)-pyrrolidine-2-methoxy]phenyl methyl]thiazolidine-2,4-dione maleic acid salt;
5-[4-[N-(6-benzyloxy-2,5,7,8-tetramethylchroman-2-ylmethyl)-(2S)-pyrrolidine-2-methoxy]phenyl methyl]thiazolidine-2,4-dione hydrochloride salt;
5-[4-[N-(6-hydroxy-2,5,7,8-tetramethylchroman-2-ylmethyl)-(2S)-pyrrolidine-2-methoxy]phenyl methyl]thiazolidine-2,4-dione;
5-[4-[N-(6-hydroxy-2,5,7,8-tetramethylchroman-2-ylmethyl)-(2R)-pyrrolidine-2-methoxy]phenyl methyl]thiazolidine-2,4-dione and its salts;
5-[4-[N-(6-hydroxy-2,5,7,8-tetramethylchroman-2-ylmethyl)-(2S)-pyrrolidine-2-methoxy]phenyl methyl]thiazolidine-2,4-dione sodium salt;
5-[4-[N-[6-hydroxy-2,5,7,8-tetramethylchroman-2-ylmethyl)-(2S)-pyrrolidine-2-methoxy]phenyl methyl]thiazolidine-2,4-dione maleic acid salt;
5-[4-[N-[6-hydroxy-2,5,7,8-tetramethylchroman-2-ylmethyl]-(2S)-pyrrolidine-2-methoxy]phenyl methyl]thiazolidine-2,4-dione hydrochloride salt;
5-[4-[N-[6-hydroxy-2,5,7,8-tetramethylchroman-2-ylmethyl]-(2S)-pyrrolidine-2-methoxy]phenyl methylene]thiazolidine-2,4-dione;
5-[4-[N-[6-hydroxy-2,5,7,8-tetramethylchroman-2-ylmethyl]-(2R)-pyrrolidine-2-methoxy]phenyl methylene]thiazolidine-2,4-dione and its salts;
5-[4-[N-[2,3-dihydro-5-benzyloxy-2,2,4,6,7-pentamethylbenzofuran-3-ylmethyl]-(2S)-pyrrolidine-2-methoxy]phenyl methylene]thiazolidine-2,4-dione;
5-[4-[N-[2,3 -dihydro-5-benzyloxy-2,2,4,6,7-pentamethylbenzofuran-3-ylmethyl]-(2R)-pyrrolidine-2-methoxy]phenyl methylene]thiazolidine-2,4-dione and its salts;
5-[4-[N-[2,3-dihydro-5-benzyloxy-2,2,4,6,7-pentamethylbenzofuran-3-ylmethyl]-(2S)-pyrrolidine-2-methoxy]phenyl methylene]thiazolidine-2,4-dione maleic acid salt;
5-[4-[N-[2,3-dihydro-5-benzyloxy-2,2,4,6,7-pentamethylbenzofuran-3-ylmethyl]-(2S)-pyrrolidine-2-methoxy]phenyl methylene]thiazolidine-2,4-dione hydrochloride salt;
5-[4-[N-[2,3-dihydro-5-benzyloxy-2,2,4,6,7-pentamethylbenzofuran-3-ylmethyl]-(2S)-pyrrolidine-2-methoxy] phenyl methyl]thiazolidine-2,4-dione;
5-[4-[N-[2,3-dihydro-5-benzyloxy-2,2,4,6,7-pentamethylbenzofuran-3-ylmethyl]-(2R)-pyrrolidine-2-methoxy] phenyl methyl]thiazolidine-2,4-dione and its salts;
5-[4-[N-[2,3-dihydro-5-benzyloxy-2,2,4,6,7-pentamethylbenzofuran-3-ylmethyl]-(2S)-pyrrolidine-2-methoxy]phenyl methyl]thiazolidine-2,4-dione maleic acid salt;
5-[4-[N-[2,3-dihydro-5-benzyloxy-2,2,4,6,7-pentamethylbenzofuran-3-ylmethyl]-(2S)-pyrrolidine-2-methoxy]phenyl methyl]thiazolidine-2,4-dione hydrochloride salt;
5-[4-[N-[2,3-dihydro-5-hydroxy-2,2,4,6,7-pentamethylbenzofuran-3-ylmethyl]-(2S)-pyrrolidine-2-methoxy]phenyl methyl]thiazolidine-2,4-dione;
5-[4-[N-[2,3-dihydro-5-hydroxy-2,2,4,6,7-pentamethylbenzofuran-3-ylmethyl]-(2R)-pyrrolidine-2-methoxy]phenyl methyl]thiazolidine-2,4-dione and its salts;
5-[4-[N-[2,3-dihydro-5-hydroxy-2,2,4,6,7-pentamethylbenzofuran-3-ylmethyl]-(2S)-pyrrolidine-2-methoxy]phenyl methyl]thiazolidine-2,4-dione hydrochloride salt;
5-[4-[N-[2,3-dihydro-5-hydroxy-2,2,4,6,7-pentamethylbenzofuran-3-ylmethyl]-(2S)-pyrrolidine-2-methoxy]phenyl methylene]thiazolidine-2,4-dione;
5-[4-[N-[2,3-dihydro-5-hydroxy-2,2,4,6,7-pentamethylbenzofuran-3-ylmethyl]-(2R)-pyrrolidine-2-methoxy]phenyl methylene]thiazolidine-2,4-dione and its salts;
5-[4-[N-[5-benzyloxy-2-methylbenzofuran-3-ylmethyl]-(2S)-pyrrolidine-2-methoxy]phenyl methylene]thiazolidine-2,4-dione;
5-[4-[N-[5-benzyloxy-2-methylbenzofuran-3-ylmethyl]-(2S)-pyrrolidine-2-methoxy]phenyl methylene] thiazolidine-2,4-dione sodium salt, and
5-[4-[N-[(5-benzyloxy-2-methylbenzofuran-3-ylmethyl]-(2S)-pyrrolidine-2-methoxy]phenyl methyl]thiazolidine-2,4-dione.

According to a feature of the present invention, there is provided a process for the preparation of novel intermediate of the general formula (III)

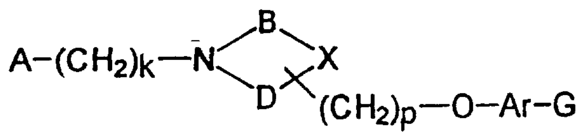

where A, B, D, X, Ar, k and p are as defined in formula (I) and G is as defined above.

In an embodiment of the invention, the novel intermediate of the general formula (III) defined above where G is CHO or $NO_2$ group, can be prepared by reacting the compound of the general formula (IV),

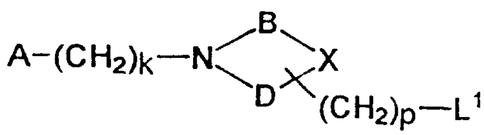

(IV)

wherein, A, B, D, X, k and p are as defined earlier and $L^1$ is a halogen atom such as chlorine, bromine or iodine or a leaving group such as methanesulfonate, trifluoromethanesulfonate, p-toluenesulfonate and the like with a compound of the formula (V)

$$HO—Ar—G \qquad (V)$$

where G is a CHO or a $NO_2$ group and Ar is as defined earlier.

The reaction of compound of formula (IV) with the compound of formula (V) to produce a compound of formula (III) may be carried out in the presence of solvents such as THF, DMF, DMSO, DME and the like. Mixtures of solvents may be used. An inert atmosphere may be used and may be maintained by using inert gases such as $N_2$, Ar or He. The reaction may be effected in the presence of a base such as $K_2CO_3$, $Na_2CO_3$, NaH, or mixtures thereof. The reaction temperature may range from 20°C. to 150°C., preferably at a temperature in the range of 30°C. to 100°C. The duration of the reaction may range from 1 to 24 hours, preferably from 2 to 6 hours.

In another embodiment of the present invention, the novel intermediate of general formula (III), where G is a CHO or $NO_2$ group, can also be prepared by the reaction of compound of general formula (VI)

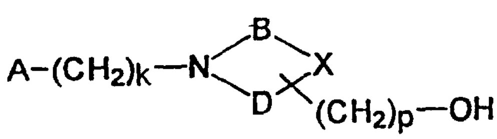

(VI)

where A, B, D, X, k and p are as defined earlier with a compound of general formula (VII)

$$L^2\text{-Ar-G} \qquad (VII)$$

where G is a CHO or $NO_2$ group and Ar is as defined earlier and $L^2$ represents a halogen atom such as chlorine or fluorine.

The reaction of compound of formula (VI) with a compound of formula (VII) to produce a compound of the formula (III) may be carried out in the presence of solvents such as THF, DMF, DMSO, DME and the like or mixtures thereof. The reaction may be carried out in an inert atmosphere which is maintained by using inert gases such as $N_2$, Ar, or He. The reaction may be effected in the presence of a base such as $K_2CO_3$, $Na_2CO_3$ or NaH or mixtures thereof. The reaction temperature may range from 20°C. to 120°C., preferably at a temperature in the range of 30°C. to 100°C. The duration of the reaction may range from 1 to 12 hours, preferably from 2 to 6 hours.

The novel intermediate of formula (III) can also be obtained by the reaction of a compound of general formula (VI) defined above with a compound of general formula (V) defined earlier.

The reaction of compound of general formula (VI) with a compound of general formula (V) may be carried out using suitable coupling agents such as dicyclohexyl urea, triarylphosphine/dialkylazadicarboxylate such as $PPh_3$/DEAD and the like. The reaction may be carried out in the presence of solvents such as THF, DME, $CH_2Cl_2$, $CHCl_3$, toluene, acetonitrile, carbontetrachloride and the like. The reaction may be carried out in an inert atmosphere which may be maintained by using inert gases such as $N_2$, Ar, or He. The reaction may be effected in the presence of DMAP, HOBT and they may be used in the range of 0.05 to 2 equivalents, preferably 0.25 to 1 equivalents. The reaction temperature may be in the range of 0°C. to 100°C., preferably at a temperature in the range of 20°C. to 80°C. The duration of the reaction may range from 0.5 to 24 hours, preferably from 6 to 12 hours.

In another embodiment of this invention, there is provided a process for the preparation of a compound of general formula (III) where G is a CHO or a NO2 group, and other symbols are as defined earlier which comprises reacting a compound of general formula (VIII)

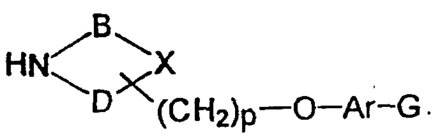

(VIII)

where B, D, X, Ar and p are as defined earlier, with a compound of general formula (IX)

$$A—(CH_2)_k—L^1 \qquad (IX)$$

where A, $L^1$ and k are as defined earlier.

The reaction of compound of general formula (VIII) with a compound of general formula (IX) may be carried out neat or in the presence of solvents such as DMF, DMSO, $CH_3CN$, EtOH, or acetone. The reaction may be carried out in the inert atmosphere which may be maintained by using inert gases such as $N_2$, Ar, or He. The reaction may be an inert atmosphere and effected in the presence of base such as $K_2CO_3$, $Na_2CO_3$, KOH, NaOH, NaH and the like or mixtures thereof. The amount of base may range from 1 to 20 equivalents, preferably 1 to 10 equivalents. The reaction may be carried out at a temperature in the range 20°C. to 180°C., preferably at a temperature in the range 50°C. to 150°C. Duration of the reaction may range from 1 to 48 hours, preferably from 1 to 12 hours. The amounts of the compound of general formula (VIII) and (IX) may range from 1 to 20 equivalents, preferably from 1 to 5 equivalents.

The compound of general formula (VIII) in turn can be prepared by reacting a compound of general formula (X)

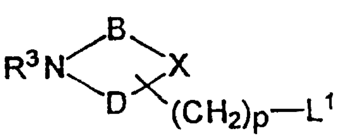

(X)

where B, D, $L^1$, X and p are as defined earlier and $R^3$ is a hydrogen atom or a protecting group, with a compound of general formula (V) followed by removal of N-protecting group using conventional methods.

The reaction of compound of general formula (X) with a compound of general formula (V) may be carried out in the presence of solvents such as THF, DMF, DMSO, DME and the like or mixtures thereof. The inert atmosphere may be maintained by using inert gases such as $N_2$, Ar, or He. The reaction may be effected in the presence of a base such as $K_2CO_3$, $Na_2CO_3$, NaH and the like or mixtures thereof. The reaction temperature may range from 20°C. to

120°C., preferably at a temperature in the range of 30°C. to 80°C. The duration of the reaction may range from 1 to 24 hours, preferably from 2 to 12 hours.

The compound of general formula (VIII) can also be prepared by reacting a compound of general formula (XI)

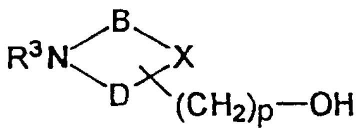

(XI)

where B, D, X, p, and $R^3$ are as defined earlier, with a compound of general formula (V) or with a compound of general formula (VII) defined earlier.

The reaction of compound of general formula (XI) with compound of general formula (V) may be carried out out using suitable coupling agents such as dicyclohexyl urea, triarylphosphine/dialkylazadicarboxylate such as $PPh_3$/DEAD and the like. The reaction may be carried out in the presence of solvents such as THF, DME, $CH_2Cl_2$, $CHCl_3$, toluene, acetonitrile, carbontetrachloride and the like. The reaction may be carried out in an inert atmosphere and it may be maintained by using inert gases such as $N_2$, Ar, or He. The reaction may be effected in the presence of DMAP, HOBT and they may be used in the range of 0.05 to 2 equivalents, preferably 0.25 to 1 equivalents. The reaction temperature may be in the range of 0°C. to 100°C., preferably at a temperature in the range of 20°C. to 80°C. The duration of the reaction may range from 0.5 to 24 hours, preferably from 6 to 12 hours.

The reaction of compound of general formula (XI) with a compound of general formula (VII) to produce a compound of general formula (VIII) may be carried out in the presence of solvents such as THF, DMF, DMSO, DME and the like or mixtures thereof. The reaction may be carried out in an inert atmosphere which is maintained by using inert gases such as $N_2$, Ar, or He. The reaction may be effected in the presence of a base such as $K_2CO_3$, $Na_2CO_3$ or NaH or mixtures thereof. The reaction temperature may range from 20°C. to 120°C., preferably at a temperature in the range of 30°C. to 100°C. The duration of the reaction may range from 1 to 12 hours, preferably from 2 to 6 hours.

The present invention provides a process for the preparation of novel azolidinedione derivatives of general formula (I), their tautomeric forms, their stereoisomers, their polymorphs, their pharmaceutically acceptable salts and their pharmaceutically acceptable solvates wherein $R^1$ and Y together represent a bond and Z represents a sulfur or oxygen atom and all symbols are as defined earlier which comprises:

reacting the compound of general formula (III), where G is a CHO group with 2,4-thiazolidinedione or 2, 4- oxazolidinedione to yield a compound of general formula (XII)

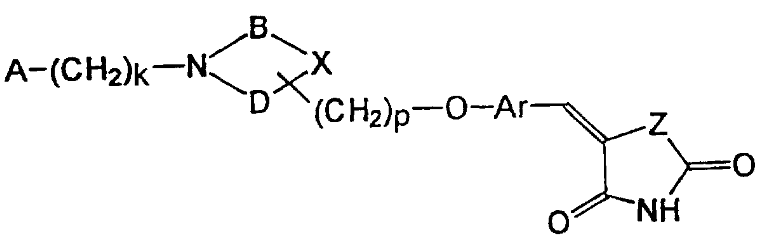

(XII)

where A, B, D, X, Ar, k, p and Z are as defined earlier and removing the water formed during the reaction by conventional methods.

The reaction between the compound of the general formula (III) where G is a CHO group with 2,4-thiazolidinedione or 2,4-oxazolidinedione to yield a compound of general formula (XII) may be carried out neat in the presence of sodium acetate or in the presence of a solvent such as benzene, toluene, methoxyethanol or mixtures thereof. The reaction temperature may range from 80°C. to 140°C. depending upon the solvents employed and in the range from 80°C. to 180°C. when the reaction is carried out neat in the presence of sodium acetate. Suitable catalyst such as piperidinium acetate or benzoate, sodium acetate or mixtures of catalysts may also be employed. Sodium acetate can be used in the presence of solvent, but it is preferred that sodium acetate is used neat. The water produced in the reaction may be removed, for example, by using Dean Stark water separator or by using water absorbing agents like molecular sieves. Oxazolidine-4-oxo-2-thione may be used instead of 2,4-oxazolidinedione. However, the thio group needs to be converted to oxo group by oxidation using agents such as hydrogen peroxide or peroxyacids like mCPBA.

The compound of the general formula (XII) obtained in the manner described above is reduced by known methods to obtain the compound of general formula (XIII)

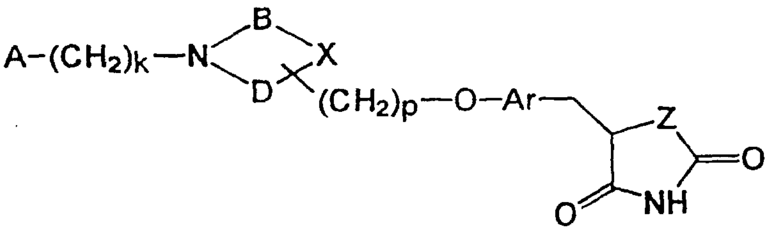

(XIII)

where A, B, D, X, Ar, k, p and Z are as defined earlier. The compound of general formula (XIII) represents the compound of general formula (I), wherein $R^1$ is hydrogen and Y is $CR^2$ where $R^2$ is hydrogen atom and all other symbols are as defined earlier.

The reduction of compound of the formula (XII) to yield a compound of the general formula (XIII) may be carried out in the presence of gaseous hydrogen and a catalyst such as Pd/C, Rh/C, Pt/C, and the like. Mixtures of catalysts may be used. The reaction may also be conducted in the presence of solvents such as dioxane, acetic acid, ethyl acetate and the like. Mixtures of solvents may be used. A pressure between atmospheric pressure and 80 psi may be employed. The catalyst may be 5 - 10 % Pd/C and the amount of catalyst used may range from 50 - 300 % w/w. The reaction may also be carried out by employing metal solvent reduction such as magnesium in methanol or sodium amalgam in methanol. The reaction may also be carried out with alkali metal borohydrides such as $LiBH_4$, $NaBH_4$, $KBH_4$ and the like in the presence of cobalt salt such as $CoCl_2$ and ligands, preferably bidentated ligands such as 2, 2'-bipyridyl; 1, 10-phenanthroline; bisoximes and the like.

The compound of the general formula (XII) and of general formula (XIII) obtained above may be converted into pharmaceutically acceptable salts, or pharmaceutically acceptable solvates by conventional methods.

In yet another embodiment of the present invention, the compound of the general formula (I) can also be prepared by reacting a compound of the general formula (IV) defined above with a compound of general formula (XIV)

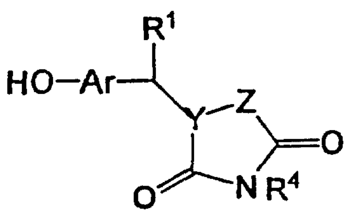

(XIV)

where $R^1$, Y, Z and Ar are as defined earlier and $R^4$ is hydrogen or a nitrogen protecting group which is removed after the reaction.

The reaction of compound of general formula (IV) with a compound of general formula (XIV) to produce a compound of general formula (I) may be carried out in the presence of solvents such as THF, DMF, DMSO, DME and the like or mixtures thereof. The reaction may be carried out in an inert atmosphere which is maintained by using inert gases such as $N_2$, Ar or He. The reaction may be effected in the presence of a base such as alkalis like sodium hydroxide or potassium hydroxide; alkali metal carbonates like sodium carbonate or potassium carbonate; alkali metal hydrides such as sodium hydride; organometallic bases like n-butyl lithium; alkali metal amides like sodamide, or mixtures thereof. Multiple solvents and bases can be used. The amount of base may range from 1 to 5 equivalents, preferably 1 to 3 equivalents. The reaction temperature may be in the range of 0°C. to 120°C., preferably at a temperature in the range of 20°C. to 100°C. The duration of the reaction may range from 0.5 to 24 hours, preferably from 0.5 to 6 hours.

The removal of protecting groups may be carried out by conventional methods which include treatment with acid such as, hydrochloric acid, trifluoroacetic acid or bases such as, KOH, NaOH, $Na_2CO_3$, $NaHCO_3$, $K_2CO_3$ and the like or mixtures thereof. These reagents may be used as aqueous solution or as solutions in alcohols like methanol, ethanol etc. Deprotection can also be effected by gaseous hydrogen in the presence of catalyst such as Pd/carbon or conventional transfer hydrogenation methods when the protecting group is a benzyl or substituted benzyl group.

The compound of general formula (I) can also be obtained by reacting a compound of general formula (VI)

with a compound of general formula (XIV).

The reaction of compound of general formula (VI) with a compound of general formula (XIV) to produce a compound of general formula (I) may be carried out using suitable coupling agents such as dicyclohexyl urea, triarylphosphine dialkylazadicarboxylate such as $PPh_3$/DEAD, and the like. The reaction may be carried out in the presence of solvents such as THF, DME, $CH_2Cl_2$, $CHCl_3$, toluene, acetonitrile, carbontetrachloride and the like. An inert atmosphere may be used and the inert atmosphere may be maintained by using inert gases such as $N_2$, Ar, or He. The reaction may be effected in the presence of DMAP-HOBT and they may be used in the range of 0.05 to 2 equivalents, preferably 0.25 to 1 equivalents. The reaction temperature may be in the range of 0°C. to 100°C., preferably at a temperature in the range of 20°C. to 80°C. The duration of the reaction may range from 0.5 to 24 hours, preferably from 6 to 12 hours.

In another embodiment of the present invention, the compound of general formula (I), where A, B, D, X, k, p and Ar are as defined earlier and R' represents hydrogen, Y represents CH and Z represents a oxygen or a sulfur atom can be prepared by the reaction of compound of general formula (XV)

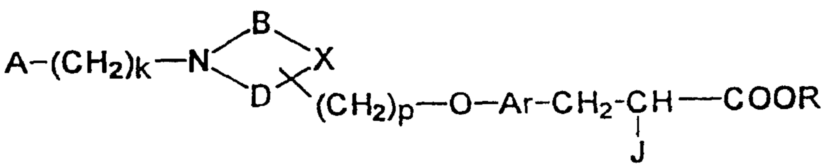

(XV)

where A, B, D, X, k, p and Ar are as defined earlier, J is a halogen atom like chlorine, bromine or iodine or a hydroxy group and R is a lower alkyl group, with thiourea when J is a halogen atom, or with urea when J is a hydroxy group followed by treatment with an acid.

The reaction of compound of general formula (XV) with thiourea or urea is normally carried out in the presence of alcoholic solvent such as methanol, ethanol, propanol, isobutanol, 2-methoxybutanol, etc or DMSO or sulfolane. The reaction may be conducted at a temperature in the range between 20°C. and the reflux temperature of the solvent used. Bases such as NaOAc, KOAc, NaOMe, NaOEt etc. can be used. The reaction is normally followed by treatment with a mineral acid such as hydrochloric acid at 20°C. to 100°C.

The compound of general formula (XV) where J is hydroxy group is prepared by the reaction of compound of general formula (XV) where J is a halogen atom with aqueous alkali at a temperature ranging from 20°C. to 100°C. followed by reesterification of the hydrolysed acid group during the reaction, by conventional methods.

The compound of general formula (XV) where J is a OH group may also be prepared from compound of formula (XV) where J is a halogen atom by reacting with formamide in the presence of water. The amount of formamide used in the reaction ranges from 0.5 to 1.5 mL and water used ranges from 20 µL to 0.1 mL for one mmol of the halo compound (XV). The reaction is conducted at a temperature ranging from 80°C. to 180°C., preferably from 120°C. to 150°C., over a period ranging from 1 to 8 hours.

The compound of general formula (XV) where J is a halogen atom can be prepared by the diazotization of the amino compound of the general formula (XVI)

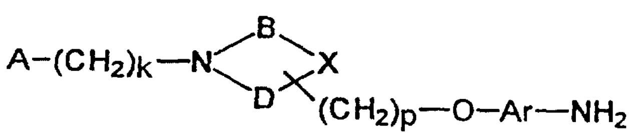

(XVI)

where all symbols are as defined earlier, using alkali metal nitrites followed by treatment with acrylic acid esters in the presence of hydrohalo acids and catalytic amount of copper oxide or copper halide.

The compound of general formula (XVI) can in turn be prepared by the conventional reduction of the novel intermediate (III) where G is NO2 group and other symbols are as defined earlier.

The intermediates of the formula (VI) defined above can be prepared by a process which comprises reacting a compound of general formula (IX) defined earlier with a compound of general formula (XVII)

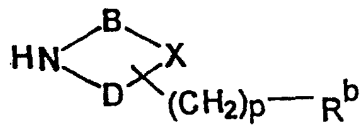

(XVII)

where B, D, X and p are as defined earlier, and $R^b$ is a hydroxy group or a group, such as carboxylic acid group, ester, amide, ether and the like, which can be converted to hydroxy group by conventional methods.

The reaction of compound of general formula (XVII) with a compound of general formula (IX) to yield a compound of general formula (VI) may be carried out in neat or in the presence of solvents such as DMF, DMSO, $CH_3CN$, EtOH, or acetone. The reaction may be conducted in an inert atmosphere and the inert atmosphere is maintained by using inert gases such as $N_2$, Ar, or He. The reaction may be effected in the presence of base such as $K_2CO_3$, $Na_2CO_3$, KOH, NaOH, NaH and the like. The amount of base may range from 1 to 20 equivalents, preferably 1 to 10 equivalents. The reaction may be carried out at a temperature in the range 20°C. to 180°C., preferably at a temperature in the range 50°C. to 150°C. Duration of the reaction may range from 1 to 48 hours, preferably from 1 to 12 hours. The amounts of the compound of general formula (XVII) and (IX) may range from 1 to 20 equivalents, preferably from 1 to 5 equivalents.

The compound of general formula (VI) where k = 1 can also be prepared by reacting the compound of general formula (XVII) defined above in the presence of formaldehyde solution or a source of formyl moiety thereof, with A-H where A may be substituted or unsubstituted single or fused aromatic or heterocycle or their tautomeric forms. Suitable catalyst may be employed.

The reaction of compound of general formula (XVII) with A-H to yield a compound of general formula (VI) where k = 1, may be carried out using formaldehyde, paraformaldehyde, $POCl_3$-DMF and the like. The reaction may be carried out in the presence of solvents such as methanol, ethanol, 2-methoxyethanol or neat. These reagents may be used in the range of 1 to 4 equivalents, preferably 1 to 2 equivalents. The reaction may be carried out at a temperature in the range of -10°C to 100°C., preferably from 0°C. to 90°C. Duration of the reaction may range from 1 to 24 hours, preferably from 2 to 12 hours.

In yet another embodiment of the present invention, the compound of general formula (I), where A, B, D, X, Ar, k and p are as defined earlier and Y is nitrogen atom and Z is oxygen atom can be prepared by a process which comprises : reaction of novel intermediate of formula (III) defined earlier, where G represents a CHO group with $NH_2OH$.HCl to yield a compound of general formula (III) where G represents CH=NOH group and all symbols are as defined earlier, followed by metal borohydride reduction to yield a compound of general formula (XVIII)

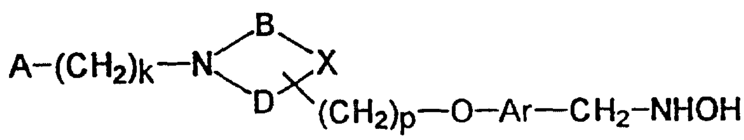

(XVIII)

where all symbols are as defined earlier.

The reaction of compound of general formula (III), where G is CHO group and other symbols are as defined earlier, with hydroxylamine hydrochloride is carried out in solvents such as ethanol, methanol, THF, dioxane and the like following the conventional method to make oximes. 1 to 10 equivalents of $NH_2OH$.HCl may be used, preferably, 2 to 5 equivalents. Bases such as alkali metal acetates or ammonium acetate may be used. Reaction may be carried out in the presence of water. Temperature in the range of 0°C. to reflux temperature of the solvent may be used. The oxime obtained in the manner described above is reduced using reducing agents such as alkali metal borohydrides like sodium borohydride or sodium cyanoborohydride or borane reagents using conventional conditions to yield the compound of general formula (XVIII).

The compound of general formula (XVIII) in turn is reacted with halocarbonyl isocyanate or alkoxycarbonyl isocyanate to yield a compound of general formula (I) or with KOCN to yield a compound of general formula (III) where G is $CH_2N(OH)CONH_2$, followed by treatment with carbonylating agents such as alkylhaloformate to produce the compound of general formula (I) where A, B, D, X, Ar, p and k are as defined earlier, Y represents nitrogen atom and Z is oxygen atom.

The reaction of compound of general formula (XVIII) with halocarbonyl isocyanate such as chlorocarbonyl isocyanate or alkoxycarbonyl isocyanate such as ethoxycarbonyl isocyanate may be carried out in inert solvents such

as THF, dioxane, etc. at a temperature in the range -15°C. to 50°C. The reaction may be carried out for 0.5 to 12 hours depending on the substrates used for the reaction.

Alternatively, the compound of general formula (XVIII) may be treated with excess of KOCN in organic acids such as acetic acid. Water may be used in the reaction. The reaction may be carried out at a temperature in the range of 20°C. to 120°C. The product isolated in the reaction is further treated with alkyl haloformate such as ethyl chloroformate in the presence of 1 to 10 equivalents of alkali such as sodium hydroxide, potassium hydroxide and the like to obtain compound of general formula (I) where all the symbols are as defined earlier and Y represents nitrogen atom and Z represents oxygen atom.

The pharmaceutically acceptable salts are prepared by reacting the compound of formula (I) with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol etc. Mixture of solvents may be used. Organic bases like lysine, arginine, diethanolamine, choline, guanidine and their derivatives etc. may also be used. Alternatively, acid addition salts are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid, salicylic acid, hydroxynaphthoic acid, ascorbic acid, palmitic acid, succinic acid, benzoic acid, benzene sulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, resolving the diastereomeric salts formed with chiral acids such as mandelic acid, camphorsulfonic acid, tartaric acid, lactic acid and the like or chiral bases such as brucine, cinchona alkaloids and their derivatives and the like.

Various polymorphs of compound of general formula (I) forming part of this invention may be prepared by crystallization of compound of formula (I) under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or slow cooling. The presence of polymorphs may be determined by solid probe NMR spectroscopy, IR spectroscopy, differential scanning calorimetry, powder X-ray data or such other techniques.

The present invention also provides a pharmaceutical composition, containing the compounds of the general formula (I), as defined above, their tautomeric forms, their stereoisomers, their polymorphs, their pharmaceutically acceptable salts, their pharmaceutically acceptable solvates in combination with the usual pharmaceutically employed carriers, diluents and the like, useful for the treatment and / or prophylaxis of diseases in which insulin resistance is the underlying pathophysiological mechanism such as type II diabetes, impaired glucose tolerance, dyslipidaemia, hypertension, coronary heart disease and other cardiovascular disorders including atherosclerosis; insulin resistance associated with obesity and psoriasis, for treating diabetic complications and other diseases such as polycystic ovarian syndrome (PCOS), certain renal diseases including diabetic nephropathy, glomerulonephritis, glomerular sclerosis, nephrotic syndrome, hypertensive nephrosclerosis, end-stage renal diseases and microalbuminuria as well as certain eating disorders, as aldose reductase inhibitors and for improving cognitive functions in dementia.

The pharmaceutical composition may be in the forms normally employed, such as tablets, capsules, powders, syrups, solutions, suspensions and the like, may contain flavorants, sweeteners etc. in suitable solid or liquid carriers or diluents, or in suitable sterile media to form injectable solutions or suspensions. Such compositions typically contain from 1 to 25 %, preferably 1 to 15 % by weight of active compound, the remainder of the composition being. pharmaceutically acceptable carriers, diluents or solvents.

A typical tablet production method is exemplified below:

**Tablet Production Example**

| | | | |
|---|---|---|---|
| a) | 1) | Active ingredient | 30 g |
| | 2) | Lactose | 95 g |
| | 3) | Corn starch | 30 g |
| | 4) | Carboxy methyl cellulose | 44 g |
| | 5) | Magnesium stearate | 1 g |
| | | | 200 g for 1000 tablets |

The ingredients 1-3 are uniformly blended with water and granulated after drying under reduced pressure. The ingredients 4 & 5 are mixed well with the granules and compressed by a tabletting machine to prepare 1000 tablets each containing 30 mg of ingredient.

| | | | |
|---|---|---|---|
| b) | 1) | Active ingredient | 30 g |
| | 2) | Calcium phosphate | 90 g |
| | 3) | Lactose | 40 g |
| | 4) | Corn starch | 35 g |
| | 5) | Polyvinyl pyrrolidone | 3.5 g |
| | 6) | Magnesium stearate | 1.5 g |
| | | | 200 g for 1000 tablets |

The ingredients 1-4 are uniformly moistened with an aqueous solution of 5 and granulated after drying under reduced pressure. Ingredient 6 is added and granules are compressed by a tabletting machine to prepare 1000 tablets containing 30 mg of ingredient 1.

The compound of the formula (I) as defined above are clinically administered to mammals, including man, *via* either oral or parenteral routes. Administration by the oral route is preferred, being more convenient and avoiding the possible pain and irritation of injection. However, in circumstances where the patient cannot swallow the medication, or absorption following oral administration is impaired, as by disease or other abnormality, it is essential that the drug be administered parenterally. By either route, the dosage is in the range of about 0. 10 to about 200 mg/kg body weight of the subject per day or preferably about 0. 10 to about 50 mg/kg body weight per day administered singly or as a divided dose. However, the optimum dosage for the individual subject being treated will be determined by the person responsible for treatment, generally smaller doses being administered initially and thereafter increments made to determine the most suitable dosage.

Suitable pharmaceutically acceptable carriers include solid fillers or diluents and sterile aqueous or organic solutions. The active compound will be present in such pharmaceutical compositions in amounts sufficient to provide the desired dosage in the range as described above. Thus, for oral administration, the compounds can be combined with a suitable solid or liquid carrier or diluent to form capsules, tablets, powders, syrups, solutions, suspensions and the like. The pharmaceutical compositions, may, if desired, contain additional components such as flavorants, sweeteners, excipients and the like. For parenteral administration, the compounds can be combined with sterile aqueous or organic media to form injectable solutions or suspensions. For example, solutions in sesame or peanut oil, aqueous propylene glycol and the like can be used, as well as aqueous solutions of water-soluble pharmaceutically-acceptable acid addition salts or alkali or alkaline earth metal salts of the compounds. The injectable solutions prepared in this manner can then be, administered intravenously, intraperitoneally, subcutaneously, or intramuscularly, with intramuscular administration being preferred in humans.

The invention is explained in detail in the examples given below which are provided by way of illustration only and therefore should not be construed to limit the scope of the invention.

**Preparation 1**

**N-[(2RS)-6-Benzyloxy-2,5,7,8-tetramethylchroman-2-ylmethyl]-(2S)-pyrrolidine-2-methanol:**

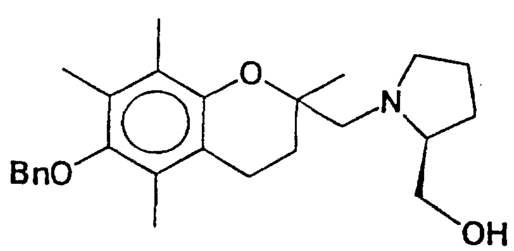

A mixture of (2RS)-6-benzyloxy-2,5,7,8-tetramethylchroman-2-ylmethyl methanesulphonate (100 g) and L-prolinol (100 g) was heated under nitrogen atmosphere at 120°C. with stirring for 6 h. The mixture was cooled to room temperature and poured into water and the solution was extracted with $CH_2Cl_2$ repeatedly. The combined organic

extracts were washed with brine, dried ($Na_2SO_4$) and evaporated to dryness under reduced pressure to give 101 g (100 %) of the crude compound which was chromatographed on silica gel using 0.5 % methanol in chloroform to afford 75.7 g (75 %) of the title compound as a thick liquid.

$[\alpha]_D^{27}$ = -9.5 (c, 1.0, $CHCl_3$).

$^1H$ NMR ($CDCl_3$, 200 MHz) : δ 1.19, 1.25 (2S, 3H), 1.55 - 2.05 (m, 6H), 2.11 (s, 3H), 2.18 (s, 3H), 2.22 (s, 3H), 2.35 - 3.0 (m, 6H), 3.25 - 3.75 (m, 3H), 4.7 (s, 2H), 7.2 - 7.6 (m, 5H).

**Preparation 2**

**N-[(3RS)-5-Benzyloxy-2,3-dihydro-2,2,4,6,7-pentamethylbenzofuran-3-ylmethyl]-(2S)-pyrrolidine-2-methanol**

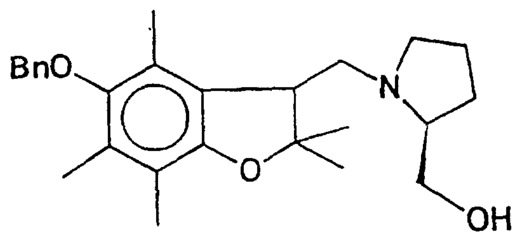

The title compound (1 g, 66 %) was prepared as a pale yellow solid from (3RS)-5-benzyloxy-2,3-dihydro-2,2,4,6,7-pentamethylbenzofuran-3-ylmethyl methanesulfonate (1.49 g) and L-prolinol (1.5 g) by an analogous procedure to that described in preparation 1, mp 99 - 101°C.

$[\alpha]_D^{26}$ = -32.8 (c, 1.0, $CHCl_3$).

$^1H$ NMR ($CDCl_3$, 200 MHz) : δ 1.30, 1.34 (2s, 3H), 1.58, 1.62 ( 2s, 3H), 1.78 (m, 4H), 2.12 (s, 3H), 2.20, 2.22 (2s, 3H), 2.28, 2.30 (2s, 3H), 2.33 (m, 2H), 2.68 (d, J = 6.0 Hz, 1H), 3.09 (m, 1H), 3.30 (m, 2H), 3.36 (m, 1H), 3.68 (m, 1H), 4.76 (s, 2H), 7.46 (m, 5H).

**Preparation 3**

**N-[5-Benzyloxy-2,4,6,7-tetramethylbenzofuran-3-ylmethyl]-(2S)-pyrrolidine-2-methanol:**

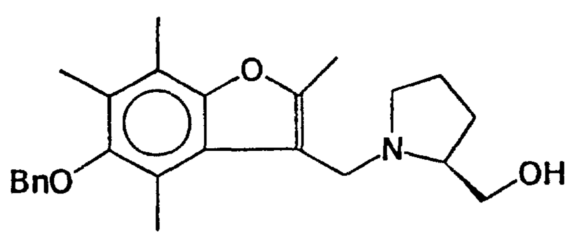

The title compound (7.5 g, 69 %) was prepared as a white solid from 5-benzyloxy-2,4,6,7-tetramethylbenzofuran-3-methylchloride (9.0 g) and L-prolinol (4.15 g) by an analogous procedure to that described in preparation 1. mp 98 - 100°C.

$[\alpha]_D^{20}$ = -15.5 (c, 1.0, $CHCl_3$)

$^1H$ NMR ($CDCl_3$, 200 MHz): δ 1.85 (m, 4H), 2.36 (s, 3H), 2.41 (s, 3H), 2.48 (s, 3H), 2.50 (m, 1H), 2.64 (s, 3H), 2.76 (m, 1H), 3.04 (m, 1H), 3.34 (dd, J = 10.8 and 2.5 Hz, 1H)), 3.51 (dd, J 10.2, 3.5 Hz, 1H), 3.60 (d, J = 13.2 Hz, 1H), 3.95 (d, J = 13.2 Hz, 1H), 4.78 (s, 2H), 7.48 (m, 5H).

**Preparation 4**

**N-(5-Benzyloxy-2-methylbenzofuran-3-ylmethyl)-(2S)-pyrrolidine-2-methanol**

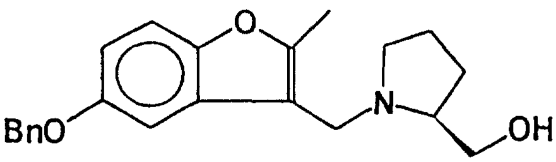

The title compound (4.1 g, 67 %) was prepared as an oil from 5-benzyloxy-2-methylbenzofuran-3-methylchloride (5.0 g) and L-prolinol (1.76 g) by an analogous procedure to that described in preparation 1.

$[\alpha]_D^{21}$ = -26.3 (c, 0.5, $CHCl_3$)

$^1H$ NMR ($CDCl_3$, 200 MHz): δ 1.63 (m, 2H), 1.88 (m, 2H), 2.32 (m, 1H), 2.43 (s, 3H), 2.75 (m, 1H), 2.92 (m, 1H), 3.41 (m, 2H), 3.75 (dd, J = 10.7 Hz and 3.34 Hz, 1H), 3.90 (d, J = 13.3 Hz, 1H), 5.09 (s, 2H), 6.89 (m, 1H), 7.06 (m, 1H), 7.45 (m, 6H).

**Preparation 5**

**N-(1-Methylindol-3-ylmethyl)-(2S)-pyrrolidine-2-methanol:**

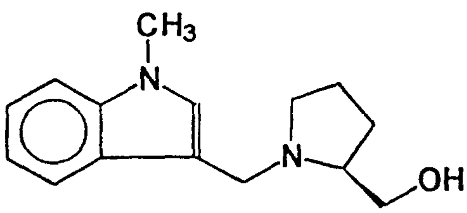

To a mixture of formalin (0.33 ml, 48 % in $H_2O$) and acetic acid (0.9 ml) was added L-prolinol (0.43 g) with stirring under ice-cooling. After 5 min, 1-methyl indole (0.56 g) was added dropwise to the above reaction mixture, and stirring was continued for 2 h. At the end of this time, reaction mixture was basified with 2N NaOH; the oily residue was extracted with ethyl acetate, dried ($Na_2SO_4$), and the solvent was evaporated to dryness to give the title compound (0.87 g, 83 %), as a white solid, mp. 46-48°C.

$[\alpha]_D^{25}$ = -65.1 (c, 1.0, $CH_3OH$)

$^1H$ NMR ($CDCl_3$, 200 MHz): δ 1.68 (m, 2H), 1.85 (m, 2H), 2.36 (q, J = 9.3 Hz and 16.9 Hz, 1H), 2.75 (m, 1H), 3.03 (m, 1H), 3.42 (d, J = 9.9 Hz, 1H), 3.56 (d, J = 13.3 Hz, 1H), 3.79 (m, 4H), 4.08 (d, J = 13.3 Hz, 1H), 6.96 (s, 1H), 7.20 (m, 3H), 7.70 (d, J = 7.75 Hz, 1H).

**Preparation 6**

**1-[(2RS)-6-Benzyloxy-2,5,7,8-tetramethylchroman-2-ylmethyl]piperazine:**

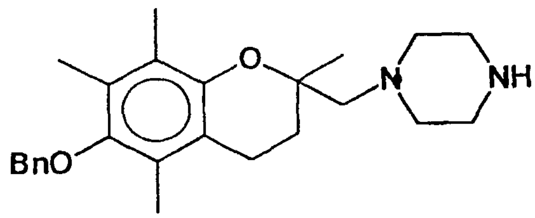

The title compound (7 g, 47 %) was prepared as a syrupy liquid from (2RS)-6-benzyloxy-2,5,7,8-tetramethylchroman-2-ylmethyl methanesulphonate (15 g) and piperazine (19 g) by an analogous procedure to that described in preparation 1.

$^1$H NMR ($CDCl_3$, 200 MHz): δ 1.22 (s, 3H), 1.7 (m, 1H), 1.95 (m, 1H), 2.03 (s, 3H), 2.17 (s, 3H), 2.21 (s, 3H), 2.60 (m, 4H), 2.95 (m, 4H), 3.2 (m, 4H), 4.7 (s, 2H), 7.3 - 7.6 (m, 5H).

**Preparation 7**

**N-[(2RS)-6-Benzyloxy-2,5,7,8-tetramethylchroman-2-ylmethyl]-(3R)-chloropiperidine:**

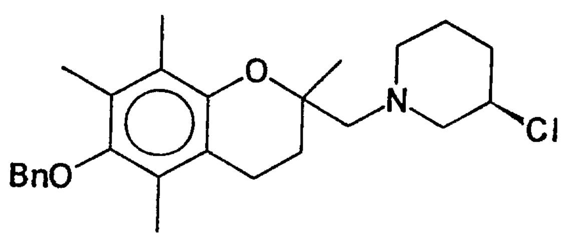

Thionyl chloride (6 ml) was added dropwise to a stirred, ice cooled solution of N-[(2RS)-6-benzyloxy-2,5,7,8-tetramethylchroman-2-ylmethyl]-(2S)-pyrrolidine-2-methanol (17 g) obtained in preparation 1, in dry benzene (200 ml). The resulting mixture was stirred at room temperature for 1 h and then diluted with ethyl acetate, washed with saturated aqueous sodium bicarbonate solution, water, brine and dried ($Na_2SO_4$). The organic layer was evaporated and the residue was chromatographed on silica gel using 12 % ethyl acetate in petroleum ether as eluent to give 13 g (73 %) of the title compound as a thick liquid.

$^1$H NMR ($CDCl_3$, 200 MHz): δ 1.24 (s, 3H), 1.4 - 2.5 (m, 8H), 2.1 (s, 3H), 2.18 (s, 3H), 2.23 (s, 3H), 2.53 (s, 2H), 2.63 (t, J = 6.8 Hz, 2H), 2.8 (m, 1H), 3.3 (m, 1H), 4.0 (m, 1H), 4.7 (s, 2H), 7.3 - 7.6 (m, 5H).

**Preparation 8**

**N-[(3RS)-5-Benzyloxy-2,3-dihydro-2,2,4,6,7-pentamethylbenzofuran-3-ylmethyl]-(3R)-chloropiperidine:**

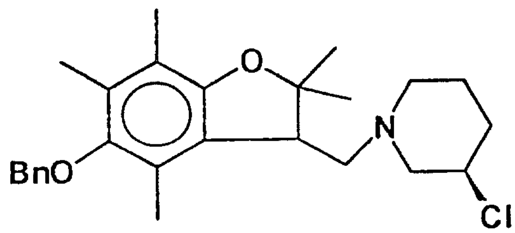

The title compound (0.85 g, 90 %) was prepared as a pale yellow solid from the product (0.9 g) obtained in preparation 2 and thionylchloride (0.79 ml) by a similar procedure to that used in preparation 7.

$^1$H NMR ($CDCl_3$, 200 MHz): δ 1.35 (s, 3H), 1.62 (bs, 5H), 1.80 (m, 2H), 2. 11 (s, 3H), 2.23 (s, 6H), 2.30 (m, 2H), 2.53 (m, 2H), 2.86 (m, 1H), 3.06 (m, 1H), 3.22 (m, 1H), 4.0 (m, 1H), 4.71 (s, 2H), 7.43 (m, 5H).

**Preparation 9**

**[N-(5-Benzyloxy-2,4,6,7-tetramethylbenzofuran-3-ylmethyl)-(3R)-chloropiperidine:**

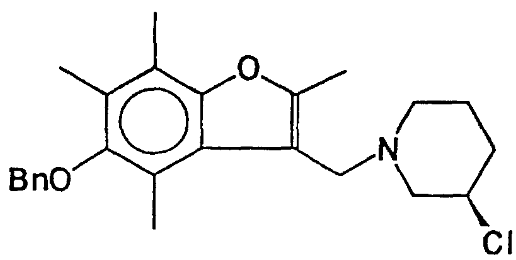

The title compound (2g, 63 %) was prepared as a white solid from N-(5-benzyloxy-2,4,6,7-tetramethylbenzofuran-3-ylmethyl)-(2S)-pyrrolidine-2-methanol (3.0 g) obtained in preparation 3 and thionylchloride (2.78 ml) by a similar procedure to that used in preparation 7. mp 90 - 92°C.

$[\alpha]_D^{20}$ = -49.7 (c, 1.0, $CHCl_3$)

$^1$H NMR ($CDCl_3$, 200 MHz): δ 1.68 (m, 3H), 2.19 (m, 3H), 2.36 (s, 3H), 2.45 (s, 6H), 2.62 (s, 3H), 2.78 (m, 1H), 3.13 (m, 1H), 3.51 (d, J = 6.2 Hz, 2H), 3.92 (m, 1H), 4.72 (s, 2H), 7.47 (m, 5H).

**Preparation 10**

**N-(5-benzyloxy-2-methylbenzofuran-3-ylmethyl)-(3R)-chloropiperidine:**

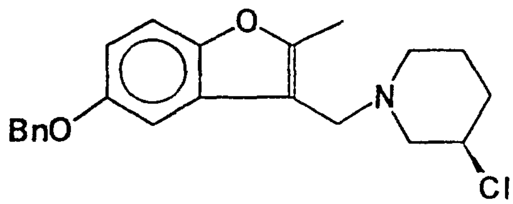

The title compound (2.3 g, 100 %) was prepared as an oil from N-(5-benzyloxy-2-methylbenzofuran-3-ylmethyl)-(2S)-pyrrolidine-2-methanol (2.25 g) obtained in preparation 4 and thionylchloride (2.34 ml) by a similar procedure to that used in preparation 7.

$[\alpha]_D^{25}$ = -20.6 (c, 1.83, $CHCl_3$)

$^1H$ NMR ($CDCl_3$, 200 MHz): δ 1.62 (m, 3H), 2.20 (m, 3H), 2.39 (s, 3H), 2.68 (m, 1H), 3.05 (m, 1H), 3.54 (s, 2H), 3.89 (m, 1H), 5.11 (s, 2H), 6.88 (m, 1H), 7.42 (m, 7H).

**Preparation 11**

**N-(1-Methylindol-3-ylmethyl)-(2S)-pyrrolidine-2-methyl methanesulfonate:**

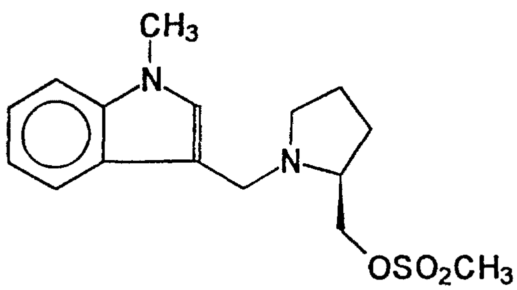

To an ice cooled solution of the product (0.5 g) obtained in preparation 5 and triethylamine (0.85 ml) in $CH_2Cl_2$ (10 ml) was added methanesulfonyl chloride (0.2 ml). The mixture was stirred at 5°C. for 2 h. At the end of this time, the reaction mixture was washed with aqueous $NaHCO_3$ solution followed by $H_2O$, dried ($CaCl_2$) and concentrated to get 0.65 g (98 %) of the title compound. The crude product was used in the next step without further purification.

**Preparation 12**

**2-[4-[(2RS)-6-Benzyloxy-2,5,7,8-tetramethylchroman-2-ylmethyl]piperazine-1-yl]ethanol:**

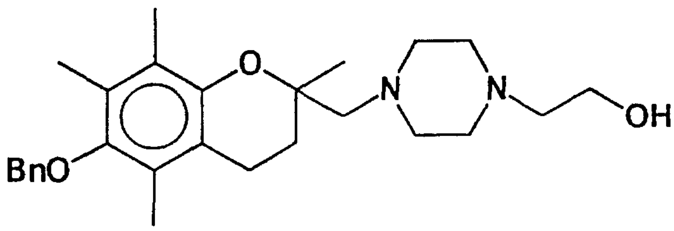

To a mixture of 1-[(2RS)-6-benzyloxy-2,5,7,8-tetramethylchroman-2-ylmethyl]piperazine (2.3 g) obtained in preparation 6 and 2-bromoethanol (0.62 ml) in dry DMSO (15 ml), $K_2CO_3$ (2.4 g) was added and the mixture was stirred at room temperature for 12 h. At the end of this time, the reaction mixture was cooled, added water and extracted with EtOAc. The EtOAc extract was washed with water followed by brine and dried over anhydrous sodium sulphate. The solvent was then removed by distillation under reduced pressure and the resulting crude compound was chromatographed on silica gel using 1 % methanol in chloroform to afford (0.7 g, 27 %) the pure compound as a thick liquid.

$^1H$ NMR ($CDCl_3$, 200 MHz): δ 1.24 (s, 3H), 1.7 (m, 1H), 2.0 (m, 1H), 2.07 (s, 3H), 2.18 (s, 3H), 2.22 (s, 3H), 2.5 - 3.0 (m, 14 H), 3.68 (t, J = 5.2 Hz, 2H), 4.7 (s, 2H), 7.3 - 7.6 (m, 5H).

**Preparation 13**

**2-[4-[(2RS)-6-Benzyloxy-2,5,7,8-tetramethylchroman-2-ylmethyl]piperazine-1-yl]ethyl chloride:**

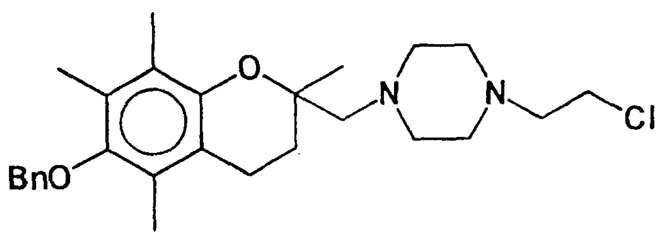

The title compound (0.2 g, 33 %) was prepared as a thick liquid from the product (0.6 g) obtained in preparation 12 and thionyl chloride (0.2 ml) by a similar procedure to that used in preparation 7.

$^1H$ NMR ($CDCl_3$, 200 MHz): δ 1.24 (s, 3H), 1.7 (m, 1H), 2.0 (m, 1H), 2.07 (s, 3H), 2.17 (s, 3H), 2.22 (s, 3H), 2.4 - 2.8 (m, 14H), 3.6 (t, J = 7 Hz, 2H), 4.7 (s, 2H), 7.3 - 7.6 (m, 5H).

**Preparation 14**

**a) 4-[N-[(2RS)-6-Benzyloxy-2,5,7,8-tetramethylchroman-2-ylmethyl]-(2S)-pyrrolidine-2-methoxy]benzaldehyde (14a)**

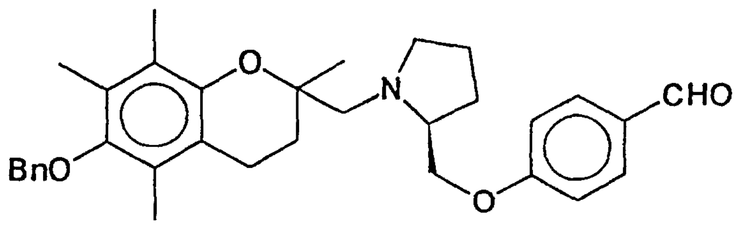

and

**b) 4-[N-[(2RS)-6-Benzyloxy-2,5,7,8-tetramethylchroman-2-ylmethyl]-(3R)-piperidinyloxy]benzaldehyde (14b)**

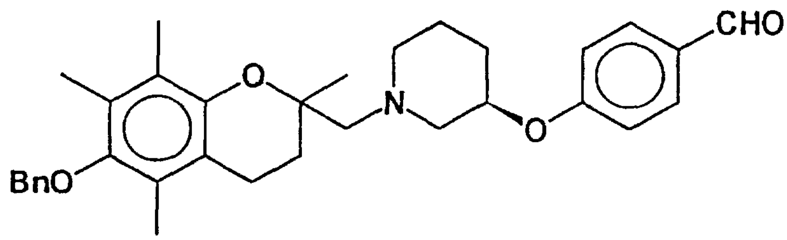

**Method A**

To a mixture of N-[(2RS)-6-benzyloxy-2,5,7,8-tetramethylchroman-2-ylmethyl]-(3R)-chloropiperidine (5 g) obtained in preparation 7 and 4-hydroxybenzaldehyde (1.7g) in dry DMF, $K_2CO_3$ (6.4 g) was added and the mixture was stirred at 80°C. for 2 h. At the end of this time, the mixture was cooled, water added and extracted with EtOAc. The EtOAc extract was washed with 5 % aqueous $Na_2CO_3$ solution, followed by brine and dried over anhydrous sodium sulphate. The solvent was then removed by distillation under reduced pressure to give 4.2 g (70 %) of the crude product as a mixture of **14a** and **14b** (1:1), which was separated by column chromatography on silica gel using 2 to 10 % (gradient elution) ethyl acetate in petroleum ether to afford **14a** (2.0 g, 33 %, as a syrupy liquid) and **14b** (2.1 g, 35 % as semi solid).

**Method B**

To a mixture of N-[(2RS)-6-benzyloxy-2,5,7,8-tetramethylchroman-2-ylmethyl]-(2S)-pyrrolidine-2-methanol (16 g) obtained in preparation 1, 4-hydroxybenzaldehyde (5.2 g) and triphenyl phosphine (11.8 g) in THF (200 ml), diisopropyl azodicarboxylate (15 ml) was added and the mixture was stirred at room temperature (30°C.) for 1 h. At the end of this time, it was diluted with EtOAc, washed with water, dried ($Na_2SO_4$) and concentrated under reduced pressure to give a mixture of **14a** and **14b** (1.1 : 1.0) which was separated by column chromatography using 2 to 10 % (gradient elution) ethyl acetate in petroleum ether to give **14a** (8.2 g, 41 %, as a syrupy liquid) and **14b** (8g, 40 %, as a semi solid).

**14a:** $^1H$ NMR ($CDCl_3$, 200 MHz): δ 1.2 (s, 3H), 1.5 - 2.05 (m, 6H), 2.09 - 2.3 (6s, 9H), 2.35 - 3.2 (m, 6H), 3.4 (m, 1H), 3.8 (m, 1H), 4.05 (m, 1H), 4.7 (s, 2H), 7.0 (m, 2H), 7.3 - 7.6 (m, 5H), 7.75 (m, 2H), 9.9 (s, 1H).

**14b:** $^1H$ NMR ($CDCl_3$, 200 MHz): δ 1.2, (s, 3H), 1.3 - 2.7 (m, 12H), 1.9 (s, 3H), 2.09 (s, 3H), 2.12 (s, 3H), 2.8 (m, 1H), 3.25 (m, 1H), 4.4 (m, 1H), 4.62 (s, 2H), 6.9 (m, 2H), 7.2 - 7.6 (m, 5H), 7.78 (m, 2H), 9.8 (s, 1H).

**Preparation 15**

**a) 3-[N-[(2RS)-6-Benzyloxy-2,5,7,8-tetramethylchroman-2-ylmethyl]-(2S)-pyrrolidine-2-methoxy]benzaldehyde (15):**

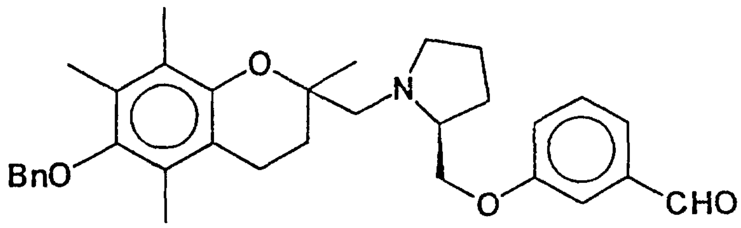

The title compound **15** (1.5 g, 35 %) was prepared as a thick liquid from N-[(2RS)-6-benzyloxy-2,5,7,8-tetramethylchroman-2-ylmethyl)-(3R)-chloropiperidine (3.5 g), obtained in preparation 7 and 3-hydroxybenzaldehyde (1.2 g) in a similar manner to that described in Method A of preparation 13. The crude compound was chromatographed on silica gel to get **15.**

$^1$H NMR ($CDCl_3$, 200 MHz): δ 1.21, 1.28 (2s, 3H), 1.50 - 2.05 (m, 6H), 2.11 - 2.23 (m, 9H), 2.35 - 3.25 (m, 6H), 3.40 (m, 1H), 3.85 (m, 1H), 4.05 (m, 1H), 4.70 (m, 2H), 7.10 - 7.60 (m, 9H), 9.97, 9.99 (2s, 1H).

**Preparation 16**

**4-[N-[(3R/S)-5-Benzyloxy-2,3-dihydro-2,2,4,6,7-pentamethylbenzofuran-3-ylmethyl]-(2S)-pyrrolidine-2-methoxy]benzaldehyde (16):**

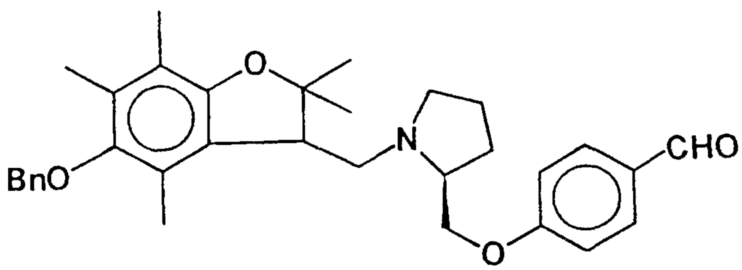

The title compound (1.6, 5g, 22 %) was prepared as an oil from N-[(3R/S)-5-benzyloxy-2,3-dihydro-2,2,4,6,7-pentamethyl-benzofuran-3ylmethyl]-(3R)-chloropiperidine (6.4 g) obtained in preparation 8 and 4-hydroxybenzaldehyde (1.83 g) in a similar manner to that described in, method A of preparation 14. The crude compound was chromatographed on silica gel to get 16.

$^1$H NMR ($CDCl_3$, 200 MHz): δ 1.35 (s, 3H), 1.64 (s, 3H), 1.80 (m, 4H), 2.12 (s, 3H), 2.21 (s, 3H), 2.30 (s, 3H), 2.45 (m, 2H), 2.78 (m, 1H), 3.15 (m, 2H), 3.35 (m, 1H), 3.89 (m, 1H), 4.08 (m, 1H), 4.75 (s, 2H), 6.96 (d, J = 10.4 Hz, 2H), 7.44 (m, 5H), 7.85 (d, J = 10.4 Hz, 2H), 9.94 (s, 1H).

**Preparation 17**

**3-[N-((3R/S)-5-Benzyloxy-2,3-dihydro-2,2,4,6,7-pentamethylbenzofuran-3-ylmethyl]-(2S)-pyrrolidine-2-methoxy]benzaldehyde:**

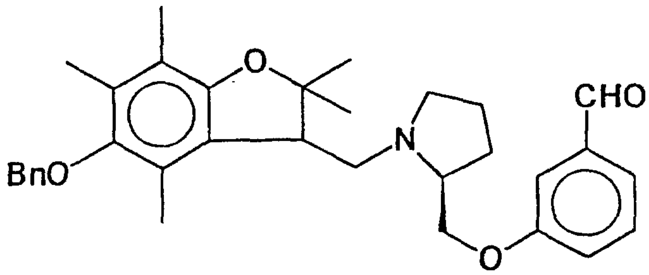

The title compound (0.8 g, 11 %) was prepared as an oil from N-[(R/S)-5-benzyloxy-2,3-dihydro-2,2,4,6,7-pentamethylbenzofuran-3-ylmethyl]-(3R)-chloropiperidine (6.0g) obtained in preparation 8 and 3-hydroxybenzaldehyde (1.71 g) in a similar manner to that described in method A of preparation 14. The crude compound was chromatographed on silica gel to get 17.

$[\alpha]_D^{21}$ = -81.7 (c, 1.24, $CHCl_3$)

$^1H$ NMR ($CDCl_3$, 200 MHz): δ 1.34 (s, 3H), 1.60 (s, 3H), 1.83 (m, 4H), 2.09 (s, 3H), 2.19 (8, 3H), 2.27 (s, 3H), 2.32 (m, 2H), 2.78 (m, 1H), 3.19 (m, 2H), 3.35 (m, 1H), 3.85 (dd, J = 9.0 and 6.0 Hz, 1H), 4.04 (dd, J = 9.0 and 5.0 Hz, 1H), 4.72 (s, 2H), 7.17 (m, 1H), 7.48 (m, 8H), 9.97 (s, 1H).

**Preparation 18**

**a) 4-[N-(5-Benzyloxy-2,4,6,7-tetramethylbenzofuran-3-ylmethyl)-(2S)-pyrrolidine-2-methoxy]]benzaldehyde (18a):**

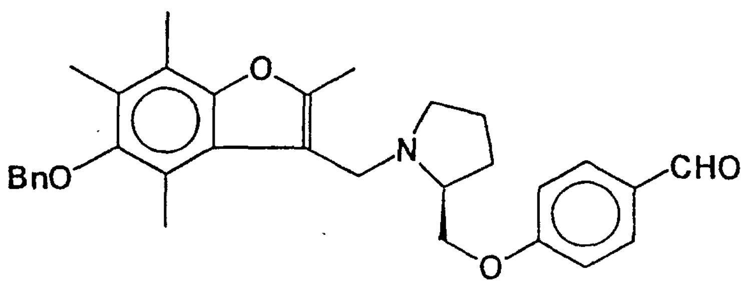

and

**b) 4-[N-[5-Benzyloxy-2,4,6,7-tetramethylbenzofuran-3-ylmethyl]-(3R)-piperidinyloxy] benzaldehyde (18b):**

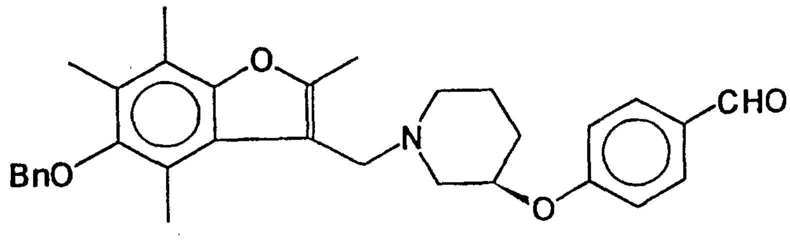

The title compounds **18a** and **18b** were prepared from N-(5-benzyloxy-2,4,6,7-tetramethylbenzofuran-3-ylmethyl)-(3R)-chloropiperidine (3 g) obtained in preparation 9 and 4-hydroxybenzaldehyde (0.889 g) in a similar manner to_that described in method A of preparation 14. The crude compound was chromatographed on silica gel to get **18a** (1.0 g, 27 %) as an oil and **18b** (0.89g, 22%) as a white solid, mp: 142-144°C.

**18a:** $[\alpha]_D^{22}$ = + 51.0 (c, 1.0, $CH_2Cl_2$)

$^1H$ NMR ($CDCl_3$, 200 MHz): δ 1.80 (m, 4H), 2.08 (m, 1H), 2.30 (s, 3H), 2.37 (s, 3H), 2.42 (s, 3H), 2.59 (s, 3H), 3.03 (m, 2H), 3.59 (d, J = 12.9 Hz, 1H), 3.79 (dd, J = 9.6 Hz and 6.8 Hz, 1H), 3.93 (dd, J = 9.6 Hz and 5.2 Hz, 1H), 4.05 (d, J = 12.9 Hz, 1H), 4.70 (s, 2H), 6.80 (d, J = 8.6 Hz, 2H), 7.48 (m, 5H), 7.77 (d, J = 8.6 Hz, 2H), 9.86 (s, 1H).

**18b:** $[\alpha]_D^{23}$ = - 57.5 (c, 0.2, $CH_2Cl_2$)

$^1H$ NMR ($CDCl_3$, 200 MHz): δ 1.74 (m, 4H), 2.18 (m, 2H), 2.29 (s, 3H), 2.32 (s, 6H), 2.62, (s, 3H), 2.73 (m, 1H), 3.05 (m, 1H), 3.45 (d, J = 5.4 Hz, 2H), 4.40 (m, 1H), 4.77 (s, 2H), 6.91 (d, J = 8.6 Hz, 2H), 7.49 (m, 5H), 7.75 (d, J = 8.7 Hz, 2H), 9.84 (s, 1H).

**Preparation 19**

**4-[N-[5-Benzyloxy-2-methylbenzofuran-3-ylmethyl]-(2S)-pyrrolidine-2-methoxy] benzaldehyde (19):**

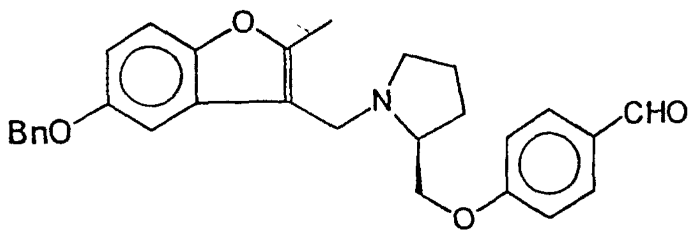

The title compound (0.25 g, 41 %) was prepared as an oil from N-(5-benzyloxy-2 methylbenzofuran-3-ylmethyl)-(3R)-chloropiperidine (0.5 g) obtained in preparation 10 and 4-hydroxybenzaldehyde (0.247 g) in a similar manner to that described in method A of preparation 14. The crude compound was chromatographed on silica gel to get 19.

$[\alpha]_D^{25}$ = -6.9 (c, 0.28, $CHCl_3$)

$^1H$ NMR ($CDCl_3$, 200 MHz): δ 1.73 (m, 3H), 2.04 (m, 1H), 2.32 (m, 1H), 2.41 (s, 3H), 2.99 (m, 2H), 3.60 (d, J = 13.3 Hz, 1H), 4.02 (m, 2H), 4.12 (d, J = 13.3 Hz, 1H), 4.90 (d, J = 4.1 Hz, 2H), 6.86 (m, 1H), 6.96 (d, J = 8.5 Hz, 2H), 7.36 (m, 7H), 7.77 (d, J = 8.6 Hz, 2H), 9.81 (s, 1H).

**Preparation 20**

**4-[N-[1-Methylindol-3-ylmethyl]-(2S)-pyrrolidine-2-methoxy]benzaldehyde:**

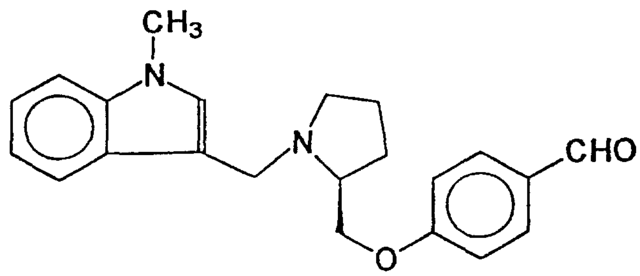

The title compound (0.16 g, 29 %) was prepared as a viscous oil from N-[1-methylindol-3-ylmethyl]-(2S)-pyrrolidine-2-methyl methanesulfonate (0.5 g), obtained in preparation 11 and 4-hydroxybenzaldehyde (0.2 g), in a similar manner to that described in method A of preparation 14.

$[\alpha]_D^{22}$ = -30.65 (c, 1.65, $CHCl_3$).

$^1H$ NMR ($CDCl_3$, 200 MHz): δ 1.5 (m, 4H), 3.04 (m, 1 H), 3.76 (m, 3H), 3.84 (s, 3H), 4.22 (m, 1H), 4.56 (m, 2H), 7.02 (d, J = 7.8 Hz, 2H), 7.34 (m, 4H), 7.66 (d, J = 7.8 Hz, 1H), 7.88 (m, 2H), 9.94 (s, 1H)

**Preparation 21**

**4-[2-[4-[(2RS)-6-Benzyloxy-2,5,7,8-tetramethylchroman-2-ylmethyl)piperazine-1-yl]ethoxy]benzaldehyde:**

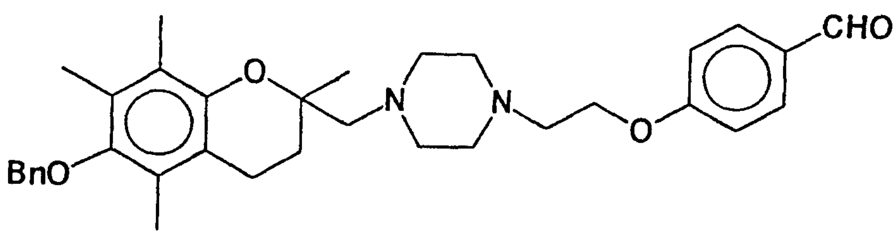

The title compound (0.2 g, 84 %) was prepared as a thick liquid from 2-[4-[(2RS)-6-benzyloxy-2,5,7,8-tetramethylchroman-2-ylmethyl)piperazin-1-yl]ethyl chloride (0.2 g), obtained in preparation 13 and 4-hydroxybenzaldehyde (0.06 g) in a similar manner to that described in method A of preparation 14.

$^1H$ NMR ($CDCl_3$, 200 MHz): δ 1.25 (s, 3H), 1.7 (m, 1H), 2.0 (m, 1H), 2.07 (s, 3H), 2.18 (s, 3H), 2.22 (s, 3H), 2.4 - 2.8 (m, 12H), 2.86 (t, J = 5.8 Hz, 2H), 4.2 (t, J = 5.8 Hz, 2H), 4.7 (s, 2H), 7.0 (d, J = 8.4 Hz, 2H), 7.3 - 7.6 (m, 5H), 7.83 (d, J = 8.4 Hz, 2H), 9.9 (s, 1H).

**Preparation 22**

**4-[2-[4-(1-Methylindol-3-ylmethyl)piperazin-1-yl]ethoxy]benzaldehyde:**

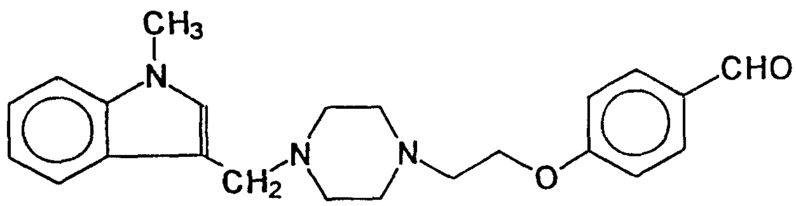

A suspension of 1-(l-methylindole-3-ylmethyl)piperazine (1.3 g) in dimethylsulfoxide (15 ml) and potassium hydroxide (0.63 g) was stirred for 30 min under nitrogen at 22°C. and then a solution of 4-[2-bromoethoxy]benzaldehyde (1.3 g) was added and stirring was continued for further 4 h. The reaction mixture was diluted with water and extracted with ethyl acetate and the organic layer was washed with water, brine, dried ($Na_2SO_4$) and solvent was evaporated under reduced pressure. The crude product was chromatographed on silica gel using mixture of methanol: chloroform (1:9) as eluent to afford the pure product (0.76 g, 51 %) as an oil.

$^1H$ NMR ($CDCl_3$, 200 MHz): δ 2.61 (bs, 8H), 2.83 (t, J = 5.72 Hz, 2H), 3.73 (s, 2H), 3.76 (s, 3H), 4.16 (t, J = 5.72 Hz, 2H), 6.98 (d, J = 8.5 Hz, 2H), 7.28 (m, 4H), 7.70 (d, J = 7.9 Hz, 1H), 7.81 (d, J = 8.5 Hz, 2H), 9.87 (s, 1H).

**Preparation 23**

**4-[N-(3,5-Dimethyl-4-methoxy-2-pyridyl methyl]-(2S)-pyrrolidine-2-methoxy]benzaldehyde:**

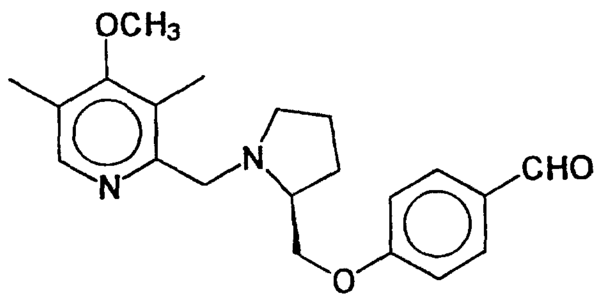

A solution of N-[(3,5-dimethyl-4-methoxy-2-pyridyl methyl)-(2S)-pyrrolidine-2-methanol (2.5 g) in 5 ml of dimethyl formamide (5 ml) was added dropwise while cooling to a suspension of (60 % w/w dispersion) sodium hydride (0.48 g) in dimethyl formamide (20 ml). The mixture was then stirred for 1 h at room temperature, after which 4-fluorobenzaldehyde (1.3 ml) in dimethyl formamide (5 ml) was added dropwise at room temperature. The reaction mixture was then stirred at 70°C. for 12 h. At the end of this time, water was added to the reaction mixture and extracted with EtOAc, dried and concentrated under reduced pressure to get 1.8 g (51 %) of the title compound. The crude product was used in the next step without further purification.

**Preparation 24**

**4-[N-[(2RS)-6-Benzyloxy-2,5,7,8-tetramethylchroman-2-ylmethyl]-(2S)-pyrrolidine-2-methoxy]nitrobenzene:**

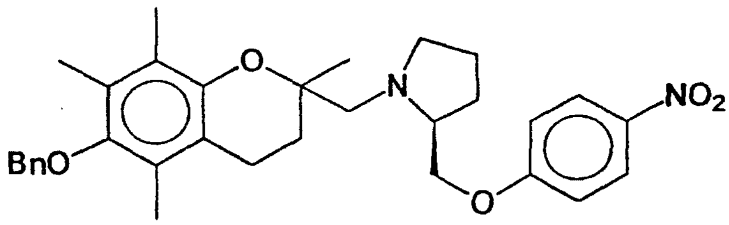

A solution of 16 g of the product obtained in preparation 1 in dimethyl formamide (100 ml) was added dropwise to a suspension of (50 % w/w dispersion) sodium hydride (2.81 g) in dimethyl formamide (50 ml). The mixture was then stirred at room temperature for 0.5 h, after which 1-fluoro-4-nitrobenzene (6.6 g) was added dropwise and the mixture was then stirred at the same temperature for 2 h. At the end of this time, water was added and the mixture was extracted with ethyl acetate, dried ($Na_2SO_4$), and the solvent was removed by distillation under reduced pressure to give 20 g of the crude compound which was chromatographed on silica gel using 10 to 20 % (gradient elution) of ethyl acetate in petroleum ether to afford 16.8 g (81%) of the title compound as a thick liquid.

$^1H$ NMR ($CDCl_3$, 200 MHz): δ 1.25 (m, 3H), 1.55 - 3.2 (complex, 21H), 3.4 (m, 1H), 3.85 (m, 1H), 4.1 (m, 1H), 4.7 (s, 2H), 6.9 (m, 2H), 7.3 - 7.6 (m, 5H), 8.2 (m, 2H).

**Preparation 25**

**4-[N-((3RS)-5-Benzyloxy-2,3-dihydro-2,2,4,6,7-pentamethylbenzofuran-3-ylmethyl)-(2S)-pyrrolidine-2-methoxy]nitrobenzene:**

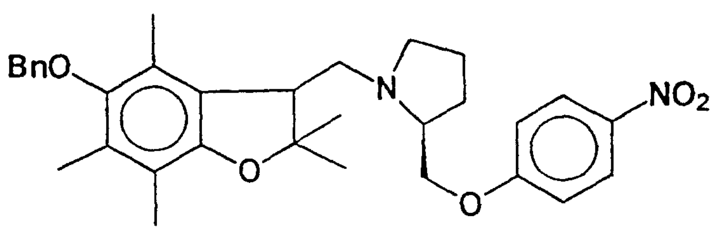

The title compound (7.25g, 67 %) was prepared as an oil from N-[(3RS)-5-benzyloxy-2,3-dihydro-2,2,4,6,7-pentamethylbenzofuran-3-ylmethyl]-(2S)-pyrrolidine-2-methanol (8.4g) obtained in preparation 2 and 4-fluoronitrobenzene (2.6 ml) by a similar procedure to that used in preparation 24.

$^1H$ NMR ($CDCl_3$, 200 MHz): δ 1.29 (s, 3H), 1.35 (s, 3H), 1.56 (s, 3H), 1.59 (s, 3H), 1.85 (m, 8H), 2.06 (s, 3H), 2.09 (s, 3H), 2.12 (s, 3H), 2.20 (s, 3H), 2.27 (s, 6H), 2.45 (m, 3H), 2.82 (m, 3H), 3.0 (m, 1H), 3.18 (m, 4H), 3.38 (m, 1H), 3.58 (m, 2H), 3.86 (m, 1H), 4.08 (m, 1H), 4.65 (d, J = 7.8 Hz, 2H), 4.72 (s, 2H), 6.74 (d, J = 9 Hz, 2H), 6.93 (d, J = 9 Hz, 2H), 7.48 (m, 10H), 8.13 (d, J = 9 Hz, 2H), 8.19 (d, J = 9 Hz, 2H).

**Preparation 26**

**4-[N-[5-Benzyloxy-2,4,6,7-tetramethylbenzofuran-3-ylmethyl]-(2S)-pyrrolidine-2-methoxy]nitrobenzene:**

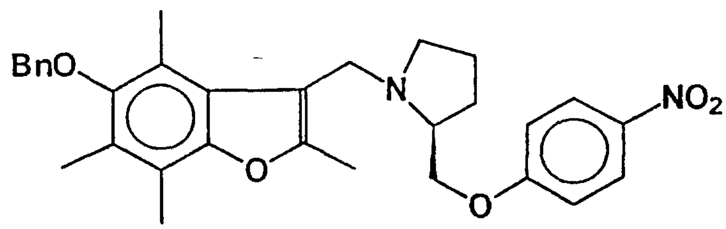

The title compound (25 g, 79 %) was prepared as an yellow solid from N-[5-benzyloxy-2,4,6,7-tetramethylbenzofuran-3-ylmethyl]-(2S)-pyrrolidine-2-methanol (24 g) obtained in preparation 3 and 4-fluoronitrobenzene (11.19 g) by a similar procedure to that used in preparation 24. mp. 136 -139°C.

$^1$H NMR ($CDCl_3$, 200 MHz): δ 1.78 (m, 4H), 2.06 (m, 1H), 2.32 (s, 3H), 2.38 (8, 3H), 2.43 (s, 3H), 2.59 (s, 3H), 3.03 (m, 2H), 3.63 (d, J = 12.8 Hz, 1H), 3.85 (m, 2H), 4.0 (d, J = 12.8 Hz, 1H), 4.72 (s, 2H), 6.72 (d, J = 9 Hz, 2H), 7.45 (m, 5H), 8.13 (d, J = 9 Hz, 2H).

**Preparation 27**

**4-[N-[(2RS)-6-Benzyloxy-2,5,7,8-tetramethylchroman-2ylmethyl]-(2S)-pyrrolidine-2-methoxy]aniline:**

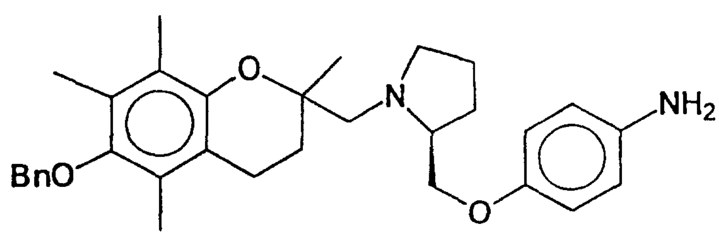

4-[N-[(2RS)-6-Benzyloxy-2,5,7,8-tetramethylchroman-2-ylmethyl]-(2S)-pyrrolidine-2-methoxy] nitrobenzene (5.8 g) obtained in preparation 24 was dissolved in EtOAc (30 ml) and was reduced with hydrogen (60 psi) in the presence of 10 % palladium on charcoal (0.6 g) at ambient temperature until hydrogen uptake (nearly 6 h) ceased. The solution was filtered through a bed of celite, the filter pad was washed exhaustively with EtOAc. The combined filtrate was evaporated to dryness under reduced pressure. The crude product was chromatographed on silica gel using 2 to 10 % (gradient elution) of methanol in chloroform to afford 5 g (91 %) of the title compound as a thick liquid.

$^1$H NMR ($CDCl_3$, 200 MHz): δ 1.2, 1.3 (2s, 3H), 1.5 - 3.2 (complex, 12 H), 2.1 (s, 3H), 2.15 (s, 3H), 2.2 (s, 3H), 3.4 (m, 1H), 3.75 (m, 1H), 3.9 (m, 1H), 4.7 (s, 2H), 6.7 (m, 4H), 7.4 (m, 5H).

**Preparation 28**

**4-[N-[(3RS)-5-Benzyloxy-2,3-dihydro-2,2,4,6,7-pentamethylbenzofuran-3-ylmethyl]-(2S)-pyrrolidine-2-methoxy]aniline:**

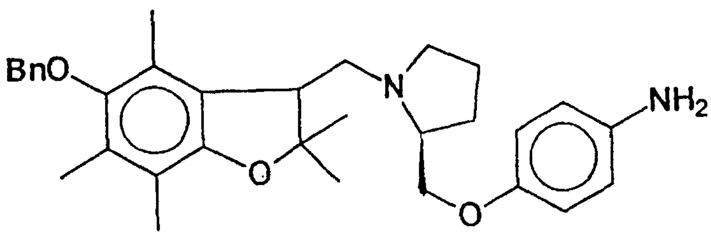

The title compound (5. 1 g, 55 %) was prepared as a brown oil from 4-[N-[(3RS)-5-benzyloxy-2,3-dihydro-2,2,4,6,7-pentamethylbenzofuran-3-ylmethyl]-(2S)-pyrrolidine-2-methoxy] nitrobenzene (9.8 g) obtained in preparation 25, by a similar procedure to that described in preparation 27.

$^1H$ NMR ($CDCl_3$, 200 MHz): δ 1.31, 1.33 (2s, 3H), 1.56, 1.61 (2s, 3H), 1.84 (m, 4H), 2.10 (s, 3H), 2.22 (s, 3H), 2.28 (s, 3H), 2.45 (m, 2H), 2.79 (m, 1H), 3.12 (m, 2H), 3.35 (m, 1H), 3.75 (m, 1H), 3.98 (m, 1H), 4.75 (m, 2H), 6.68 (m, 4H), 7.46 (m, 5H).

**Preparation 29**

**4-[N-[5-Benzyloxy-2,4,6,7-tetramethylbenzofuran-3-ylmethyl]-(2S)-pyrrolidine-2-methoxy]aniline:**

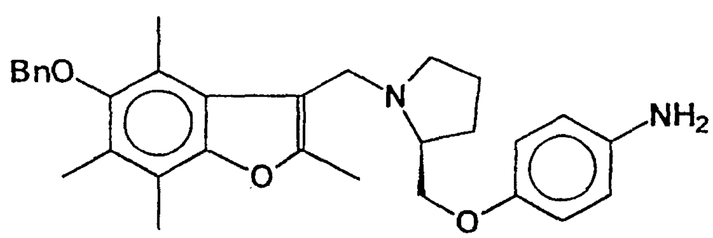

The title compound (3.3 g, 70 %) was prepared as a thick liquid from 4-[N-[5-benzyloxy-2,4,6,7-tetramethylbenzofuran-3-ylmethyl]-(2S)-pyrrolidine-2-methoxy]nitrobenzene (5g) obtained in preparation 26 by a similar procedure to that described in preparation 27.

$^1H$ NMR ($CDCl_3$, 200 MHz): δ 1.61 (m, 4H), 2.10 (m, 1H), 2.29 (s, 3H), 2.34 (s, 3H), 2.40 (s, 3H), 2.65 (s, 3H), 2.96 (m, 2H), 3.41 (bs, exchangeable with $D_2O$, 2H), 3.48 (d, J = 13 Hz, 1H), 3.65 (m, 1H), 3.85 (m, 1H), 4.12 (d, J = 13 Hz, 1H), 4.75 (s, 2H), 6.62 (s, 4H), 7.48 (m, 5H).

**Preparation 30**

**Ethyl-2-bromo-3-[4-[N-[(2RS)-6-benzyloxy-2,5,7,8-tetramethylchroman-2ylmethyl]-(2S)-pyrrolidine-2-methoxy]phenyl]propanoate:**

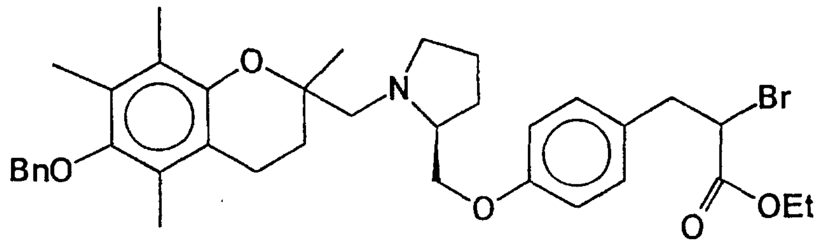

A solution of $NaNO_2$ (0.72 g) in $H_2O$ (1.3 ml) was added dropwise to a stirred and ice cooled mixture of 4-[N-[(2RS)-6-benzyloxy-2,5,7,8-tetramethylchroman-2ylmethyl]-(2S)-pyrrolidine-2-methoxy]aniline (4.8 g) obtained in preparation 27, aqueous HBr (48 %, 6.5 ml) MeOH (8.8 ml) and acetone (21 ml) below 5°C. The solution was stirred at 5°C. for 30 min and ethyl acrylate (6 ml) was added and the temperature was raised to 38°C. Powdered $Cu_2O$ (77 mg) was added in small portions to the vigorously stirred mixture. After the $N_2$ gas evolution has ceased, the reaction mixture was concentrated in vacuo. The residue was diluted with water, made alkaline with concentrated $NH_4OH$ and extracted with EtOAc. The EtOAc extract was washed with brine, dried ($Na_2SO_4$) and concentrated in vacuum. The crude product was chromatographed on silica gel using 10 - 20 % (gradient elution) of ethyl acetate in petroleum ether to afford 3.0 g (47 %) of the title compound as a thick liquid.

$^1H$ NMR ($CDCl_3$, 200 MHz): δ 1.2 (m, 6H), 1.55 - 3.5 (complex, 15H), 2.1 (s, 3H), 2.15 (s, 3H), 2.2 (s, 3H), 3.75 (m, 1H), 3.9 (m, 1H), 4.2 (m, 2H), 4.35 (m, 1H), 4.7 (s, 2H), 6.7 (m, 2H), 7.1 (m, 2H), 7.3 - 7.6 (m, 5H).

**Preparation 31**

**Ethyl 2-bromo-3[4-[N-[(3RS)-5-benzyloxy-2,3-dihydro-2,2,4,6,7-pentamethyl benzofuran-3-ylmethyl]-(2S)-pyrrolidine-2-methoxy] phenyl]propanoate:**

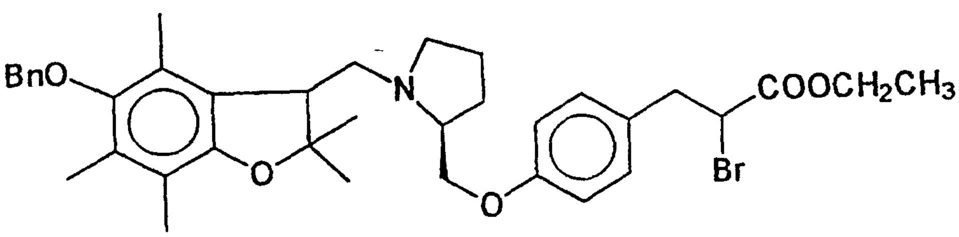

The title compound (2.5 g, 38 %) was prepared as an oil from 4-[N-[(3RS)-5-benzyloxy-2,3-dihydro-2,2,4,6,7-pentamethylbenzofuran-3-ylmethyl]-(2S)-pyrrolidine-2-methoxy]aniline (5 g), obtained in preparation 28, by a similar procedure to that described in preparation 30.

$^1H$ NMR ($CDCl_3$, 200 MHz): δ 1.26 (m, 6H), 1.58, 1.60 (2s, 3H), 1.76 (m, 4H), 2.08 (s, 3H), 2.22 (s, 3H), 2.28 (s, 3H), 2.42 (m, 2H), 2.78 (m, 1H), 2.99 (m, 1H), 3.15 (m, 2H), 3.32 (m, 1H), 3.56 (m, 1H), 3.72 (m, 1H), 3.96 (m, 1H), 4.15 (q, J = 6 Hz, 2H), 4.33 (m, 1H), 4.71 (m, 2H), 6.68 (d, J = 8 Hz, 1H), 6.76 (d, J = 8 Hz, 1H), 7.10 (m, 2H), 7.45 (m, 5H).

**Preparation 32**

**Ethyl 2-bromo-3-[4-[N-[5-benzyloxy-2,4,6,7-tetramethylbenzofuran-3-ylmethyl]-(2S)-pyrrolidine-2-methoxy] phenyl]propanoate:**

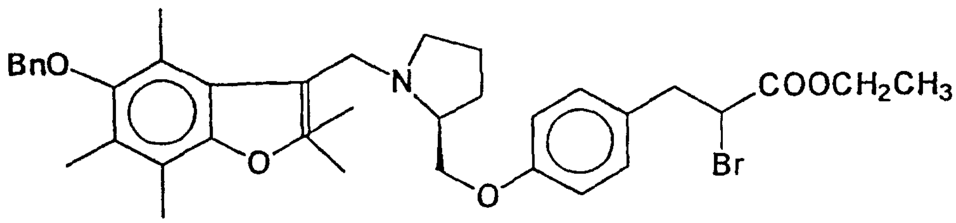

The title compound (2.8 g, 14 %) was prepared as a viscous oil from 4-[N-[5-benzyloxy- 2,2,4,6,7-tetramethylbenzofuran-3-ylmethyl]-(2S)-pyrrolidine-2-methoxy]aniline (15 g), obtained in preparation 29, by a similar procedure to that described in preparation 30.

$^1$H NMR ($CDCl_3$, 200 MHz): δ 1.26 (t, J = 7 Hz, 3H), 1.79 (m, 4H), 2.05 (m, 1H), 2.31 (s, 3H), 2.39 (s, 3H), 2.42 (s, 3H), 2.65 (s, 3H), 2.98 (m, 2H), 3.21 (m, 1H), 3.40 (m, 1H), 3.58 (d, J = 12 Hz, 1H), 3.74 (m, 1H), 3.91 (m, 1H), 4.21 (m, 4H), 4.76 (s, 2H), 6.73 (d, J = 7.7 Hz, 2H), 7.10 (d, J = 7.7 Hz, 2H), 7.48 (m, 5H).

**Example 1**

**5-[4-[N-[(2RS)-6-Benzyloxy-2,5,7,8-tetramethylchroman-2-ylmethyl)]-(2S)-pyrrolidine-2-methoxy]phenyl methylene]thiazolidine-2,4-dione:**

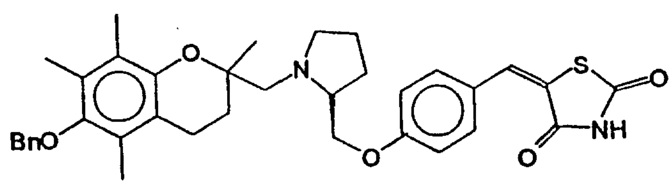

A solution of 4-[N-[(2RS)-6-benzyloxy-2,5,7,8-tetramethylchroman-2-ylmethyl]-(2S)-pyrrolidine-2-methoxy] benzaldehyde (1.2 g) obtained in preparation 14 and 2,4-thiazolidinedione (0.27 g) in toluene (30 ml) containing piperidine (30 mg) and benzoic acid (37 mg) was heated at reflux for 2 h using Dean-Stark apparatus. The reaction mixture was cooled, diluted with EtOAc and filtered, the filtrate was washed with $H_2O$, dried ($Na_2SO_4$) and evaporated under reduced pressure (1.4 g, 100 %).

**Preparation of polymorphs**

**Form 1**

The product (0.5 g) obtained in example 1 was dissolved in 5 ml of EtOAc and the solution was heated at 80°C. At this temperature 2 ml of pet. ether was added dropwise and the resulting solution was filtered through a cotton plug and allowed to stay at 0°C. for 12 h. The resulting solid (220 mg, 44 %) as pale yellow granules was filtered, washed with petroleum ether and dried under vacuum. mp 186.6°C.

$^1$H NMR ($CDCl_3$, 200 MHz): δ identical with that of Form 3.

Form 2

The compound (500 mg) obtained in example 1 was taken in a single neck RB flask equipped with $N_2$ and inlet was heated at 100°C. until the solid melts (5 min). Then the RB flask was immersed in an ice bath to get a deep yellow amorphous solid (500 mg, 100 %) mp: 88°C.

$^1$H NMR ($CDCl_3$, 200 MHz): δ identical with that of Form 3.

Form 3

The crude product obtained above was chromatographed on silica gel using 0 to 0.5 % (gradient elution) of methanol in chloroform to afford 1.3 g (93 %) of the title compound as a pale yellow fluffy solid. mp. 86°C.

$[\alpha]_D^{24}$ = - 17.3 (c, 1.0, $CDCl_3$)

$^1$H NMR ($CDCl_3$, 200 MHz): δ 1.21, 1.26 (2s, 3H), 1.5 - 2.05 (m, 6H), 2.08 - 2.22 (6s, 9H), 2.35 - 3.15 (m, 6H), 3.4 (m, 1H), 3. 8 (m, 1H), 4.0 (m, 1H), 4.7 (s, 2H), 6.95 (m, 2H), 7.3 - 7.6 (m, 7H), 7.8 (s, 1 H).

**Example 2**

**5-[4-[N-[(2RS)-6-Benzyloxy-2,5,7,8-tetramethylchroman-2-ylmethyl)]-(2S)-pyrrolidine-2-methoxy]phenyl methylene]thiazolidine-2,4-dione, maleate:**

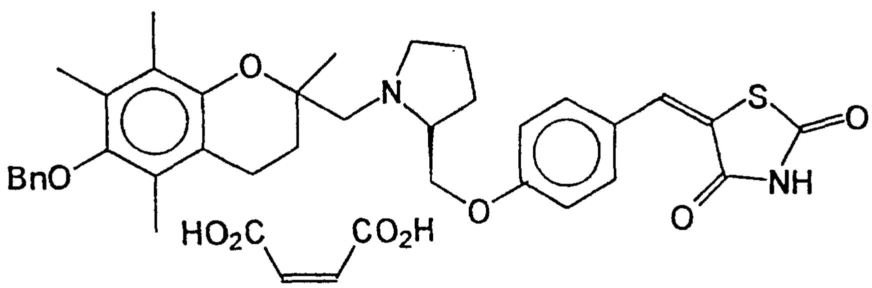

To a solution of 5-[4-[N-[(2RS)-6-benzyloxy-2,5,7,8-tetramethylchroman-2-ylmethyl)]-(2S)-pyrrolidine-2-methoxy]phenyl methylene]thiazolidine-2,4-dione (250 mg), obtained in example 1, in dry $Et_2O$ (5 ml) at room temperature, maleic acid (47 mg) in $Et_2O$ (5 ml) was added. The reaction mixture was stirred for an additional 30 min. and the $Et_2O$ layer was decanted. The resulting solid was washed twice with $Et_2O$ (2 x 5 ml) and dried under reduced pressure over $P_2O_5$ for 6 h to get a pale yellow solid (220 mg, 74 %). mp: 210°C.

$[\alpha]_D^{27}$ = 27.0 (c, 1.0, $CHCl_3$)

$^1$H NMR ($CDCl_3$, 200 MHz): δ 1.25, 1.3 (2s, 3H), 1.95, 2.0,2.1 (3s, 9H), 1.5 - 4.5 (complex m, 15 H), 4.65 (2s, 2H), 6.1 (s, 2H), 7.0 - 7.7 (m, 9H), 7.8 (s, 1H).

**Example 3**

**5-[4-[N-[(2RS)-6-Benzyloxy-2,5,7,8-tetramethylchroman-2-ylmethyl]-(2S)-pyrrolidine-2-methoxy]phenyl methylene]thiazolidine-2,4-dione, sodium salt:**

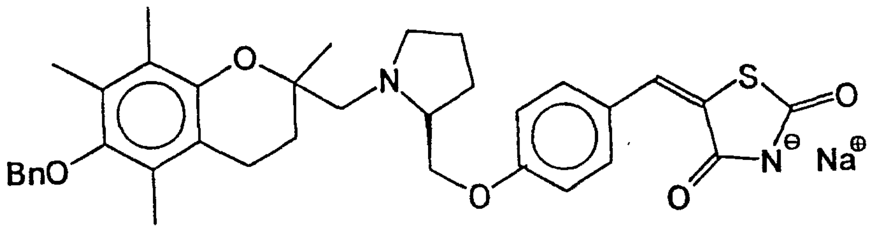

To a solution of 5-[4-[N-[(2RS)-6-benzyloxy-2,5,7,8-tetramethylchroman-2-ylmethyl]-(2S)-pyrrolidine-2-methoxy]phenyl methylene]thiazolidine-2,4-dione (250 mg), obtained in example 1, in dry $Et_2O$ (15 ml) at room temperature, NaOMe in MeOH [prepared in situ by dissolving Na (13 mg) in MeOH (1 ml)] was added. The reaction mixture was stirred at room temperature for 30 min and the supernatant solvent was decanted. The resulting solid was washed twice with $Et_2O$ (2 x 5 ml) and dried over $P_2O_5$ under reduced pressure for 6 h to get a pale yellow solid (235 mg, 65 %), mp : 245°C.

$[\alpha]_D^{27}$ = -9.3 (c, 0.82, $CHCl_3$).

$^1H$ NMR ($CDCl_3$, 200 MHz): δ 1.15, 1.2 (2s, 3H), 2.05, 2.1, 2.15 (3s, 9H), 1.5 - 3.6 (complex m, 13 H), 3.8 (m, 1H), 4.0 (m, 1H), 4.6 (s, 2H), 7.0 (m, 2H), 7.5 (m, 8H).

**Example 4**

**5-[4-[N-[(2RS)-6-Benzyloxy-2,5,7,8-tetramethylchroman-2-ylmethyl]-(2S)-pyrrolidine-2-methoxy]phenyl methylene]thiazolidine-2,4-dione, hydrochloride:**

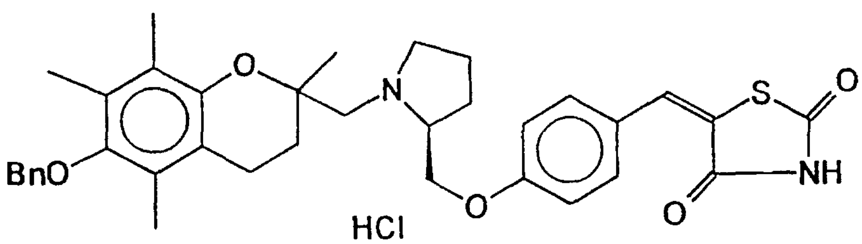

To a solution of 5-[4-[N-[(2RS)-6-benzyloxy-2,5,7,8-tetramethylchroman-2-ylmethyl]-(2S)-pyrrolidine-2-methoxy]phenyl methylene]thiazolidine-2,4-dione (300 mg) obtained in example 1 in $Et_2O$ (15 ml) at 0°C., HCl gas was bubbled for 30 min. The resulting solution was stirred for an additional 30 min; the supernatant liquid was decanted and the resulting solid was washed with $Et_2O$ (2 x 5 ml) and dried under reduced pressure over $P_2O_5$ for 6 h to get a pale yellow solid (200 mg, 75 %). mp. 212-214°C.

$[\alpha]_D^{27}$ = -25.5 (c, 0.4, $CHCl_3$).

$^1H$ NMR (DMSO-$d_6$, 200 MHz): δ 1.25,1.35 (2s, 3H), 1.99, 2.03, 2.12 (3s, 9H), 1.05 - 4.6 (complex m, 15H), 4.6,4.62 (2s, 2H), 7.0 - 7.7 (m, 9H), 7.8 (s, 1H), 9.6 (bs, 1H, exchangeable with $D_20$).

**Example 5**

**5-[4-[N-[(2RS)-6-Benzyloxy-2,5,7,8-tetramethylchroman-2-ylmethyl]-(2S)-pyrrolidine-2-methoxy]phenyl methyl]thiazolidine-2,4-dione:**

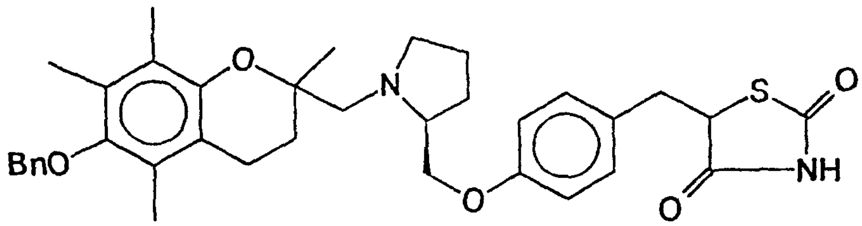

**Method A:**

To a stirred suspension of the product obtained in example 1 (0.5 g) in methanol (20 ml) at room temperature was added magnesium turnings (0.36 g) and the reaction mixture was stirred at the same temperature for 1.5 h. The reaction mixture was added to ice water (10 ml), the pH was adjusted to 6.5 - 7.0 using 10 % aqueous hydrochloric acid and the solution was extracted with chloroform (3 x 25 ml). The combined organic extract was washed with $H_2O$, dried ($CaCl_2$) and the solvent was removed under reduced pressure. The residual mass was chromatographed on silica gel using 0.5 % methanol in chloroform to give 0.46 g (92 %) of the title compound, mp: 62 - 64°C.

Method B:

A mixture of ethyl 2-bromo-3-[4-[N-[(2RS)-6-benzyloxy-2,5,7,8-tetramethylchroman-2-ylmethyl]-(2S)-pyrrolidine-2-methoxy]phenyl]propanoate (7 g), obtained in preparation 29, thiourea (1.6 g), NaOAc (1.73 g) and EtOH (42 ml) was stirred under reflux for 5 h. The reaction mixture was cooled and extracted with EtOAc, dried ($Na_2SO_4$) and concentrated to get 2-imino-5-[4-[N-[(2RS)-6-benzyloxy-2,5,7,8-tetramethylchroman-2-ylmethyl]-(2S)-pyrrolidine-2-methoxy]benzyl]-4-thiazolidinone which was used in the next step without further purification.

A mixture of the above crude product, 2N HCl (60 ml) and EtOH (60 ml) was stirred under reflux for 12 h. The reaction mixture was concentrated in vacuo. The residue was diluted with water, neutralized with saturated aqueous $NaHCO_3$ and extracted with ethyl acetate. The EtOAc extract was washed with brine, dried ($Na_2SO_4$) and concentrated in vacuo. The residue was chromatographed on silica gel with 40 % EtOAc in pet. ether as eluent to afford the title compound (5.5 g, 85 %) as a fluffy solid, mp 62 - 64°C.

$[\alpha]_D^{27}$ = -26.4 (c, 1.0, $CHCl_3$).

$^1H$ NMR ($CDCl_3$, 200 MHz): δ 1.25 (m, 3H), 1.5 - 2.05 (m, 6H), 2.1 - 2.2 (m, 9H), 2.3 - 3.25 (m, 7H), 3.4 (m, 2H), 3.75 (m, 1H), 3.95 (m, 1H), 4.5 (m, 1H), 4.7 (s, 2H), 6.8 (m, 2H), 7.15 (m, 2H), 7.3 - 7.6 (m, 5H).

**Example 6**

**5-[4-[N-[(2RS)-6-Benzyloxy-2,5,7,8-tetramethylchroman-2-ylmethyl]-(2S)-pyrrolidine-2-methoxy]phenyl methyl]thiazolidine-2,4-dione, Maleate:**

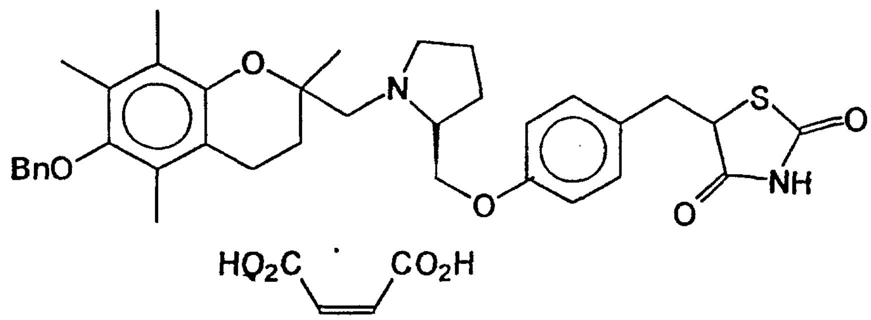

The title compound (0.28 g, 94 %) was prepared as a pale yellow solid from 5-[4-[N-[(2RS)-6-benzyloxy-2,5,7,8-tetramethylchroman-2-ylmethyl]-(2S)-pyrrolidine-2-methoxy]phenyl methyl]thiazolidine-2,4-dione (0.25 g), obtained in example 5 by an analogous procedure to that described in example 2, mp: 180°C.

$[\alpha]_D^{27}$ = 19.4 (c, 0.66, $CHCl_3$).

$^1$H NMR ($CDCl_3$, 200 MHz): δ 1.2, 1.25 (2s, 3H), 2.0, 2.05, 2.1 (3s, 9H), 1.5 - 4.5 (complex m, 17H), 4.6 (s, 2H), 4.9 (m, 1H), 6.1 (s, 2H), 6.9 (m, 2H), 7.2 (m, 2H), 7.5 (m, 5H), 12.1 (bs, 1H, exchangeable with $D_2O$).

**Example 7**

**5-[4-[N-(2RS)-6-Benzyloxy-2,5,7,8,-tetramethylchroman-2-ylmethyl]-(2S)-pyrrolidine-2-methoxy]phenyl methyl]thiazolidine-2,4-dione, sodium salt:**

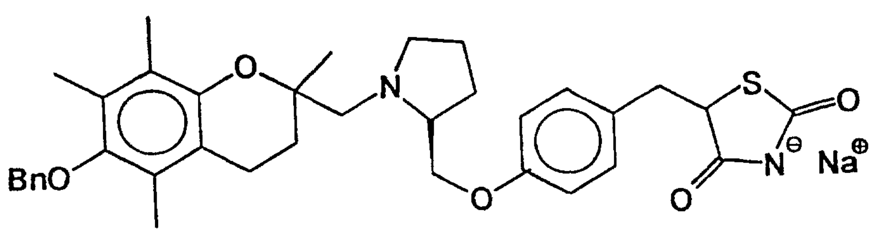

The title compound (0.27 g, 75 %) was prepared as a white solid from 5-[4-[N-[(2RS)-6-benzyloxy-2,5,7,8-tetramethylchroman-2-ylmethyl]-(2S)-pyrrolidine-2-methoxy]phenyl methyl] thiazolidine-2,4-dione (0.35 g), obtained in example 5, by an analogous procedure to that described in example 3, mp : 191°C.

$[\alpha]_D^{27}$ = -23.1 (c, 1.0, $CHCl_3$).

$^1$H NMR ($CDCl_3$, 200 MHz): δ 1.5, 1.25 (2s, 3H), 2.0 (s, 3H), 2.1 (s, 6H), 1.4-4.0 (complex m, 17H), 4.1 (m, 1H), 4.6 (s, 2H), 6.8 (m, 2H), 7.1 (m, 2H), 7.5 (m, 5H).

**Example 8**

**5-[4-[N-[(2RS)-6-Benzyloxy-2,5,7,8-tetramethylchroman-2-ylmethyl]-(2S)-pyrrolidine-2-methoxy]phenyl methyl]thiazolidine-2,4-dione, Hydrochloride:**

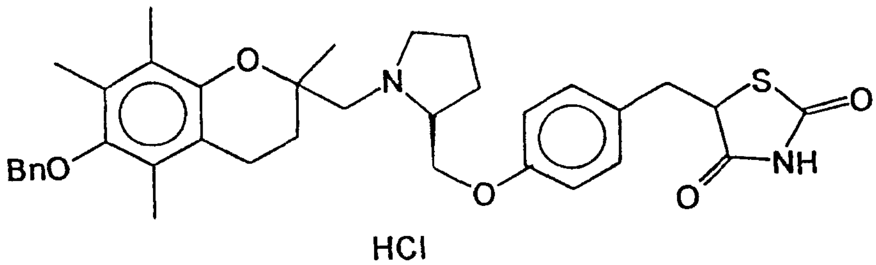

HCl

The title compound (0.18 g, 86 %) was prepared as a pale yellow solid from 5-[4-[N-[(2RS)-6-benzyloxy-2,5,7,8-tetramethylchroman-2-ylmethyl]-(2S)-pyrrolidine-2-methoxy]phenyl methyl] thiazolidine-2,4-dione (0.2 g), obtained in example 5, by an analogous procedure to that described in example 4, mp: 230°C.

$[\alpha]_D^{27}$ = -9.5 (c, 1.0, $CHCl_3$).

$^1H$ NMR ($CDCl_3$, 200 MHz): δ 1.30, 1.4 (2s, 3H), 2.0, 2.05, 2.2 (3s, 9H), 1.5 - 4.5 (complex m, 17H), 4.6 (s, 2H), 4.9 (m, 1H), 6.9 (m, 2H), 7.2 (m, 2H), 7.5 (m, 5H), 9.8 (bs, 1H, exchangeable with $D_2O$), 12.1 (bs, 1H, exchangeable with $D_2O$).

**Example 9**

**a) 5-[3-[N-[(2RS)-6-Benzyloxy-2,5,7,8-tetramethylchroman-2-ylmethyl]-(2S)-pyrrolidine-2-methoxy]phenyl methylene]thiazolidine-2,4-dione (9a):**

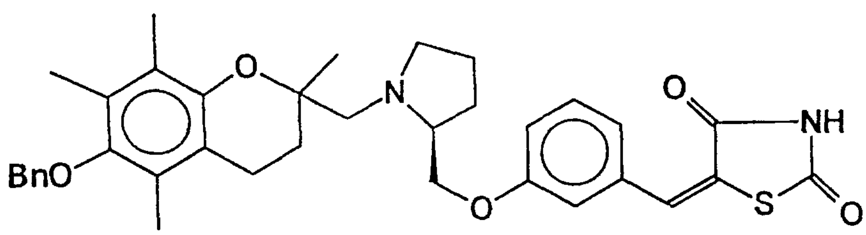

**and**

**b) 5-[3-[N-[(2RS)-6-Benzyloxy-2,5,7,8-tetramethylchroman-2-ylmethyl]-(3R)-piperidinyloxy]phenyl methylene] thiazolidine-2,4-dione (9b):**

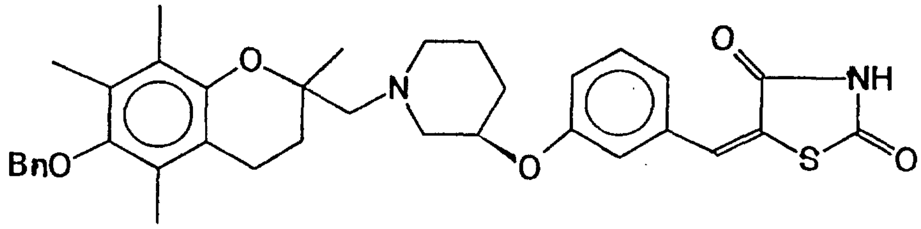

The title compounds **9a** and **9b** (4.5 g, 94 %) were prepared from the crude mixture obtained in preparation 15, by a similar procedure to that described in example 1 and the crude product was separated by column chromatography on silica gel using 0.5 % methanol in chloroform to afford **9a** (1.5 g, 31 %, gummy solid) and **9b** (2.0 g, 42 %, gummy solid).

**9a:** $^1$H NMR ($CDCl_3$, 200 MHz): δ 1.21,1.27 (2s, 3H), 1.5 - 2.05 (m, 6H), 2.05 - 2.2 (m, 9H), 2.3 - 3.2 (m, 6H), 3.4 (m, 1H), 3.8 (m, 1H), 4.05 (m, 1H), 4.7 (s, 2H), 7.0 (m, 4H), 7.25 - 7.6 (m, 5H), 7.77,7.81 (2s, 1H).

**9b:** $^1$H NMR ($CDCl_3$, 200 MHz): δ 1.21 (s, 3H), 1.3 - 2.8 (m, 12H), 1.91 (s, 3H), 2.16 (s, 6H), 3.1 (m, 1H), 3.55 (m, 1H), 4.4 (m, 1H), 4.7 (s, 2H), 6.85 - 7.55 (m, 9H), 7.78 (s, 1H).

**Example 10**

**5-[4-[N-(2RS)-6-Benzyloxy-2,5,7,8-tetramethylchroman-2-ylmethyl]-(3R)-piperidinyloxy] phenyl methylene] thiazolidine-2,4-dione:**

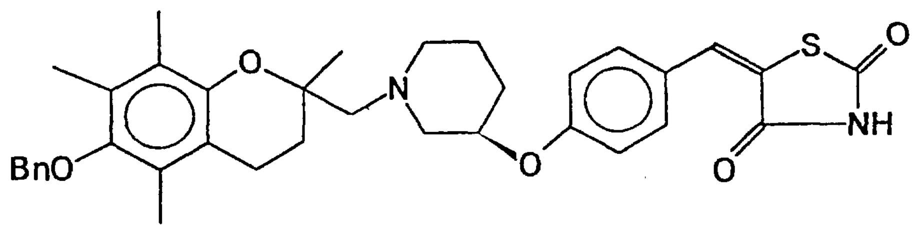

The title compound (1 g, 57 %) was prepared as a pale yellow solid from 4-[N-[(2RS)-6-benzyloxy-2,5,7,8-tetramethylchroman-2-ylmethyl]-(3R)-piperidinyloxy]benzaldehyde (**14b**, 1.5 g), obtained in preparation 14 by a similar procedure to that described in example 1. mp: 142°C.

$^1$H NMR ($CDCl_3$, 200 MHz): δ 1.22 (s, 3H), 1.3 - 1.90 (m, 6H), 2.0 (s, 3H), 2.19 (s, 6H), 1.95-2.8 (m, 6H), 2.9 (m, 1H), 3.25 (m, 1H), 4.4 (m, 1H), 4.69 (s, 2H), 6.95 (m, 2H), 7.3 - 7.6 (m, 7H), 7.78 (s, 1H).

**Example 11**

**5-[4-[N-(5-Benzyloxy-2,3-dihydro-2,2,4,6,7-pentamethylbenzofuran-3-(R or S)-ylmethyl)-(2S)-pyrrolidine-2-methoxy]phenyl methylene]thiazolidine-2,4-dione:**

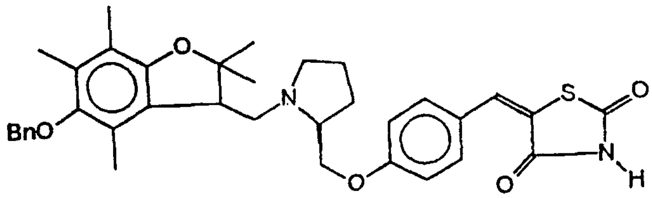

The title compound (0.8 g, 67 %) was prepared as a pale yellow solid from 4-[N-[(3R/S)-5-benzyloxy-2,3-dihydro-2,2,4,6,7-pentamethylbenzofuran-3-ylmethyl)-(2S)-pyrrolidine-2-methoxy]benzaldehyde (1.0 g) obtained from preparation 16 by a similar procedure to that described in example 1, mp 68 - 70°C.

$[\alpha]_D^{24}$ = -201.5 (c, 1.0, $CHCl_3$).

$^1H$ NMR ($CDCl_3$, 200 MHz): δ 1.32 (s, 3H), 1.58 (s, 3H), 1.82 (m, 4H), 2.08 (s, 3H), 2.18 (s, 3H), 2.25 (s, 3H), 2.42 (m, 2H), 2.76 (m, 1H), 3.14 (m, 2H), 3.35 (m, 1H), 3.84 (m, 1H), 4.02 (m, 1H), 4.71 (s, 2H), 6.95 (d, J = 8.7 Hz, 2H), 7.45 (m, 7H), 7.80 (s, 1H).

**Example 12**

**5-[4-[N-[(3RS)-5-Benzyloxy-2,3-dihydro-2,2,4,6,7-pentamethylbenzofuran-3-ylmethyl]-(3R)-piperidinyloxy] phenyl methylene]thiazolidine-2,4-dione (12):**

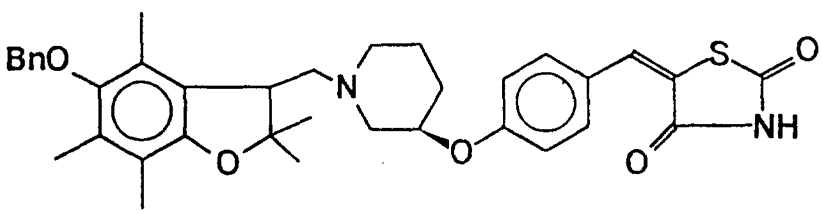

The title compound (0.75 g, 42 %) was prepared as a pale yellow solid from the crude mixture (1.5 g) obtained in preparation 16 in a similar manner to that described in example 1. The crude product was chromatographed on silica gel to get **12**. mp 150 -152°C.

$[\alpha]_D^{24}$ = + 87.1 (c, 1.0, $CHCl_3$)

$^1H$ NMR ($CDCl_3$, 200 MHz): δ 1. 13, 1.37 (2s, 3H), 1.43 - 1.69 (complex m, 5H), 1.88 (m, 2H), 2.09 (s, 3H), 2.11 (s, 3H), 2.19 (s, 3H), 2.36 (m, 2H), 2.63 (m, 2H), 2.91 (m, 1H), 3.09 (m, 2H), 4.42 (m, 1H), 4.71 (s, 2H), 6.95 (m, 2H), 7.45 (m, 7H), 7.81 (s, 1H).

**Example 13**

**5-[4-[N-[3(RS)-5-Benzyloxy-2,3-dihydro-2,2,4,6,7-pentamethylbenzofuran-3-ylmethyl]-(2S)-pyrrolidine-2-methoxy]phenyl methylene]thiazolidine-2,4-dione maleate:**

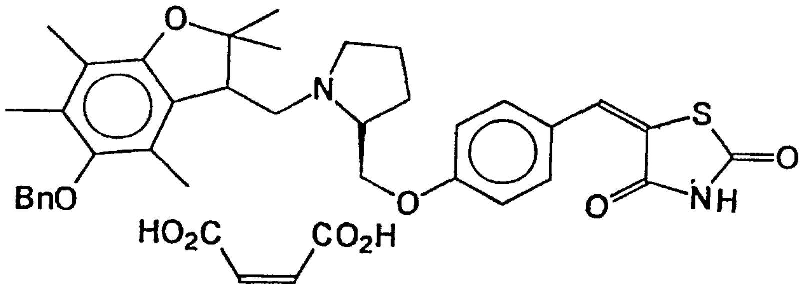

The title compound (0.11 g, 92 %) was prepared as a yellow solid from 5-[4-[N-[(3R/S)-5- benzyloxy-2,3-dihydro-2,2,4,6,7-pentamethylbenzofuran-3-ylmethyl]-(2S)-pyrrolidine-2-methoxy]phenyl methylene]thiazolidine-2,4-dione (0. 1 g) obtained in example 11 by an analogous procedure to that described in example 2, mp : 158 - 161°C.

$^1$H NMR ($CDCl_3$, 200 MHz): δ 1.37 (s, 3H), 1.63 (s, 3H), 1.95 (m, 4H), 2.10 (s, 3H), 2.22 (s, 3H), 2.27 (s, 3H), 2.63 (m, 2H), 3.16 (m, 1H), 3.30 (m, 2H), 3.52 (m, 1H), 3.85 (m, 1H), 4.03 (m, 1H), 4.72 (s, 2H), 6.28 (s, 2H), 7.03 (d, J = 8.4 Hz, 2H), 7.48 (m, 7H), 7.79 (s, 1H).

**Example 14**

**5-[4-[N-[(3R/S)-5-benzyloxy-2,3-dihydro-2,2,4,6,7-pentamethylbenzofuran-3-ylmethyl]-(2S)-pyrrolidine-2-methoxy]phenyl methylene]thiazolidine-2,4-dione, Hydrochloride:**

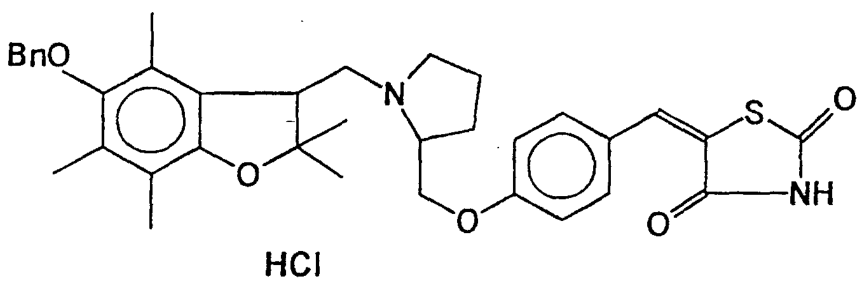

The title compound (0.27 g, 85 %) was prepared as a colorless solid from 5-[4-[N-(3R/S)-5-benzyloxy-2,3-dihydro-2,2,4,6,7-pentamethylbenzofuran-3-ylmethyl]-(2S)-pyrrolidine-2-methoxy] phenyl methylene]thiazolidine-2,4-dione (0.3 g) obtained in example 11 by an analogous procedure to that described in example 4, mp : 218 - 224°C.

$^1$H NMR ($CDCl_3$, 200 MHz): δ 1.45 (s, 3H),1.60 (s, 3H), 1.80 (m, 4H), 2.08 (s, 3H), 2.19 (s, 3H), 2.26 (s, 3H), 2.50 (m, 2H), 2.90 (m, 1H), 3.25 (m, 2H), 3.40 (m, 1H), 4.12 (m, 1H), 4.30 (m, 1H), 4.71 (s, 2H), 7.08 (d, J = 8.6 Hz, 2H), 7.49 (m, 7H), 7.76 (s, 1H).

**Example 15**

**a) 5-[4-[N-[(3RS)-5-Benzyloxy-2,3-dihydro-2,2,4,6,7-pentamethylbenzofuran-3-ylmethyl]-(2S)-pyrrolidine-2-methoxy]phenyl methyl]thiazolidine-2,4-dione (15a):**

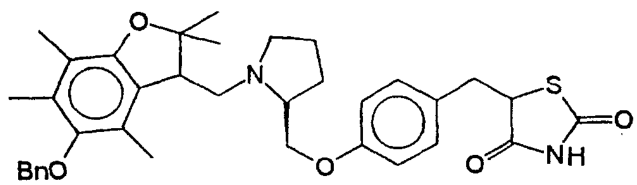

**and**

**b) 5-[4-[N-[(3RS)-2,3-Dihydro-5-hydroxy-2,2,4,6,7-pentamethylbenzofuran-3-ylmethyl]-(2S)-pyrrolidine-2-methoxy]phenyl methyl]thiazolidine-2,4-dione (15b):**

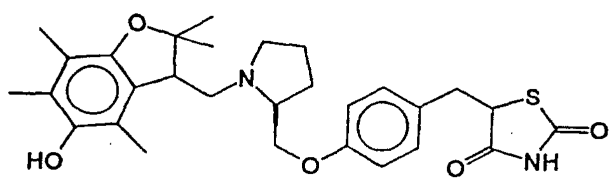

The title compounds **15a** and **15b** were prepared from ethyl 2-bromo-3-[4-[N-[(3RS)-5-benzyloxy-2,3-dihydro-2,2,4,6,7-pentamethylbenzofuran-3-ylmethyl]-(2S)-pyrrolidine-2-methoxy] phenyl]propanoate (2.5 g) obtained in preparation 31 by a similar procedure to that described in method B of example 5. The crude compound was chromatographed on silica gel to get **15a** as a colorless fluffy solid (1.74 g, 75 %) and **15b** as a fluffy solid (0.4 g, 20 %)

**15a** : mp: 107 - 109°C.

$[\alpha]_D^{29}$ = - 79.5 (c, 1.0, $CHCl_3$)

$^1H$ NMR ($CDCl_3$, 200 MHz): δ 1.30,1.34 (2s, 3H), 1.57,1.60 (2s, 3H), 1.79 (m, 4H), 2.09 (s, 3H), 2.19 (s, 3H), 2.26 (s, 3H), 2.45 (m, 2H), 2.78 (m, 1H), 3.03 (m, 1H), 3.14 (m, 2H), 3.38 (m, 1H), 3.55 (m, 1H), 3.74 (m, 1H), 3.98 (m, 1H), 4.46 (m, 1H), 4.70 (m, 2H), 6.78 (m, 2H), 7.10 (m, 2H), 7.42 (m, 5H).

**15b** : mp. 72 - 75°C.

$[\alpha]_D^{29}$ = - 84.2 (c, 1.0, $CHCl_3$).

$^1H$ NMR ($CDCl_3$, 200 MHz): δ 1.26,1.30 (2s, 3H), 1.53,1.58 (2s, 3H), 1.78 (m, 4H), 2.08 (s, 3H), 2.11 (s, 3H), 2.17, 2.20 (2s, 3H), 2.36 (m, 2H), 2.72 (m, 1H), 2.98 (m, 1H), 3.13 (m, 2H), 3.45 (m, 2H), 3.75 (m, 1H), 3.98 (m, 1H), 4.19 (s, 1H), 4.50 (dd, J = 9.4, 3.8 Hz, 1H), 6.76 (m, 2H), 7.12 (m, 2H).

**Example 16**

**5-[4-[N-[(3RS)-5-Benzyloxy-2,3-dihydro-2,2,4,6,7-pentamethylbenzofuran-3-ylmethyl]-(2S)-pyrrolidine-2-methoxy]phenyl methyl]thiazolidine-2,4-dione, maleate:**

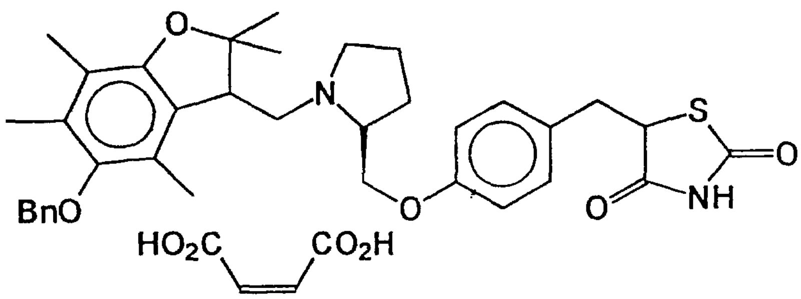

The title compound (0.44 g, 79 %) was prepared as a pale yellow solid from 5-[4-[N-[(RS)-5-benzyloxy-2,3-dihydro-2,2,4,6,7-pentamethyl]benzofuran-3-ylmethyl]-(2S)-pyrrolidine-2-methoxy]phenyl methyl]thiazolidine-2,4-dione (15a, 0.47 g), obtained in example 15 by an analogous procedure to that described in example 2, mp. 179-182°C.

$[\alpha]_D^{25}$ = -53.4 (c, 1.0, $CHCl_3$)

$^1H$ NMR ($CDCl_3$, 200 MHz): δ 1.36, 1.40 (2s, 3H), 1.62, 1.67 (2s, 3H), 2.06 - 2.45 (complex m, 15H), 2.81 (m, 1H), 3.14 - 3.64 (complex m, 5H), 4.16 - 4.21 (complex m, 2H), 4.50 (m, 1H), 4.64 - 4.72 (m, 2H), 6.27 (s, 2H), 6.82 (m, 2H), 7.17 (m, 2H), 7.43 (m, 5H).

**Example 17**

**5-[4-[N-[(3RS)-5-Benzyloxy-2,3-dihydro-2,2,4,6,7-pentamethyl benzofuran-3-ylmethyl-(2S)-pyrrolidine-2-methoxy]phenyl methyl]thiazolidine-2,4-dione hydrochloride:**

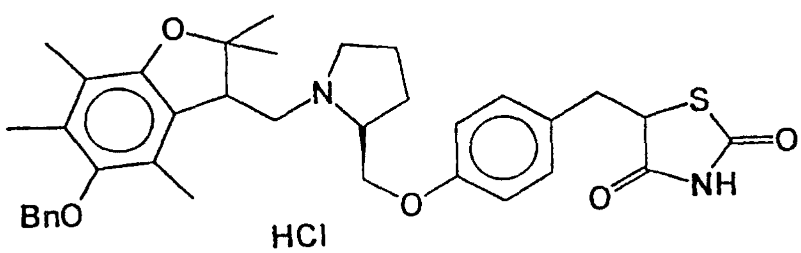

The title compound (0.4 g, 76 %) was prepared as a colorless solid from 5-[4-[(3RS)-5-benzyloxy-2,3-dihydro-2,2,4,6,7-pentamethyl]benzofuran-3-ylmethyl]-(2S)-pyrrolidine-2-methoxy]phenyl methyl]thiazolidine-2,4-dione (15a, 0.5 g) obtained in example 15 by an analogous procedure to that described in example 4, mp 161-163°C.

$[\alpha]_D^{25}$ = -2.4 (c, 1.0, $CHCl_3$).

$^1H$ NMR ($CDCl_3$, 200 MHz): δ 1.35 (s, 3H), 1.66 (s, 3H), 1.85 - 2.27 (complex m, 15 H), 3.02 -3.96 (complex m, 6H), 4.22 - 4.37 (m, 2H), 4.63 (m, 2H), 4.86 (m, 1H), 6.95 (d, J = 7.8 Hz, 2H), 7.22 (d, J = 7.8 Hz, 2H), 7.45 (m, 5H), 9.96 (bs, 1H, exchangeable with $D_2O$), 12.05 (s, 1H, exchangeable with $D_2O$).

**Example 18**

**5-[4-[N-[(3RS)-2,3-dihydro-5-hydroxy-2,2,4,6,7-pentamethylbenzofuran-3-ylmethyl]-(2S)-pyrrolidine-2-methoxy]phenyl methyl]thiazolidine-2,4-dione, hydrochloride:**

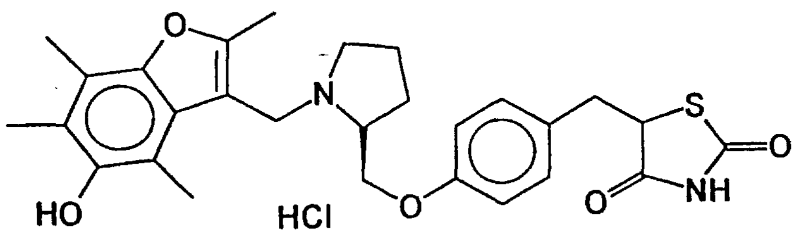

The title compound (0.32 g, 75 %) was prepared as a colorless solid from 5-[4-[N-[(3RS)-2,3-dihydro-5-hydroxy-2,2,4,6,7-pentamethylbenzofuran-3-ylmethyl]-(2S)-pyrrolidine-2-methoxy] phenyl methyl]thiazolidine-2,4-dione (15b, 0.4 g) obtained in example 15 by an analogous procedure to that described in example 4, mp 193-195°C.

$[\alpha]_D^{25}$ = -53.9 (c, 1.0, $CHCl_3$).

$^1H$ NMR ($CDCl_3$, 200 MHz): δ 1.30 (s, 3H), 1.63 (s, 3H), 1.77 - 2.28 (complex m, 15 H), 3.03 - 3.38 (complex m, 6H), 4.21 (m, 1H), 4.42 (m, 1H), 4.90 (dd, J = 8.72,4.19 Hz, 1H), 6.94 (d, J = 8.2, 2H), 7.22 (d, J = 7.9 Hz, 2H), 9.86 (bs, 1H, exchangeable with $H_2O$), 12.06 (s, 1H, exchangeable with $D_2O$).

**Example 19**

**5-[3-[N-[(3RS)-5-Benzyloxy-2,3-dihydro-2,2,4,6,7-pentamethylbenzofuran-3-ylmethyl]-(2S)-pyrrolidine-2-methoxy]phenyl methylene]thiazolidine-2,4-dione:**

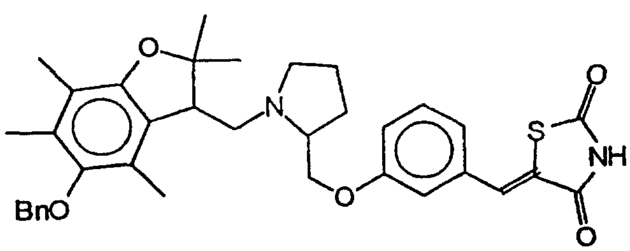

The title compound (0.3 g, 51 %) was prepared from 3-[N-(5-benzyloxy-2,3-dihydro-2,2,4,6,7-pentamethylbenzofuran-3-ylmethyl)-(2S)-pyrrolidine-2-methoxy]benzaldehyde (0.5 g) obtained in, preparation 17 by a similar procedure to that described in example 1. mp 58 - 60°C.

$[\alpha]_D^{23}$ = -96.7 (c, 1.0, $CHCl_3$)

$^1H$ NMR ($CDCl_3$, 200 MHz): δ 1.33 (s, 3H), 1.60 (s, 3H), 1.92 (m, 4H), 2.08 (s, 3H), 2.19, (s, 3H), 2.26 (s, 3H), 2.45 (m, 2H), 2.78 (m, 1H), 3.14 (m, 2H), 3.38 (m, 1H), 3.82 (m, 1H), 4.02 (m, 1H), 4.71 (s, 2H), 6.94 (m, 2H), 7.07 (d, J = 7.8 Hz, 1H), 7.45 (m, 6H), 7.80 (s, 1H).

**Example 20**

**5-[4-[N-(5-Benzyloxy-2,4,6,7-tetramethylbenzofuran-3-ylmethyl)-(2S)-pyrrolidine-2-methoxy]phenyl methylene]thiazolidine-2,4-dione:**

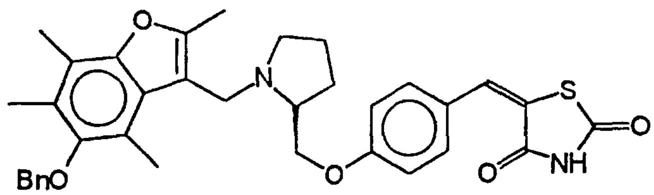

The title compound (0.4 g, 56 %) was prepared from 4-[N-(5-benzyloxy-2,4,6,7-tetramethylbenzofuran-3-ylmethyl)-(2S)-pyrrolidine-2-methoxy]benzaldehyde (**18a**, 0.6 g) obtained in preparation 18 by an analogous procedure to that described in example 1, mp. 180 -183°C.

$^1$H NMR ($CDCl_3$, 200 MHz): δ 1.75 (m, 4H), 2.05 (m, 1H), 2.31 (s, 3H), 2.38 (s, 3H), 2.42 (s, 3H), 2.60 (s, 3H), 3.00 (m, 2H), 3.63 (d, J = 12.9 Hz, 1H), 3.78 (m, 1H), 3.83 (m, 1H), 4.04 (d, J = 12.9 Hz, 1H), 4.71 (s, 2H), 6.78 (d, J = 8.6 Hz, 2H), 7.44 (m, 7H), 7.78 (s, 1H).

**Example 21**

**5-[4-[N-(5-Benzyloxy-2,4,6,7-tetramethylbenzofuran-3-ylmethyl)-(2S)-pyrrolidine-2-methoxy]phenyl methylene]thiazolidine-2,4-dione sodium salt:**

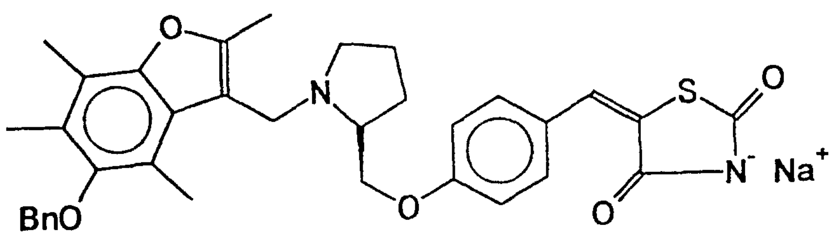

The title compound (0.28 g, 67 %) was prepared as a pale yellow solid from 5-[4-[N-[5-benzyloxy-2,4,6,7-tetramethylbenzofuran-3-ylmethyl]-(2S)-pyrrolidine-2-methoxy]phenyl methylene]thiazolidine-2,4-dione (0.4 g) obtained in example 20 by an analogous procedure to that in example 3, mp. 210 - 218°C.

$[\alpha]_D^{25}$ = + 65.1 (c, 1.0, $CHCl_3$)

$^1$H NMR ($CDCl_3$, 200 MHz): δ 1.68 (m, 4H), 2.01 (m, 1H), 2.23 (s, 3H), 2.30 (s, 3H), 2.40 (s, 3H), 2.59 (s, 3H), 2.88 (m, 2H), 3.46 (d, J = 12.8 Hz, 1H), 3.90 (m, 2H), 4.12 (d, J=12.9 Hz, 1H), 4.62 (s, 2H), 6.86 (d, J = 8.7 Hz, 2H), 7.41 (m, 8H).

**Example 22**

**5-[4-[N-(5-Benzyloxy-2,4,6,7-tetramethylbenzofuran-3-ylmethyl)-(3R)-piperidinyloxy] phenyl methylene] thiazolidine-2,4-dione:**

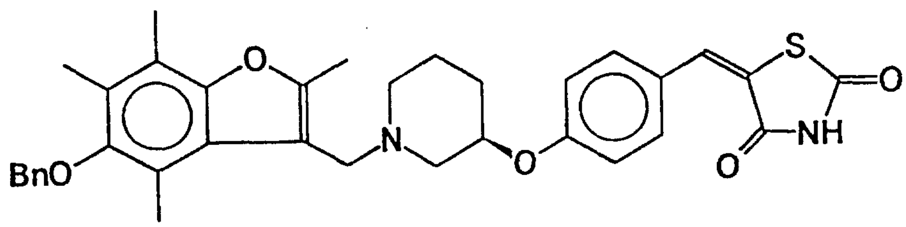

The title compound (0.9 g, 75 %) was prepared as a yellow solid from 4-[N-(5-benzyloxy-2,4,6,7-tetramemylbenzofuran-3-ylmemyl)-(3R)-piperidinyloxy]benzaldehyde (**18b,** 1.0 g) obtained in preparation 18 by a similar procedure as described in example 1, mp 100 -103°C.

$[\alpha]_D^{20}$ = -52.4 (c, 1.0, $CHCl_3$)

$^1H$ NMR ($CDCl_3$, 200 MHz): δ 1.58 (m, 3H), 1.78 (m, 1H), 2.16 (s, 2H), 2.33 (s, 3H), 2.40 (s, 6H), 2.73 (s, 3H), 2.78 (m, 1H), 3.15 (m, 1H), 3.48 (d, J = 4.9 Hz, 2H), 4.34 (m, 1H), 4.77 (s, 2H), 6.91 (d, J = 8.7 Hz, 2H), 7.42 (m, 7H), 7.74 (s, 1H).

**Example 23**

**5-[4-[N-(5-Benzyloxy-2,4,6,7-tetramethylbenzofuran-3-ylmethyl)-(2S)-pyrrolidine-2-methoxy]phenyl methyl] thiazolidine-2,4-dione:**

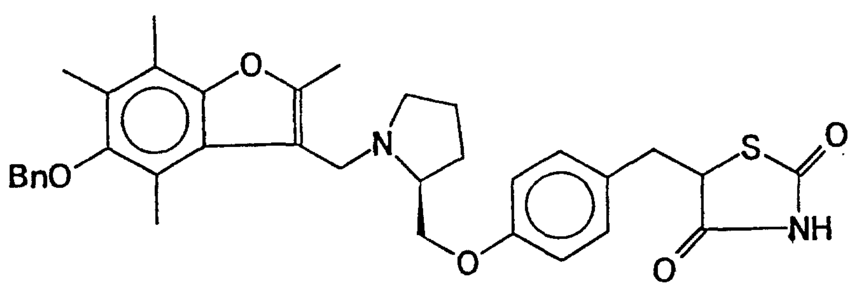

The title compound (0.4 g, 20 %) was prepared as a colorless solid from ethyl 2-bromo-3-[4-[N-(5-benzyloxy-2,4,6,7-tetramethylbenzofuran-3-ylmethyl)-(2S)-pyrrolidine-2-methoxy]phenyl] propanoate (2.25 g) obtained in preparation 32 by an analogous procedure to that described in method B of example 5. mp, 55 - 57°C.

$[\alpha]_D^{25}$ = + 25.2 (c, 0.5, $CHCl_3$)

$^1H$ NMR ($CDCl_3$, 200 MHz): δ 1.75 (m, 4H), 2.05 (m, 1H), 2.30 (s, 3H), 2.37 (s, 3H), 2.41 (s, 3H), 2.60 (s, 3H), 2.98 (m, 2H), 3.10 (m, 1H), 3.41 (m, 1H), 3.55 (m, 1H), 3.72 (m, 1H), 3.90 (m, 1H), 4.12 (m, 1H), 4.45 (m, 1H), 4.74 (s, 2H), 6.70 (m, 2H), 7.04 (d, J = 7 Hz, 2H), 7.42 (m, 5H).

**Example 24**

**5-[4-[N-(5-Benzyloxy-2-methylbenzofuran-3-ylmethyl)-(2S)-pyrrolidine-2-methoxy]phenyl methylene] thiazolidine-2,4-dione:**

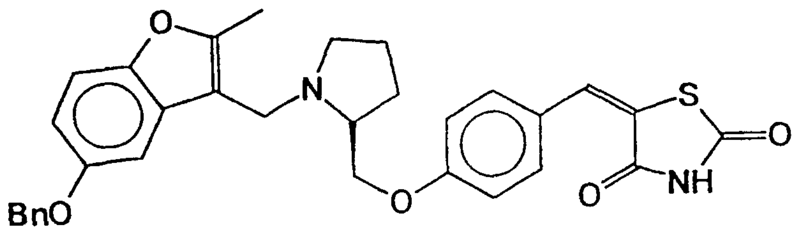

The title compound (0.4 g, 73 %) was prepared as a pale yellow solid from 4-[N-[5-benzyloxy-2-methylbenzofuran-3-ylmethyl]-(2S)-pyrrolidine-2-methoxy]benzaldehyde (0.45 g) obtained in preparation 19 by a similar procedure to that described in example 1.

$[\alpha]_D^{24}$ = -12.2 (c, 1.0, $CHCl_3$)

$^1$H NMR ($CDCl_3$, 200 MHz): δ 1.75 (m, 3H), 1.93 (m, 2H), 2.46 (s, 3H), 3.12 (m, 2H), 3.89 (d, J = 14.1 Hz, 1H), 4.05 (m, 1H), 4.13 (m, 1H), 4.19 (d, J = 14.1 Hz, 1H), 4.92 (s, 2H), 6.87 (m, 1H), 6.94 (d, J = 8.9 Hz, 2H), 7.13 (d, J = 2.2 Hz, 1H), 7.36 (m, 8H), 7.55 (s, 1H).

**Example 25**

**4-[N-[5-Benzyloxy-2-methylbenzofuran-3-ylmethyl]-(3R)-piperidinyloxy]phenyl methylene] thiazolidine-2,4-dione (25):**

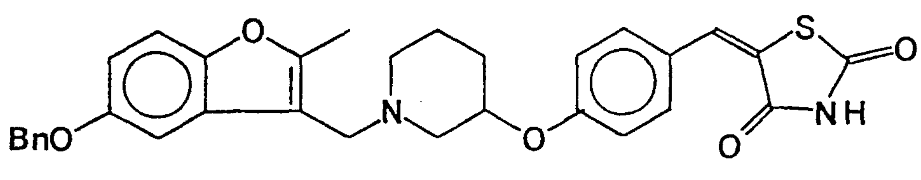

The title compound (1.5 g, 41 %) was prepared as a yellow solid from the crude mixture (3 g) obtained in preparation 19 by a similar procedure to that described in example 1. The crude product was chromatographed on silica gel to get **25**, mp 150-155°C.

$[\alpha]_D^{30}$ = -7.8 (c, 1.0, $CHCl_3$).

$^1$H NMR ($CDCl_3$, 200 Mhz) : δ 1.33 (m, 2H), 1.84 (m, 1H), 1.92 -2.28 (complex m, 3H), 2.39 (s, 3H), 2.76 (m, 1H), 3.08 (m, 1H), 3.62 (d, J = 2.5 Hz, 2H), 4.35 (m, 1H), 5.09 (s, 2H), 6.89 (m, 3H), 7.35 (m, 9H), 7.73 (s, 1H).

**Example 26**

**5-[4-[N-(5-Benzyloxy-2-methylbenzofuran-3-ylmethyl)-(2S)-pyrrolidine-2-methoxy]phenyl methyl]thiazolidine-2,4-dione:**

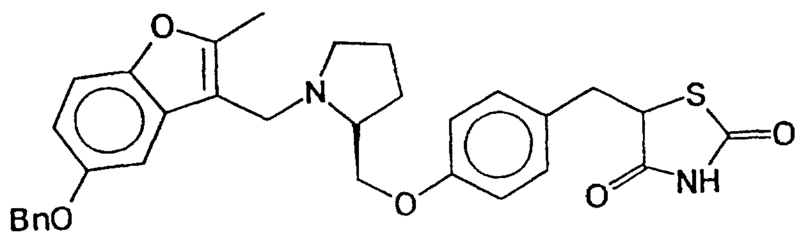

The title compound (0.7 g, 54 %) was prepared as a colorless solid from 5-[4-[N-(5-benzyloxy-2-methylbenzofuran-3-ylmethyl)-(2S)-pyrrolidine-2-methoxy]phenyl methylene]thiazolidine-2,4-dione (1.3 g) obtained in example 24, by an analogous procedure to that described in method A of example 5. mp : 68 - 71 °C.

$[\alpha]_D^{25}$ = -11.6 (c, 1.0, $CHCl_3$)

$^1H$ NMR ($CDCl_3$, 200 MHz): δ 1.76 (m, 4H), 2.05 (m, 1H), 2.41 (s, 3H), 3.05 (m, 3H), 3.34 (m, 1H), 3.64 (m, 1H), 3.96 (m, 2H), 4.19 (m, 1H), 4.36 (dd, J = 8.5,3.1 Hz, 1H), 4.89 (s, 2H), 6.84 (m, 3H), 7.14 (m, 3H), 7.35 (m, 6H).

**Example 27**

**5-[4-[N-(1-Methylindol-3-ylmethyl)-(2S)-pyrrolidine-2-methoxy]phenyl methylene]thiazolidine-2,4-dione:**

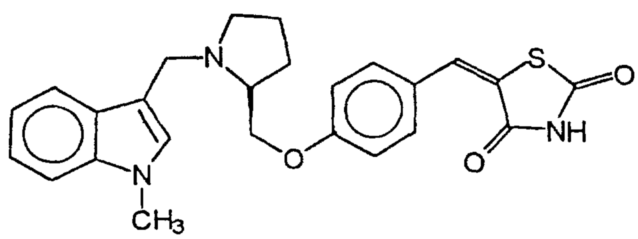

The title compound (0.35 g, 27 %) was prepared from 4-[N-(l-methylindol-3-ylmethyl)-(2S)-pyrrolidine-2-methoxy]benzaldehyde (1.02 g) obtained in preparation 20, by a similar procedure to that described in example 1. mp 159 - 160°C.

$[\alpha]_D^{26}$ = - 42.49 (c, 1.0, $CHCl_3$)

$^1H$ NMR ($CDCl_3$, 200 MHz): δ 1.89 (m, 2H), 2.17 (m, 2H), 2.96 (m, 1H), 3.49 (m, 1H), 3.75 (m, 1H), 3.86 (s, 3H), 4.28 (m, 2H), 4.53 (d, J = 5.3 Hz, 2H), 6.89 (d, J = 8.0 Hz, 2H), 7.28 (m, 7H), 7.75 (d, J = 6.6 Hz, 1H).

**Example 28**

**5-[4-[2-[4-[(2RS)-6-Benzyloxy-2,5,7,8-tetramethylchroman-2-ylmethyl]piperazine-1-yl]ethoxy]phenyl methylene]thiazolidine-2,4-dione:**

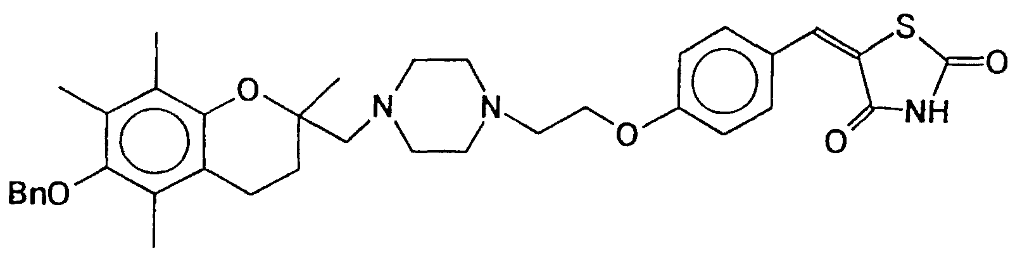

The title compound (0.16 g, 67 %) was prepared as a pale yellow solid from 4-[2-[4-(6-benzyloxy-2,5,7,8-tetramethylchroman-2-ylmethyl)piperazine-1-yl]ethoxy]benzaldehyde, (0.2 g) obtained in preparation 21 by a similar procedure to that described in example 1. mp 192-194°C.

$^1$H NMR ($CDCl_3$, 200 MHz): δ 1.24 (s, 3H), 1.7 (m, 1H), 2.0 (m, 1H), 2.07 (s, 3H), 2.17 (s, 3H), 2.22 (s, 3H), 2.45 - 3.0 (m, 14H), 4.2 (bs, 2H), 4.7 (s, 2H), 6.88 (d, J = 8.8 Hz, 2H), 7.2 - 7.6 (m, 7H).

**Example 29**

**5-[4-[2-[4-(1-Methylindol-3-ylmethyl)piperazin-1-yl]ethoxy]phenyl methylene]thiazolidine-2,4-dione:**

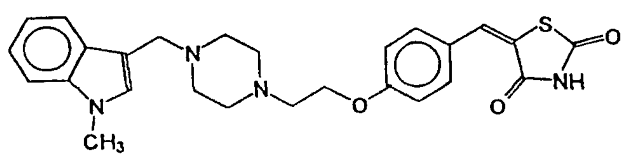

The title compound (0.7 g, 84 %) was prepared as a pale yellow solid from 4-[2-[4-[(1-methylindol-3-ylmethyl) piperazin-1-yl]ethoxy]benzaldehyde (0.6 g) obtained in preparation 22, by a similar procedure to that described in example 1. mp 183 - 188°C.

$^1$H NMR ($CDCl_3$+$CD_3OD$, 200 MHz): δ 2.69 (bs, 8H), 2.86 (t, J = 5.4 Hz, 2H), 3.81 (s, 5H), 4.17 (t, J = 5.21 Hz, 2H), 6.99 (d, J = 8.72 Hz, 2H), 7.19 (m, 4H), 7.48 (m, 2H), 7.70 (m, 2H).

**Example 30**

**5-[4-[N-[(2RS)-6-Hydroxy-2,5,7,8-tetramethylchroman-2-ylmethyl]-(2S)-pyrrolidine-2-methoxy] phenyl methylene]thiazolidine-2,4-dione:**

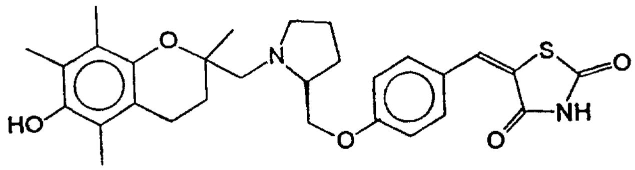

To a solution of 0.5 g of the product obtained in example 1, in 6 ml of acetic acid (6 ml) was added concentrated hydrochloric acid (2 ml). The resulting mixture was heated at 60°C. for 2 h. At the end of this time, the solvent was removed under reduced pressure and the residue was diluted with $CHCl_3$ and washed with aqueous sodium bicarbonate solution followed by brine. The organic layer was dried over anhydrous calcium chloride and the solvent was removed by distillation under reduced pressure. The crude product was purified by column chromatography on silica gel using 0 to 1 % (gradient elution) methanol in chloroform to afford 0.42 g (98 %) of the title compound as a pale yellow solid. mp 82 - 84°C.

$[\alpha]_D^{27}$ = -29.44 (c, 0.9, $CHCl_3$)

$^1H$ NMR ($CDCl_3$, 200 MHz): δ 1.21, 1.25 (2s, 3H), 1.5 - 2.05 (m, 6H), 2.13 (m, 9H), 2.3 - 3.2 (m, 6H), 3.4 (m, 1H), 3.9 (m, 1H), 4.05 (m, 1H), 6.95 (d, J = 7.4 Hz, 2H), 7.45 (m, 2H), 7.82, 7.83 (2s, 1H).

**Example 31**

**5-[4-[N-[(2RS)-6-Hydroxy-2,5,7,8-tetramethylchroman-2-ylmethyl]-(2S)-pyrrolidine-2-methoxy] phenyl methyl] thiazolidine-2,4-dione:**

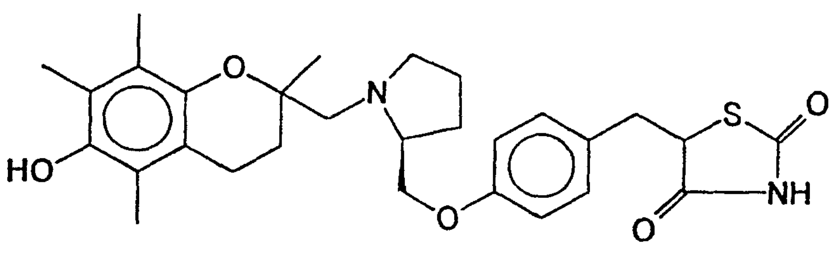

The title compound (0.22 g, 86 %) was prepared as a pale yellow solid from 5-[4-[N-[(2RS)-6-benzyloxy-2,5,7,8-tetramethylchroman-2-ylmethyl]-(2S)-pyrrolidine-2-methoxy]phenyl methyl] thiazolidine-2,4-dione (0.3 g), obtained in example 5 by an analogous procedure to that described in example 30. mp. 76 - 78°C.

$^1H$ NMR ($CDCl_3$, 200 MHz): δ 1.21 (3s, 3H), 1.5 - 2.05 (m, 6H), 2.1 (s, 6H), 2.15 (s, 3H), 2.3 - 2.8 (m, 4H), 2.9 - 3.25 (m, 3H), 3.4 (m, 2H), 3.6 - 4.0 (m, 2H), 4.5 (dd, J = 8.8 Hz and 3.8 Hz), 6.8 (m, 2H), 7.12 (d, J = 8.2 Hz, 2H).

**Example 32**

**5-[4-[N-[(3R/S)-5-Hydroxy-2,3-dihydro-2,2,4,6,7-pentamethylbenzofuran-3-ylmethyl]-(2S)-pyrrolidine-2-methoxy]phenyl methylene]thiazolidine-2,4-dione:**

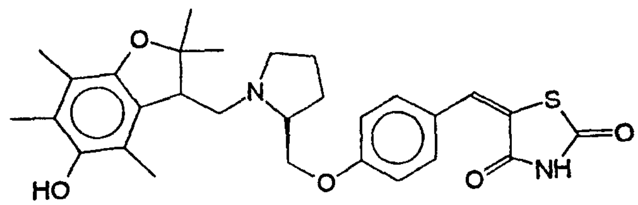

The title compound (0.13 g, 43 %) was prepared as a yellow solid from 5-[4-[N-[(3R/S)-5-benzyloxy-2,3-dihydro-2,2,4,6,7-pentamethylbenzofuran-3-ylmethyl]-(2S)-pyrrolidine-2-methoxy] phenyl methylene]thiazolidine-2,4-dione (0.4 g) obtained in example 11, by an analogous procedure to that described in example 30. mp 75 - 77°C.

$[\alpha]_D^{29}$ = -175.5 (c, 1.0, $CHCl_3$)

$^1H$ NMR ($CDCl_3$, 200 MHz): δ 1.31 (s, 3H), 1.58 (s, 3H), 1.85 (m, 4H), 2.09 (s, 3H), 2.13 (s, 3H), 2.22 (s, 3H), 2.38 (m, 2H), 2.76 (m, 1H), 3.11 (m, 2H), 3.36, (m, 1H), 3.85 (dd, J = 9.1 and 6.4 Hz, 1H), 4.06 (dd, J = 9.1 and 4.9 Hz, 1H), 4.16 (bs, 1H), 6.95 (d, J = 8.7 Hz, 2H), 7.43 (d, J = 8.7 Hz, 2H), 7.8 (s, 1H).

**Example 33**

**5-[4-[N-(5-Hydroxy-2,4,6,7-tetramethylbenzofuran-3-ylmethyl)-(2S)-pyrrolidine-2-methoxy]phenyl methylene] thiazolidine-2,4-dione:**

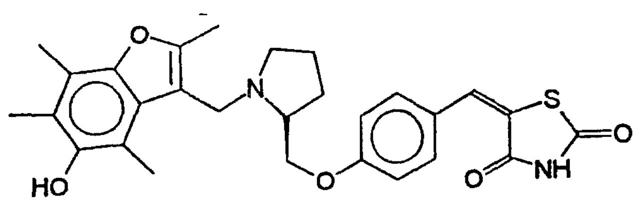

The title compound (0.27 g, 45 %) was prepared from 5-[4-[N-(5-benzyloxy-2,4,6,7-tetramethylbenzofuran-3-ylmethyl)-(2S)-pyrrolidine-2-methoxy]phenyl methylene]thiazolidine-2,4-dione (0.7 g) obtained in example 20, by an analogous procedure to that described in example 30. mp, 157 - 158°C.

$[\alpha]_D^{26}$ = + 75.1 (c, 0.7, EtOAc)

$^1H$ NMR ($CDCl_3$, 200 MHz): δ 1.78 (m, 4H), 2.04 (m, 1H), 2.24 (s, 3H), 2.38 (s, 3H), 2.40 (s, 3H), 2.51 (s, 3H), 3.04 (m, 2H), 3.62 (d, J = 12.9 Hz, 1H), 3.79 (m, 1H), 3.83 (m, 1H), 4.04 (d, J = 12.9 Hz, 1H), 6.76 (d, J = 8.9 Hz, 2H), 7.38 (d, J = 8.7 Hz, 2H), 7.78 (s, 1H).

**Example 34**

**5-[4-[N-(5-Hydroxy-2,4,6,7-tetramethylbenzofuran-3-ylmethyl)-(3R)-piperidinyloxy] phenylmethylene] thiazolidine-2,4-dione:**

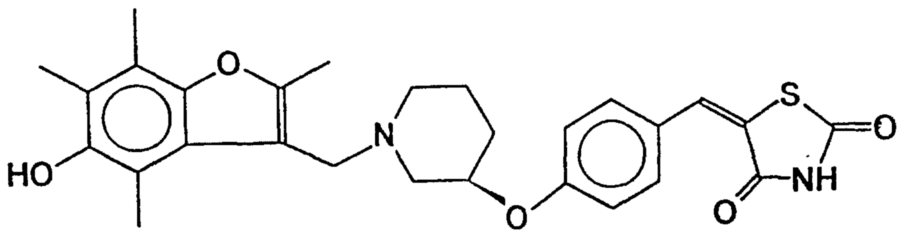

The title compound (0.1 g, 47 %) was prepared from 4-[N-(5-benzyloxy-2,4,6,7-tetramethyl-3-ylmethyl)-(3R)-piperidinyloxy]phenyl methylene]thiazolidine-2,4-dione (0.25 g) obtained in example 22, by an analogous procedure as described in example 30, mp. 192 - 195°C.

$[\alpha]_D^{20}$ = -36.3 (c, 1.0, $CHCl_3$)

$^1$H NMR ($CDCl_3$, 200 MHz): δ 1.45 (m, 3H), 1.90 (m, 1H), 2.09 (m, 2H), 2.24 (s, 3H), 2.33 (s, 3H), 2.35 (s, 3H) 2.65 (m, 1H), 3.05 (m, 1H), 3.48 (d, J = 3.1 Hz, 2H), 4.35 (m, 1H), 6.90 (d, J = 8.7 Hz, 2H), 7.37 (d, J = 8.7 Hz, 2H), 7.68 (s, 1H)

**Example 35**

**5-[3-[N-[(3RS)-[2,3-Dihydro-5-hydroxy-2,2,4,6,7-pentamethylbenzofuran-3-ylmethyl]-(2S)-pyrrolidine-2-methoxy]phenyl methylene]thiazolidine-2,4-dione:**

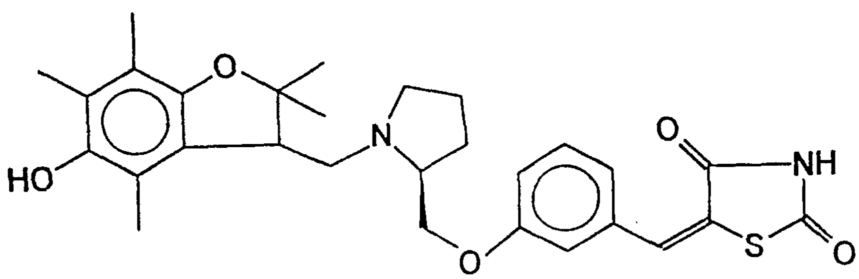

The title compound (0.68 g, 80 %) was prepared from 5-[3-[N-[(3RS)-5-benzyloxy-2,3-dihydro-2,2,4,6,7-pentamethylbenzofuran-3-ylmethyl]-(2S)-pyrrolidine-2-methoxy]phenyl methylene] thiazolidine-2,4-dione (1 g) obtained in example 19 by an analogous procedure to that described in example 30, mp 70 - 72°C.

$[\alpha]_D^{23}$ = - 83.9 (c, 1.0, $CHCl_3$)

$^1$H NMR ($CDCl_3$, 200 MHz): δ 1.31 (s, 3H), 1.59 (s, 3H), 1.85 (m, 4H), 2.08 (s, 3H), 2.12 (s, 3H), 2.21 (s, 3H), 2.35 (m, 2H), 2.78 (m, 1H), 3.14 (m, 2H), 3.38 (m, 1H), 3.84 (m, 1H), 4.04 (dd, J = 9.13 and 4.8 Hz, 1H), 4.19 (bs, 1H, exchangeable with $D_2O$), 6.96 (m, 2H), 7.06 (m, 1H), 7.34 (m, 1H), 7.79 (s, 1H).

**Example 36**

**5-[4-[N-(5-Hydroxy-2-methylbenzofuran-3-ylmethyl)-(2S)-pyrrolidine-2-methoxylphenyl methylene] thiazolidine-2,4-dione:**

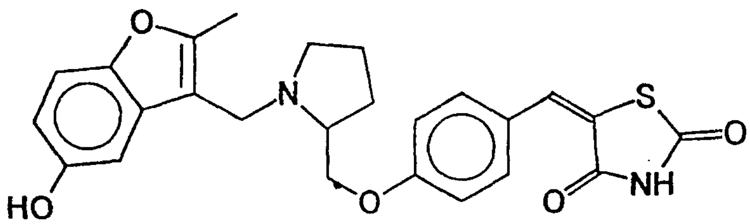

The title compound (0. 1 g, 40 %) was prepared from 5-[4-[N-(5-benzyloxy-2-methylbenzofuran-3-ylmethyl) -(2S)-pyrrolidine-2-methoxy]phenyl methylene]thiazolidine-2,4-dione, (0.3 g) obtained, in example 24 by an analogous procedure to that described in example 30, mp 206 - 208°C.

$[\alpha]_D^{25}$ = + 48.4 (c, 1.0, pyridine)

$^1$H NMR ($CDCl_3$, 200 MHz): δ 1.75 (m, 2H), 1.77 (m, 2H), 2.02 (m, 1H), 2.43 (s, 3H), 3.08 (m, 1H), 3.21 (m, 1H), 3.75 (d, J = 13.61 Hz, 1H) 4.09 (m, 2H), 4.19 (d, J = 13.61 Hz, 1H), 6.79 (m, 1H), 6.95 (d, J = 8.7 Hz, 2H), 7.06 (m, 1H), 7.16 (m, 1H), 7.34 (d, J = 8.7 Hz, 2H), 7.59 (s, 1H).

Mutation in colonies of laboratory animals and different sensitivities to dietary regimens have made the development of animal models with non-insulin dependent diabetes associated with obesity and insulin resistance possible. Genetic models such as db/db and ob/ob (See Diabetes, (1982) 31(1) : 1- 6) in mice and fa/fa and zucker rats have been developed by the various laboratories for understanding the pathophysiology of disease and testing the efficacy of new antidiabetic compounds (Diabetes, (1983) 32: 830-838 ; Annu. Rep. Sankyo Res. Lab. (1994). 46 : 1-57). The homozygous animals, C57 BL/KsJ-db/db mice developed by Jackson Laboratory, US, are obese, hyperglycemic, hyperinsulinemic and insulin resistant (J. Clin. Invest., (1990) 85 : 962-967), whereas heterozygous are lean and normoglycemic. In db/db model, a mouse progressively develops insulinopenia with age, a feature commonly observed in late stages of human type II diabetes when blood sugar levels are insufficiently controlled. The state of pancreas and its course vary according to the models. Since this model resembles that of type II diabetes mellitus, the compounds of the present invention were tested for blood sugar and triglycerides lowering activities.

The compounds of the present invention showed blood sugar and triglycerides lowering activities through improved insulin resistance. This was demonstrated by the following *in vivo* experiments.

Male C57BL/KsJ-db/db mice of 8 to 14 weeks age, having body weight range of 35 to 60 grams, procured from the Jackson Laboraotory, USA, were used in the experiment. The mice were provided with standard feed (National Institute of Nutrition, Hyderabad, India) and acidified water, ad libitum. The animals having more than 300 mg / dl blood sugar were used for testing. The number of animals in each group was 4.

The random blood sugar and triglyceride levels were measured by collecting blood (100 μl) through orbital sinus, using heparinised capillary in tubes containing EDTA which was centrifuged to obtain plasma. The plasma glucose and triglyceride levels were measured spectrometrically, by glucose oxidase and glycerol-3-$PO_4$ oxidase/peroxidase enzyme (Dr. Reddy's Lab. Diagnostic Division Kits, Hyderabad, India) methods respectively. On 6th day the blood samples were collected one hour after administration of test compounds / vehicle for assessing the biological activity.

Test compounds were suspended on 0.25 % carboxymethyl cellulose and administered to test group at a dose of 30 mg / kg through oral gavage daily for 6 days. The control group received vehicle (dose 10 ml / kg). Troglitazone (100 mg / kg, daily dose) was used as a standard drug which showed 28 % reduction in random blood sugar level on 6th day.

The blood sugar and triglycerides lowering activities of the test compound was calculated according to the formula:

$$\text{Blood sugar / triglycerides lowering activity (\%)} = 1 - \frac{DT/DC}{TC/ZC} \times 100$$

ZC = Zero day control group value

DC = Zero day treated group value
TC = Control group value on test day
DT = Treated group value on the test day

No adverse effects were observed for any of the mentioned compounds of invention in the above test.

| Compound | Reduction in Blood Glucose Level (%) | Triglyceride Lowering (%) |
|---|---|---|
| Example 7 | 36 | 70 |
| Example 13 | 45 | 42 |
| Example 20 | 16 | 43 |
| Example 22 | NA | NA |
| NA = Not Active at dose of 30 mg/Kg but has activity at a higher dose. | | |

The experimental results from the db/db mice suggest that the novel compounds of the present invention also possess therapeutic utility for prophylactic or regular treatment of obesity, cardiovascular disorders such as hypertension, hyperlipidaemia and other diseases; as it is known from the literature that such diseases are interrelated to each other.

## Claims

1. A compound of general formula (I)

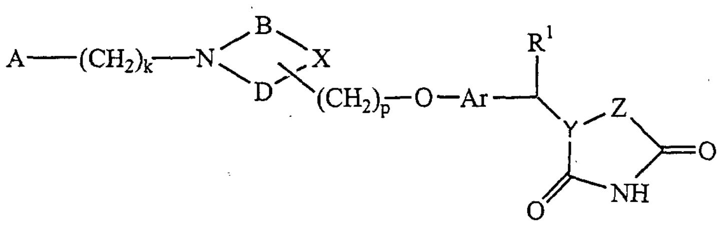

(I)

its tautomeric forms, its stereoisomers, its polymorphs, its pharmaceutically acceptable salts or its pharmaceutically acceptable solvates, where A represents dihydrobenzofuranyl, benzofuranyl, dihydrobenzopyranyl, or indolyl wherein the group represented by A may be substituted by hydroxy, $(C_1\text{-}C_6)$alkyl or $(C_1\text{-}C_6)$alkoxy, which alkoxy group may be itself substituted by an aryl group, B represents an unsubstituted linking group between N and X and B contains 1-4 carbon atoms, D represents a bond or D contains 1-4 carbon atoms, X represents $CH_2$, NH or sulfur; wherein the heterocycle comprising N, B, X and D is a single saturated unsubstituted five or six membered ring; Ar represents or phenylene or naphthylene, $R^1$ represents hydrogen atom or forms a bond together with the adjacent group Y; Y represents a nitrogen atom or a group $CR^2$ where $R^2$ forms a bond together with $R^1$; Z represents an oxygen atom or a sulfur atom when Y is $CR^2$ and Z represents an oxygen atom when Y is a nitrogen atom; k is an integer ranging from 1-4 and p is an integer ranging from 0 to 1.

2. A compound as claimed in claim 1, wherein k represents an integer 1 or 2.

3. A compound according to any one of claims 1-7, wherein the heterocycle comprising Y and Z is a 2,4-dioxothiazolidin-5-yl group.

4. A compound according to claim 1, which is selected from the group consisting of the following compounds:

5-[4-[N-(6-benzyloxy-2,5,7,8-tetramethylchroman-2-ylmethyl)-(2S)-pyrrolidine-2-methoxyl]phenyl methyl]thiazolidine-2,4-dione;
5-[4-[N-(6-benzyloxy-2,5,7,8-tetramethylchroman-2-ymiethyl)-(2R) pyrrolidine-2-methoxyl]phenyl methyl]thiazolidine-2,4-dione and its salts;
5-[4-[N-(6-benzyloxy-2,5,7,8-tetramethylchroman-2-ylmethyl)-(2S)pyrrolidine-2-methoxy]phenyl methyl]thiazolidine-2,4-dione sodium salt;
5-[4-[N-(6-benzyloxy-2,5,7,8-tetramethylchroman-2-ylmethyl)-(2S) pyrrolidine-2-methoxy]phenyl methyl]thiazolidine-2,4-dione maleic acid salt;
5-[4-[N-(6-benzyloxy-2,5,7,8-tetramethylchroman-2-ylmethyl)-(2S) pyrrolidine-2-methoxy]phenyl methyl]thiazolidine-2,4-dione hydrochloride salt;
5-[4-[N-(6-hydroxy-2,5,7,8-tetramethylchroman-2-ylmethyl)-(2S)-pyrrolidine-2-methoxy]phenyl methyl]thiazolidine-2,4-dione;
5-[4-[N-(6-hydroxy-2,5,7,8-tetramethylchroman-2-ylmethyl)-(2R)-pyrrolidine-2-methoxy]phenyl methyl]thiazolidine-2,4-dione and its salts;
5-[4-[N-(6-hydroxy-2,5,7,8-tetramethylchroman-2-ylmethyl)-(2S)-pyrrolidine-2-methoxy]phenyl methyl]thiazolidine-2,4-dione sodium salt;
5-[4-[N-[6-hydroxy-2,5,7,8-tetramethylchroman-2-ylmethyl)-(2S)-pyrrolidine-2-methoxy]phenyl methyl]thiazolidine-2,4-dione maleic acid salt;
5-[4-[N-[6-hydroxy-2,5,7,8-tetramethylchroman-2-ylmethyl)-(2S)-pyrrolidine-2-methoxy]phenyl methyl]thiazolidine-2,4-dione hydrochloride salt;
5-[4-[N-[6-hydroxy-2,5,7,8-tetramethylchroman-2-ylmethyl)-(2S)-pyrrolidine-2-methoxy]phenyl methylene] thiazolidine-2,4-dione;
5-[4-[N-[6-hydroxy-2,5,7,8-tetramethylchroman-2-ylmethyl]-(2R)-pyrrolidine-2-methoxy]phenyl methylene] thiazolidine-2,4-dione and its salts;
5-[4-[N-[2,3-dihydro-5-benzyloxy-2,2,4,6,7-pentamethylbenzofuran-3-yhnethyl]-(2S)-pyrrolidine-2-methoxy] phenyl methylene]thiazolidine-2,4-dione;
5-[4-[N-[2,3-dihydro-5-benzylozy-2,2,4,6,7-pentamethylbenzoduran-3-yl-methyl]-(2R_-pyrrolidine-2-methoxy]phenyl methylene]thiazolidine-2,4-dione and its salts;
5-[4-[N-[2,3-dihydro-5-benzyloxy-2,2,4,6,7-pentamethylbenzofuran-3-ylmethyl-(2S)-pyrrolidine-2-methoxy] phenyl methylene]thiazolidine-2,4-dione maleic acid salt;
5-[4-[N-[2,3-dihydro-5-benzyloxy-2,2,4,6,7-pentamethylbenzofuran-3-ylmethyl-(2S)-pyrrolidine-2-methoxy] phenyl methylene]thiazolidine-2,4-dione hydrochloride salt;
5-[4-[N-[2,3-dihydro-5-benzyloxy-2,2,4,6,7-pentamethylbenzofuran-3-ylmethyl]-(2S)-pyrrolidine-2-methoxy] phenyl methyl]thiazolidine-2,4-dione;
5-[4-[N-[2,3-dihydro-5-benzyloxy-2,2,4,6,7-pentamethylbenzofuran-3-ylmethyl]-(2R)-pyrrolidine-2-methoxy phenyl methyl]thiazolidine-2,4-dione and its salts;
5-[4-[N-[2,3-dihydro-5-benzylozy-2,2,4,6,7-pentamethylbenzofuran-3-yl-methyl-(2S)-pyrrolidine-2-methoxy] phenyl methyl]thiazolidine-2,4-dione maleic acid salt;
5-[4-[N-[2,3-dihydro-5-benzyloxy-2,2,4,6,7-pentamethylbenzofuran-3-ylmethyl]-(2S)-pyrrolidine-2-methoxy] phenyl methyl]thiazolidine-2,4-dione hydrochloride salt;
5-[4-[N-[2,3-dihydro-5-hydroxy-2,2,4,6,7-pentamethylbenzofuran-3-yl methyl-(2S)-pyrrolidine-2-methoxy] phenyl methyl]thiazolidine-2,4-dione;
5-[4-[N-[2,3-dihydro-5-hydroxy-2,2,4,6,7-pentamethylbenzofuran-3-ylmethyl]-(2R)-pyrrolidine-2-methoxy] phenyl methyl]thiazolidine-2,4-dione and its salts;
5-[4-[N-[2,3-dihydro-5-hydroxy-2,2,4,6,7-pentamethylbenzofuran-3-ylmethyl]-(2S)-pyrrolidine-2-methoxy] phenyl methyl]thiazolidine-2,4-dione hydrochloride salt;
5-[4-[N-[2,3-dihydro-5-hydroxy-2,2,4,6,7-pentamethylbenzofuran-3-ylmethyl]-(2S)-pyrrolidine-2-methoxy] phenyl methylene]thiazolidine-2,4-dione;
5-[4-[N-[2,3-dihydro-5-hydroxy-2,2,4,6,7-pentamethylbenzofuran-3-ylmethyl-(2R)-pyrrolidine-2-methoxy] phenyl methylene]thiazolidine-2,4-dione and its salts;
5-[4-[N-[5-benzyloxy-2-methylbenzofuran-3-ylmethyl]-(2S)-pyrrolidine-2-methoxy]phenyl methylene]thiazolidine-2,4-dione;
5-[4-[N-[5-benzyloxy-2-methylbenzofuran-3-ylmethyl]-(2S)-pyrrolidine-2-methoxy]phenyl methylene] thiazolidine-2,4-dione sodium salt, and
5-[4-[N-[(5-benzyloxy-2-methylbenzofuran-3-ylmethyl]-(2S)-pyrrolidine-2-methoxy]phenyl methyl]thiazolidine-2,4-dione;
a polymorphic form 1 of 5-[4-[N-[(2RS)-6-Benzyloly-2,5,7,8-tetramethylchroman-2-ylmethyl)]-(2S)-pyrrolidine-2-methoxy]phenylmethylene] thiazolidine-2,4-dione **characterized by** mp of about 186.6°C., and $^1$H NMR ($CDCl_3$, 200 MHz): δ 1.21, 1.26 (2s, 3H), 1.5 - 2.05 (m, 6H), 2.08 - 2.22 (6s, 9H), 2.35 - 3.15 (m, 6H), 3.4 (m, 1H), 3.8 (m, 1H), 4.0 (m, 1H), 4.7 (s, 2H), 6.95 (m, 2H), 7.3 - 7.6 (m, 7H), 7.8 (s, 1H);
a polymorphic form 2 of 5-[4-[N-[(2RS)-6-Benzyloxy-2,5,7,8-tetramethylchroman-2-ylmethyl)]-(2S)-pyrrolidine-2-methoxy]phenylmethylene] thiazolidine-2,4-dione **characterized by** mp of about 88°C., and $^1$H NMR ($CDCl_3$, 200 MHz): δ 1.21, 1.26 (2s, 3H), 1.5 - 2.05 (m, 6H), 2.08 - 2.22 (6s, 9H), 2.35 - 3.15 (m, 6H), 3.4 (m,

1H), 3.8 (m, 1H), 4.0 (m, 1H), 4.7 (s, 2H), 6.95 (m, 2H), 7.3 - 7.6 (m, 7H), 7.8 (s, 1H); and
a polymorphic form 3 of 5-[4-[N-[(2RS)-6-Benzyloxy-2,5,7,8-tetramethylchroman-2-ylmethyl)]-(2S)-pyrrolidine-2-methoxy]phenylmethylene] thiazolidine-2,4-dione **characterized by** mp of about 86°C; and $[\alpha]_D^{24}$ = 17.3 (c, 1.0, $CDCl_3$) and '200 MHz δ 1.21, 1.26 (2s, 3H), 1.5 - 2.05 (m, 6H), 2.08 - 2.22 (6s, 9H), 2.35 - 3.15 (m, 6H), 3.4 (m, 1H), 3.8 (m, 1H), 4.0 (m, 1H), 4.7 (s, 2H), 6.95 (m, 2H), 7.3 - 7.6 (m, 7H), 7.8 (s, 1H).

**5.** An intermediate of formula (III)

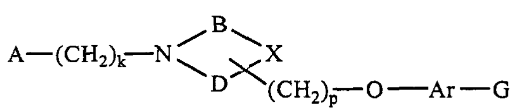

(III)

where A represents, dihydrobenzofuranyl, benzofuranyl, dihydrobenzopyranyl, or indolyl, wherein the group represented by A may be substituted by hydroxy, $(C_1-C_6)$alkyl or $(C_1-C_6)$alkoxy, which alkoxy group may be itself substituted by aryl; B represents a substituted or unsubstituted linking group between N and X and B contains 1-4 carbon atoms, D represents a bond or D contains 1-4 carbon atoms, X represents $CH_2$ or a heteroatom selected from nitrogen or sulfur wherein the group represented by A may be substituted by hydroxy, $(C_1-C_6)$alkyl or $(C_1-C_6)$ alkoxy, which alkoxy group may be itself substituted by aryl; Ar represents an phenylene or naphthylene; k is an integer ranging from 1-4 and p is an integer ranging from 0 to 1 and G represents -CHO, $-NO_2$, $-NH_2$, -CH=NHOH, $-CH_2NHOH$, $-CH_2N(OH)CONH_2$ or $-CH_2CH(J)-COOR$, where J represents a hydroxy group, or halogen atom and R represents H or lower alkyl group.

**6.** A process for preparing an intermediate of formula (III)

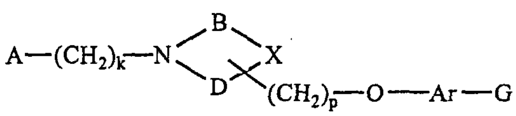

(III)

where A represents, dihydrobenzofuranyl, benzofuranyl, dihydrobenzopyranyl or indolyl, wherein the heterocycle comprising N, B, X and D is a single saturated unsubstituted five or six membered ring; B contains 1-4 carbon atoms, D represents a bond or D contains 1-4 carbon atoms, X represents $CH_2$ or a heteroatom selected from nitrogen or sulfur, wherein the heterocycle comprising N, B, X and D is a single saturated unsubstituted five or six membered ring; Ar represents a phenylene or naphthylene; k is an integer ranging from 1-4 and p is an integer ranging from 0 to 1; and G represents -CHO, or $NO_2$ which comprises

a) reacting the compound of the formula (IV),

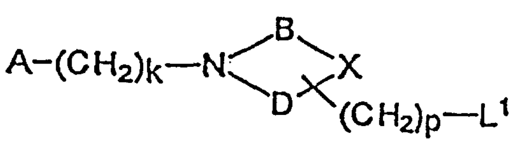

(IV)

wherein, A, B, D, X, k and p are as defined above and $L^1$ is a halogen atom or a leaving group with a compound of the formula (V)

$$HO—Ar—G \qquad (V)$$

where G is a CHO or a $NO_2$ group and Ar is as defined above;

b) reacting a compound of formula (VI)

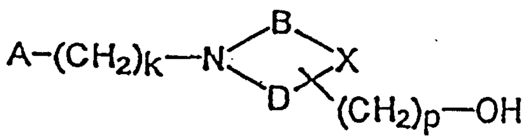

(VI)

where A, B, D, X, k and p are as defined above with a compound of formula (VII)

$$L^2\text{-Ar-G} \qquad \text{(VII)}$$

where G is a CHO or $NO_2$ group and Ar is as defined above and $L^2$ represents a halogen atom;
c) reacting a compound of formula (VI)

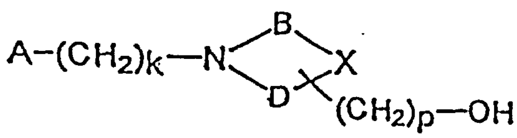

(VI).

where A, B, D, X, k and p are as defined above with a compound of formula (V)

$$\text{HO—Ar—G} \qquad \text{(V)}$$

where G is a CHO or $NO_2$ group and Ar as defined above;
d) reacting a compound of formula (VIII)

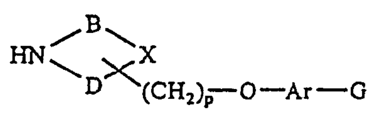

(VIII)

where B, D, X, Ar, k and p are as defined above, with a compound of formula (IX)

$$\text{A—}(CH_2)_k\text{—}L^1 \qquad \text{(IX)}$$

where A, and k are as defined above and $L^1$ is as defined in (a) above.

**7.** A process for the preparation of novel azolidinedione derivatives of formula (I),

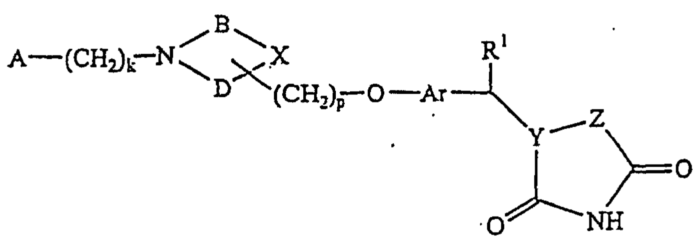

(I)

their tautomeric forms, their stereoisomers, their polymorphs, their pharmaceutically acceptable salts and their pharmaceutically acceptable solvates according to claim 1, wherein $R^1$ represent hydrogen, Y represents CH group and Z represents a sulfur or oxygen atom and where A represents, dihydrobenzofuranyl, benzofuranyl, dihydrobenzopyranyl or indolyl, wherein the group represented by A may be substituted by hydroxy, $(C_1-C_6)$alkyl or $(C_1-C_6)$alkoxy, which alkoxy group may be itself substituted by aryl; or B contains 1-4 carbon atoms, D represents a bond or D contains 1-4 carbon atoms; X represents $CH_2$ or a heteroatom selected from nitrogen or sulfur wherein the heterocycle comprising N, B, X and D is a single saturated unsubstituted five or six membered ring; Ar represents phenylene,or naphthylene, indolyl; k is an integer ranging from 1-4 and p is an integer ranging 0 to 1, which comprises:

a) i) reacting of formula (III)

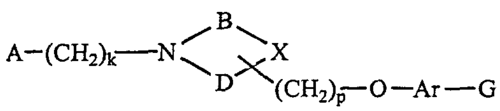

(III)

where G is a CHO group with 2,4-thiazolidinedione or 2,4-oxazolidinedione to yield a compound of formula (XII)

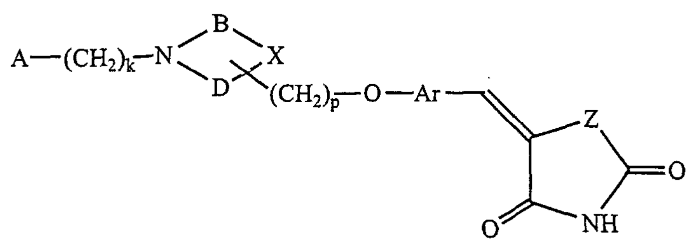

(XII)

where A, B, D, X, Ar, k, p and Z are as defined above and removing the water formed during the reaction;

ii) reducing the compound of formula (XII) obtained in step (i) above to obtain a compound of formula (XIII)

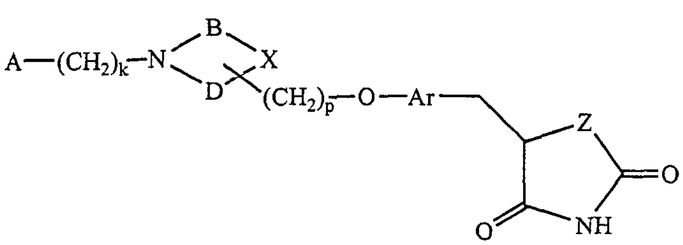

(XIII)

where A, B, D, X, Ar, k, p and Z are as defined above;

b) a process for the preparation of compound of formula (I) which comprises reacting a compound of formula (IV)

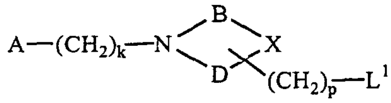

(IV)

where A, B, D, X, k and p are as defined above and $L^1$ is halogen atom or a leaving group, with a compound of formula (XIV)

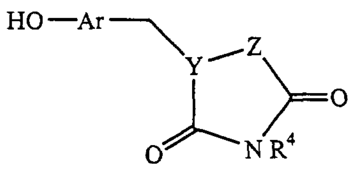

(XIV)

where Y, Z and Ar are as defined above and $R^4$ is hydrogen or a nitrogen protecting group which is removed after the reaction;

c) a process for the preparation of compound of formula (I) which comprises reacting a compound of formula (VI)

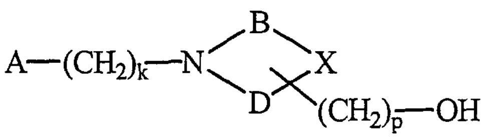

(VI)

where A, B, D, X, k and p are as defined above with a compound of formula (XIV)

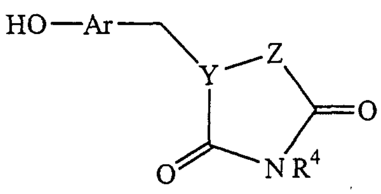

(XIV)

where Y, Z and Ar are as defined above and $R^4$ is hydrogen or a nitrogen protecting group, which is removed after the reaction; or

d) a process for the preparation of compound of formula (I) which comprises reacting a compound of formula (XV)

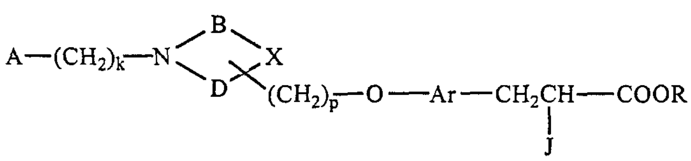

(XV)

where A, B, D, X, k and p are as defined above, J is a halogen atom or a hydroxy group and R is a lower alkyl group, with thiourea when J is a halogen atom, or urea when J is a hydroxy group followed by treatment with an acid.

**8.** A pharmaceutical composition which comprises a compound of formula (I) as defined in any one of claims 1 to 4 and a pharmaceutically acceptable carrier, diluent, excipient or solvate.

**9.** A pharmaceutical composition as claimed in claim 8, in the form of a tablet, capsule, powder, syrup, solution or suspension.

**10.** Use of a compound according to any one of claims 1-4, for the manufacture of a medicament for preventing or treating diabetes caused by insulin resistance or impaired glucose tolerance or complications of diabetes said diabetes caused by insulin resistance or impaired glucose tolerance.

**11.** Use according to claim 10, wherein the complication is dyslipidaemia, hypertension, coronary heart disease, cardiovascular disorders, atherosclerosis, obesity, psoriasis, polycystic ovarian syndrome (PCOS), renal diseases,

diabetic nephropathy, glomerulonephrithis, glomerular sclerosis, nephrotic syndrome, hypertensive nephrosclerosis, end-stage renal disease, microalbuminuria or eating disorders.

**12.** Use of a compound according to any of claims 1-4, for reducing blood glucose, triglycerides and free fatty acids in the blood.

**13.** Use of a compound according to any one of claims 1-4, for manufacture of a medicament for reducing blood glucose, triglycerides and free fatty acids in the blood.

**Patentansprüche**

**1.** Verbindung der Formel (I):

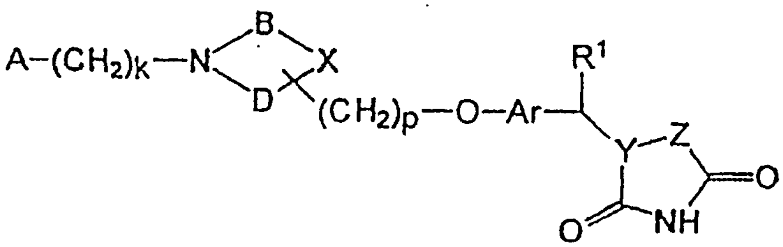

(I)

ihrer tautomeren Formen, ihrer Stereoisomere, ihrer polymorphen Formen, ihrer pharmazeutisch duldbaren Salze oder pharmazeutisch duldbaren Solvate, worin A Dihydrobenzofuranyl, Benzofuranyl, Dihydrobenzopyranyl oder Indolyl darstellt, wobei die durch A dargestellte Gruppe substituiert sein kann durch Hydroxy, $(C_1-C_6)$-Alkyl oder $(C_1-C_6)$-Alkoxy, wobei die Alkoxy-Gruppe selbst substituiert sein kann durch eine Aryl-Gruppe; B stellt eine nichtsubstituierte, verknüpfende Gruppe zwischen N und X dar, und B enthält 1 bis 4 Kohlenstoffatome; D stellt eine Bindung dar oder D enthält 1 bis 4 Kohlenstoffatome; X stellt $CH_2$, NH oder Schwefel dar; wobei der heterocyclische Ring, der N, B, X und D aufweist, ein einzelner gesättigter, unsubstituierter, fünf- oder sechsgliedriger Ring ist; Ar stellt Phenylen oder Naphthylen dar; $R^1$ stellt ein Wasserstoffatom dar oder geht zusammen mit der angrenzenden Gruppe Y eine Bindung ein; Y stellt ein Stickstoffatom dar oder eine Gruppe $CR^2$, worin $R^2$ zusammen mit $R^1$ eine Bindung eingeht; Z stellt ein Sauerstoffatom oder ein Schwefelatom dar, wenn Y $CR^2$ ist, und Z stellt ein Sauerstoffatom dar, wenn Y ein Stickstoffatom ist; k ist eine ganze Zahl von 1 bis 4 und p eine ganze Zahl von Null bis 1.

**2.** Verbindung nach Anspruch 1, bei welcher k eine ganze Zahl 1 oder 2 darstellt.

**3.** Verbindung nach einem der vorgenannten Ansprüche, bei welcher der heterocyclische Ring, der Y und Z aufweist, eine 2,4-Dioxothiazolidin-5-yl-Gruppe ist.

**4.** Verbindung nach Anspruch 1, ausgewählt aus der Gruppe, bestehend aus den folgenden Verbindungen:

5-[4-[(6-Benzyloxy-2,5,7,8-tetramethylchroman-2-ylmethyl)-(2S)pyrrolidin-2-methoxy]phenylmethyl]thiazolidin-2,4-dion;
5-[4-[(6-Benzyloxy-2,5,7,8-tetramethylchroman-2-ylmethyl)-(2R)pyrrolidin-2-methoxy]phenylmethyl]thiazolidin-2,4-dion und dessen Salze;
5-[4-[(6-Benzyloxy-2,5,7,8-tetramethylchroman-2-ylmethyl)-(2S)pyrrolidin-2-methoxy]phenylmethyl]thiazolidin-2,4-dion, Natrium-Salz;
5-[4-[(6-Benzyloxy-2,5,7,8-tetramethylchroman-2-ylmethyl)-(2S)pyrrolidin-2-methoxy]phenylmethyl]thiazolidin-2,4-dion, Maleinsäure-Salz;
5-[4-[(6-Benzyloxy-2,5,7,8-tetramethylchroman-2-ylmethyl)-(2S)pyrrolidin-2-methoxy]phenylmethyl]thiazolidin-2,4-dion, Chlorwasserstoff-Salz;
5-[4-[(6-Hydroxy-2,5,7,8-tetramethylchroman-2-ylmethyl)-(2S)pyrrolidin-2-methoxy]phenylmethyl]thiazolidin-2,4-dion;
5-[4-[(6-Hydroxy-2,5,7,8-tetramethylchroman-2-ylmethyl)-(2R)pyrrolidin-2-methoxy]phenylmethyl]thiazolidin-2,4-dion und dessen Salze;

5-[4-[(6-Hydroxy-2,5,7,8-tetramethylchroman-2-ylmethyl)-(2S)pyrrolidin-2-methoxy]phenylmethyl]thiazolidin-2,4-dion, Natrium-Salz;
5-[4-[(6-Hydroxy-2,5,7,8-tetramethylchroman-2-ylmethyl)-(2S)pyrrolidin-2-methoxy]phenylmethyl]thiazolidin-2,4-dion, Maleinsäure-Salz;
5-[4-[(6-Hydroxy-2,5,7,8-tetramethylchroman-2-ylmethyl)-(2S)pyrrolidin-2-methoxy]phenylmethyl]thiazolidin-2,4-dion, Chlorwasserstoff-Salz;
5-[4-[(6-Hydroxy-2,5,7,8-tetramethylchroman-2-ylmethyl)-(2S)pyrrolidin-2-methoxy]phenylmethylen]thiazolidin-2,4-dion;
5-[4-[(6-Hydroxy-2,5,7,8-tetramethylchroman-2-ylmethyl)-(2R)pyrrolidin-2-methoxy]phenylmethylen]thiazolidin-2,4-dion und dessen Salze;
5-[4-[(2,3-Dihydro-5-benzyloxy-2,2,4,6,7-pentamethylbenzofuran-3-ylmethyl)-(2S)pyrrolidin-2-methoxy]phenylmethylen]thiazolidin-2,4-dion;
5-[4-[(2,3-Dihydro-5-benzyloxy-2,2,4,6,7-pentamethylbenzofuran-3-ylmethyl)-(2R)pyrrolidin-2-methoxy]phenylmethylen]thiazolidin-2,4-dion
und dessen Salze;
5-[4-[(2,3-Dihydro-5-benzyloxy-2,2,4,6,7-pentamethylbenzofuran-3-ylmethyl)-(2S)pyrrolidin-2-methoxy]phenylmethylen]thiazolidin-2,4-dion, Maleinsäure-Salz;
5-[4-[(2,3-Dihydro-5-benzyloxy-2,2,4,6,7-pentamethylbenzofuran-3-ylmethyl)-(2S)pyrrolidin-2-methoxy]phenylmethylen]thiazolidin-2,4-dion, Chlorwasserstoff-Salz;
5-[4-[(2,3-Dihydro-5-benzyloxy-2,2,4,6,7-pentamethylbenzofuran-3-ylmethyl)-(2S)pyrrolidin-2-methoxy]phenylmethyl]thiazolidin-2,4-dion;
5-[4-[(2,3-Dihydro-5-benzyloxy-2,2,4,6,7-pentamethylbenzofuran-3-ylmethyl)-(2R)pyrrolidin-2-methoxy]phenylmethyl]thiazolidin-2,4-dion
und dessen Salze;
5-[4-[(2,3-Dihydro-5-benzyloxy-2,2,4,6,7-pentamethylbenzofuran-3-ylmethyl)-(2S)pyrrolidin-2-methoxy]phenylmethyl]thiazolidin-2,4-dion, Maleinsäure-Salz:
5-[4-[(2,3-Dihydro-5-benzyloxy-2,2,4,6,7-pentamethylbenzofuran-3-ylmethyl)-(2S)pyrrolidin-2-methoxy]phenylmethyl]thiazolidin-2,4-dion, Chlorwasserstoff-Salz:
5-[4-[(2,3-Dihydro-5-hydroxy-2,2,4,6,7-pentamethylbenzofuran-3-ylmethyl)-(2S)pyrrolidin-2-methoxy]phenylmethyl]thiazolidin-2,4-dion;
5-[4-[(2,3-Dihydro-5-hydroxy-2,2,4,6,7-pentamethylbenzofuran-3-ylmethyl)-(2R)pyrrolidin-2-methoxy]phenylmethyl]thiazolidin-2,4-dion und dessen Salze;
5-[4-[(2,3-Dihydro-5-hydroxy-2,2,4,6,7-pentamethylbenzofuran-3-ylmethyl)-(25)pyrrolidin-2-methoxy]phenylmethyl)thiazolidin-2,4-dion, Chlorwasserstoff-Salz;
5-[4-[(2,3-Dihydro-5-hydroxy-2,2,4,6,7-pentamethylbenzofuran-3-ylmethyl)-(2S)pyrrolidin-2-methoxy]phenylmethylen]thiazolidin-2,4-dion;
5-[4-[(2,3-Dihydro-5-hydroxy-2,2,4,6,7-pentamethylbenzofuran-3-ylmethyl-(2R)pyrrolidin-2-methoxy]phenylmethylen]thiazolidin-2,4-dion und dessen Salze;
5-[4-[(5-Benzyloxy-2-methylbenzofuran-3-ylmethyl]-(2S)pyrrolidin-2-methoxy]phenylmethylen]thiazolidin-2,4-dion;
5-[4-[(5-Benzyloxy-2-methylbenzofuran-3-ylmethyl]-(2S)pyrrolidin-2-methoxy]phenylmethylen]thiazolidin-2,4-dion, Natrium-Salz; sowie
5-[4-[(5-Benzyloxy-2-methylbenzofuran-3-ylmethyl]-(2S)pyrrolidin-2-methoxy]phenylmethyl]thiazolidin-2,4-dion;
eine polymorphe Form 1 von 5-[4-[N-[(2RS)-6-Benzyloxy-2,5,7,8-tetramethylchroman-2-ylmethyl)]-(2S)-pyrrolidin-2-methoxy]phenylmethylen]thiazolidin-2,4-dion, **gekennzeichnet durch** einen Fp von etwa 186,6°C und $^1$HNMR ($CDCl_3$, 200 MHz): δ 1,21, 1,26 (2s, 3H), 1,5- 2,05 (m, 6H), 2,08 - 2,22 (6s, 9H), 2,35 - 3,15 (m, 6H), 3,4 (m, 1H), 3,8 (m, 1H), 4,0 (m, 1H), 4,7 (s, 2H), 6,95 (m, 2H), 7,3 - 7,6 (m, 7H), 7,8 (s, 1H);
eine polymorphe Form 2 von 5-[4-[N-[(2RS)-6-Benzyloxy-2,5,7,8-tetramethylchroman-2-ylmethyl)]-(2S)-pyrrolidin-2-methoxy]phenylmethylen]thiazolidin-2,4-dion, **gekennzeichnet durch** einen Fp von etwa 88°C und 1HNMR ($CDCl_3$, 200 MHz): δ 1,21, 1,26 (2s, 3H), 1,5 -2,05 (m, 6H), 2,08 - 2,22 (6s, 9H), 2,35 - 3,15 (m, 6H), 3,4 (m, 1H), 3,8 (m, 1H), 4,0 (m, 1H), 4,7 (s, 2H), 6,95 (m, 2H), 7,3 - 7,6 (m, 7H), 7,8 (s, 1H); und
eine polymorphe Form 3 von 5-[4-[N-[(2RS)-6-Benzyloxy-2,5,7,8-tetramethylchroman-2-ylmethyl)]-(2S)-pyrrolidin-2-methoxy]phenylmethylen]thiazolidin-2,4-dion, **gekennzeichnet durch** einen Fp von etwa 86°C und $[\alpha]_D^{24}$ = 17,3 (c, 1,0, $CDCl_3$), und 200 MHz): δ 1,21, 1,26 (2s, 3H), 1,5- 2,05 (m, 6H), 2,08 - 2,22 (6s, 9H), 2,35 - 3,15 (m, 6H), 3,4 (m, 1H), 3,8 (m, 1H), 4,0 (m, 1H), 4,7 (s, 2H), 6,95 (m, 2H), 7,3 - 7,6 (m, 7H), 7,8 (s, 1H).

**5.** Intermediat der Formel (III):

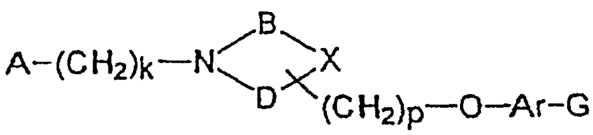

(III)

worin A Dihydrobenzofuranyl, Benzofuranyl,Dihydrobenzopyranyl oderIndolyl darstellt, wobei die durch A dargestellte Gruppe substituiert sein kann durch Hydroxy, ($C_1$-$C_6$)-Alkyl oder ($C_1$-$C_6$)-Alkoxy, wobei die Alkoxy-Gruppe selbst substituiert sein kann ein Aryl; B stellt eine nichtsubstituierte, verknüpfende Gruppe zwischen N und X dar, und B enthält 1 bis 4 Kohlenstoffatome; D stellt eine Bindung dar oder D enthält 1 bis 4 Kohlenstoffatome; X stellt $CH_2$ oder ein Heteroatom dar, ausgewählt aus Stickstoff oder Schwefel, wobei der heterocyclische Ring, der N, B, X und D aufweist, ein einzelner gesättigter, unsubstituierter, fünfoder sechsgliedriger Ring ist; Ar stellt Phenylen oder Naphthylen dar; k ist eine ganze Zahl von 1 bis 4 und p eine ganze Zahl von Null bis 1; und G stellt -CHO, $-NO_2$, $-NH_2$, -CH=NHOH, $-CH_2NHOH$, $-CH_2N(OH)CONH_2$ oder $-CH_2CH(J)$-COOR, worin J eine Hydroxy-Gruppe darstellt oder ein Halogenatom und R Wasserstoff darstellt oder eine Alkyl-Gruppe.

**6.** Verfahren zum Herstellen eines Intermediats der Formel (III):

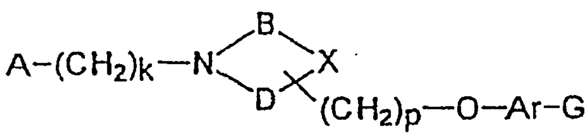

(III)

worin A Dihydrobenzofuranyl, Benzofuranyl, Dihydrobenzopyranyl oder Indolyl darstellt, wobei die durch A dargestellte Gruppe substituiert sein kann durch Hydroxy, ($C_1$-$C_6$)-Alkyl oder ($C_1$-$C_6$)-Alkoxy, wobei die Alkoxy-Gruppe selbst substituiert sein kann ein Aryl; B stellt eine nichtsubstituierte, verknüpfende Gruppe zwischen N und X dar, und B enthält 1 bis 4 Kohlenstoffatome; D stellt eine Bindung dar oder D enthält 1 bis 4 Kohlenstoffatome; X stellt $CH_2$ oder ein Heteroatom dar, ausgewählt aus Stickstoff oder Schwefel, wobei der heterocyclische Ring, der N, B, X und D aufweist, ein einzelner gesättigter, unsubstituierter, fünf- oder sechsgliedriger Ring ist; Ar stellt Phenylen oder Naphthylen dar; k ist eine ganze Zahl von 1 bis 4 und p eine ganze Zahl von Null bis 1; und G stellt -CHO oder $-NO_2$ dar;
welches Verfahren umfasst:

a) Umsetzen der Verbindung der Formel (IV)

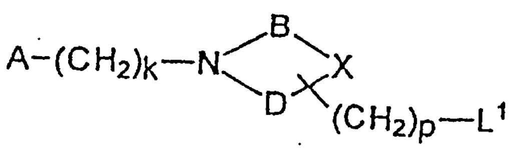

(IV)

worin A, B, D, X, k und p wie vorstehend festgelegt sind und $L^1$ ein Halogenatom oder ein spaltende Gruppe ist, mit einer Verbindung der Formel (V):

$$HO—Ar—G \qquad (V)$$

worin G eine CHO- oder $NO_2$-Gruppe ist und Ar wie vorstehend festgelegt ist.

b) Umsetzen einer Verbindung der Formel (VI):

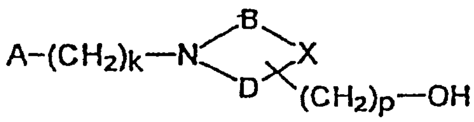

(VI)

worin G eine CHO- oder $NO_2$-Gruppe ist und Ar wie vorstehend festgelegt ist und $L^2$ ein Halogenatom darstellt, mit einer Verbindung der Formel (VII):

$$L^2\text{-Ar-G} \quad \text{(VII)}$$

worin G eine CHO- oder $NO_2$-Gruppe ist und Ar wie vorstehend festgelegt ist und $L^2$ ein Halogenatom darstellt;

c) Umsetzen einer Verbindung der Formel (VI) :

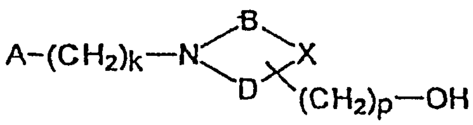

(VI)

worin G eine CHO- oder $NO_2$-Gruppe ist und Ar wie vorstehend festgelegt ist und $L^2$ ein Halogenatom darstellt, mit einer Verbindung der Formel (V):

$$\text{HO—Ar—G} \quad \text{(V)}$$

worin G eine CHO- oder $NO_2$-Gruppe ist und Ar wie vorstehend festgelegt ist;

d) Umsetzen einer Verbindung der Formel (VIII)

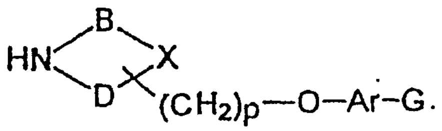

(VIII)

worin B, D, X Ar, k und p wie vorstehend festgelegt sind; mit einer Verbindung der Formel (IX)

$$\text{A—(CH}_2\text{)}_k\text{—L}^1 \quad \text{(IX)}$$

worin A und k wie vorstehend festgelegt sind und worin $L^1$ wie vorstehend in (a) festgelegt wurde.

**7.** Verfahren zum Herstellen neuartiger Azolidindion-Derivate der Formel (I):

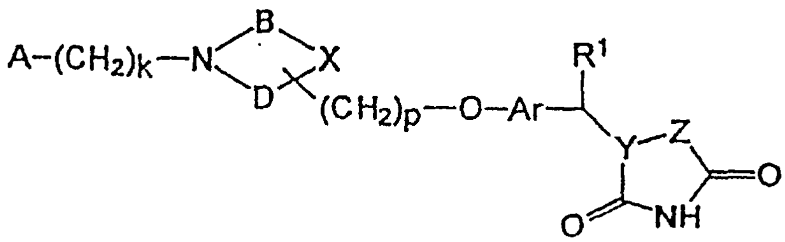

(I)

ihrer tautomeren Formen, ihrer Stereoisomere, ihrer polymorphen Formen, ihrer pharmazeutisch duldbaren Salze oder pharmazeutisch duldbaren Solvate nach Anspruch 1, worin $R^1$ Wasserstoff darstellt, Y stellt eine CH-Gruppe dar und Z stellt ein Schwefelatom oder Sauerstoffatom dar, und worin A Dihydrobenzofuranyl, Benzofuranyl, Dihydrobenzopyranyl oder Indolyl darstellt, wobei die durch A dargestellte Gruppe substituiert sein kann durch Hydroxy, ($C_1$-$C_6$)-Alkyl oder ($C_1$-$C_6$)-Alkoxy, wobei die Alkoxy-Gruppe selbst substituiert sein kann durch Aryl; B enthält 1 bis 4 Kohlenstoffatome; D stellt eine Bindung dar oder D enthält 1 bis 4 Kohlenstoffatome; X stellt $CH_2$ oder ein Heteroatom dar, ausgewählt aus Stickstoff oder Schwefel, wobei der heterocyclische Ring, der N, B, X und D aufweist, ein einzelner gesättigter, unsubstituierter, fünf- oder sechsgliedriger Ring ist; Ar stellt Phenylen oder Naphthylen dar; k ist eine ganze Zahl von 1 bis 4 und p eine ganze Zahl von Null bis 1; welches Verfahren umfasst:

a) i) Umsetzen der Verbindung der Formel (III):

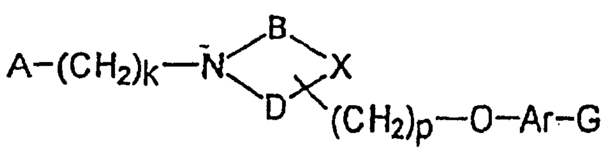

(III)

worin G eine CHO-Gruppe ist, mit 2,4-Thiazolidindion oder 2,4-Oxazolidindion, um eine Verbindung der Formel (XII) zu ergeben:

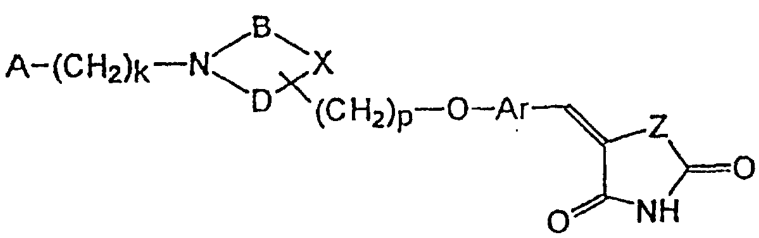

(XII)

worin A, B, D, X, Ar, k, p und Z wie vorstehend festgelegt sind; und Entfernen des während der Reaktion erzeugten Wassers;

ii) Reduzieren der Verbindung der Formel (XIII):

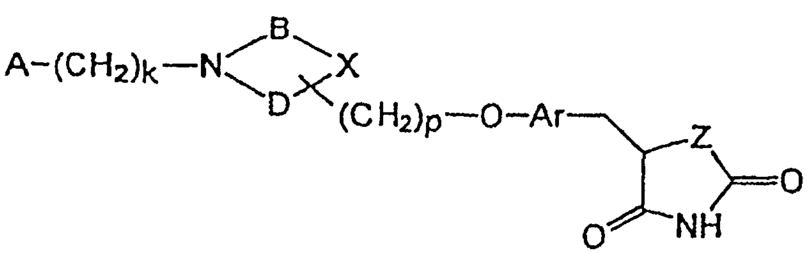

(XIII)

worin A, B, D, X, Ar, k, p und Z wie vorstehend festgelegt sind;

b) ein Verfahren zum Herstellen der Verbindung der Formel (I), umfassend das Umsetzen einer Verbindung

der Formel (IV):

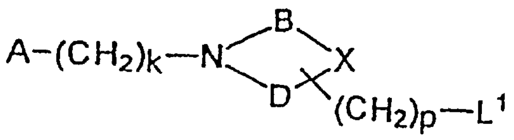

(IV)

worin A, B, D, X, k und p wie vorstehend festgelegt sind und $L^1$ ein Halogenatom ist oder eine abspaltende Gruppe; mit einer Verbindung der Formel (XIV):

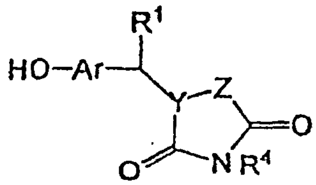

(XIV)

worin Y, Z und Ar wie vorstehend festgelegt sind und $R^4$ Wasserstoff ist oder eine Stickstoff-schützende Gruppe, die nach der Reaktion entfernt wird;

c) ein Verfahren zum Herstellen einer Verbindung der Formel (I), umfassend das Umsetzen einer Verbindung der Formel (VI):

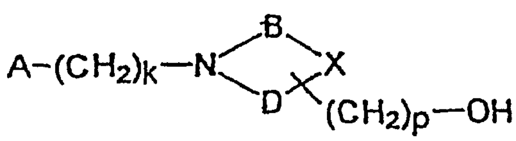

(VI)

worin A, B, D, X, k und p wie vorstehend festgelegt sind; mit einer Verbindung der Formel (XIV):

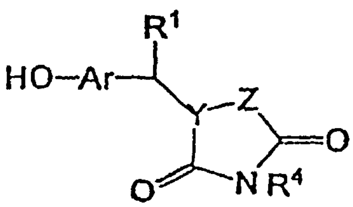

(XIV)

worin Y, Z und Ar wie vorstehend festgelegt sind und $R^4$ Wasserstoff ist oder eine Stickstoff-schützende Gruppe, die nach der Reaktion entfernt wird; oder

d) ein Verfahren zum Herstellen einer Verbindung der Formel (I), umfassend das Umsetzen einer Verbindung der Formel (XV):

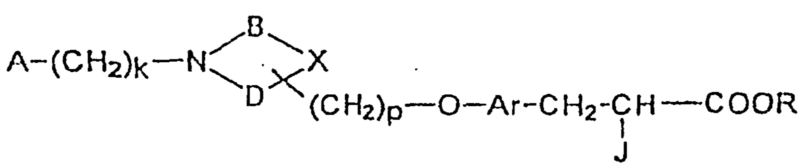

(XV)

worin A, B, D, X, k und p wie vorstehend festgelegt sind, J ein Halogenatom ist oder ein Hydroxy-Gruppe und R eine niedere Alkyl-Gruppe; mit Thioharnstoff, wenn J Halogenatom ist, oder Harnstoff, wenn J eine Hydroxy-Gruppe ist, gefolgt von einer Behandlung mit einer Säure.

**8.** Pharmazeutische Zusammensetzung, aufweisend eine Verbindung der Formel (1), wie in einem der Ansprüche 1 bis 4 festgelegt wurde, und einen pharmazeutisch duldbaren Träger, Streckmittel, Vehikel oder Solvat.

**9.** Pharmazeutische Zusammensetzung nach Anspruch 8 in Form einer Tablette, einer Kapsel, eines Pulvers, Sirups, einer Lösung oder Suspension.

**10.** Verwendung einer Verbindung nach einem der Ansprüche 1 bis 4 für die Herstellung eines Medikaments zum Vorbeugen oder Behandeln von Diabetes, hervorgerufen durch Insulinresistenz, beeinträchtigter Glucosetoleranz oder Komplikationen des Diabetes, wobei der Diabetes durch Insulinresistenz oder beeinträchtigte Glucosetoleranz hervorgerufen ist.

**11.** Verwendung nach Anspruch 10, wobei die Komplikation in Dyslipidämie, Hypertension, koronare Herzerkrankung, Herz-Kreislauf-Erkrankungen, Atherosklerose, Obesitas, Psoriasis, polyzystisches Ovar-Syndrom (PCOS), Nierenerkrankungen, diabetische Nephropathie, Glomerulonephritis, Glomerularsklerose, nephrotisches Syndrom, hypertensive Nephrosklerose, Endstadium-Nierenerkrankung, Mikroalbuminurie oder Essstörungen besteht.

**12.** Verwendung einer Verbindung nach einem der Ansprüche 1 bis 4 zum Verringern des Blutzuckers, der Triglyceride und freien Fettsäuren im Blut.

**13.** Verwendung einer Verbindung nach einem der Ansprüche 1 bis 4 zur Herstellung eines Medikaments zum Verringern des Blutzuckers, der Triglyceride und freien Fettsäuren im Blut.

**Revendications**

**1.** Composé de formule générale (I)

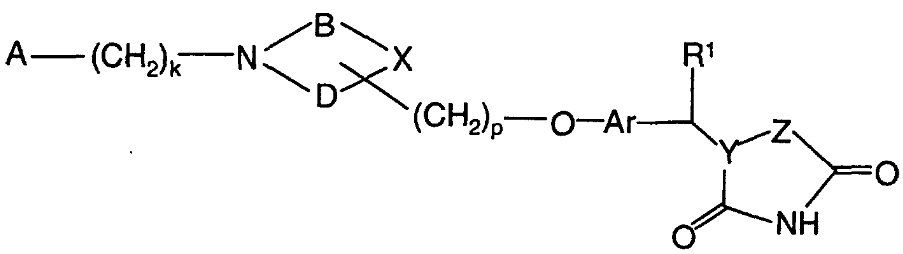

(I)

ses formes tautomères, ses stéréoisomères, ses formes polymorphes, ses sels pharmaceutiquement acceptables ou ses solvates pharmaceutiquement acceptables, où A représente le dihydrobenzofuranyle, le benzofuranyle, le dihydrobenzopyranyle ou l'indolyle, le groupe représenté par A pouvant être substitué par un groupe hydroxy, alkyle en ($C_1$-$C_6$) ou alcoxy en ($C_1$-$C_6$), ledit groupe alcoxy pouvant lui-même être substitué par un groupe aryle ; B représente un groupe de liaison non substitué entre N et X et B contient de 1 à 4 atomes de carbone ; D représente une liaison ou D contient de 1 à 4 atomes de carbone ; X représente $CH_2$, NH ou le soufre ; l'hétérocycle comprenant N, B, X et D étant un seul cycle saturé non substitué à cinq ou six éléments; Ar représente le phénylène ou le naphtylène ; $R^1$ représente un atome d'hydrogène ou forme une liaison conjointement avec le groupe adjacent Y ; Y représente un atome d'azote ou un groupe $CR^2$ où $R^2$ forme une liaison conjointement avec $R^1$ ; Z représente un atome d'oxygène ou un atome de soufre lorsque Y est $CR^2$ et Z représente un atome d'oxygène lorsque Y est un atome d'azote ; k est un nombre entier de 1 à 4 et p est un nombre entier de 0 à 1.

**2.** Composé selon la revendication 1, dans lequel k représente un nombre entier égal à 1 ou à 2.

**3.** Composé selon l'une quelconque des revendications 1 à 7, dans lequel l'hétérocycle comprenant Y et Z est un groupe 2,4-dioxothiazolidin-5-yle.

**4.** Composé selon la revendication 1, qui est sélectionné dans le groupe constitué par les composés suivants :

5-[4-[N-(6-benzyloxy-2,5,7,8-tétraméthylchroman-2-ylméthyl)-(2S)-pyrrolidine-2-méthoxyl]phénylméthyl]thiazolidine-2,4-dione ;
5-[4-[N-(6-benzyloxy-2,5,7,8-tétraméthylchroman-2-ylméthyl)-(2R)pyrrolidine-2-méthoxyl]phénylméthyl]thiazolidine-2,4-dione et ses sels ;
sel de sodium de 5-[4-[N-(6-benzyloxy-2,5,7,8-tétraméthylchroman-2-ylméthyl) - (2S)pyrrolidine-2-méthoxy] phénylméthyl]thiazolidine-2,4-dione ;
sel d'acide maléique de 5-[4-[N-(6-benzyloxy-2,5,7,8-tétraméthylchroman-2-ylméthyl)-(2S)pyrrolidine-2-méthoxy]phénylméthyl]thiazolidine-2,4-dione ;
sel chlorhydrate de 5-[4-[N-(6-benzyloxy-2,5,7,8-tétraméthylchroman-2-ylméthyl)-(2S)pyrrolidine-2-méthoxy] phénylméthyl]thiazolidine-2,4-dione ;
5-[4-[N-(6-hydroxy-2,5,7,8-tétraméthylchroman-2-yl-méthyl)-(2S)-pyrrolidine-2-méthoxy]phénylméthyl]thiazolidine-2,4-dione ;
5-[4-[N-(6-hydroxy-2,5,7,8-tétraméthylchroman-2-ylméthyl)-(2R)-pyrrolidine-2-méthoxy]phénylméthyl]thiazolidine-2,4-dione et ses sels ;
sel de sodium de 5-[4-[N-(6-hydroxy-2,5,7,8-tétraméthylchroman-2-ylméthyl)-(2S)-pyrrolidine-2-méthoxy] phénylméthyl]thiazolidine-2,4-dione ;
sel d'acide maléique de 5-[4-[N-[6-hydroxy-2,5,7,8-tétraméthylchroman-2-ylméthyl)-(2S)-pyrrolidine-2-méthoxy]phénylméthyl]thiazolidine-2,4-dione ;
sel chlorhydrate de 5-[4-[N-[6-hydroxy-2,5,7,8-tétraméthylchroman-2-ylméthyl)-(2S)-pyrrolidine-2-méthoxy] phénylméthyl]thiazolidine-2,4-dione ;
5-[4-[N-[6-hydroxy-2,5,7,8-tétraméthylchroman-2-yl-méthyl)-(2S)-pyrrolidine-2-méthoxy]phénylméthylène] thiazolidine-2,4-dione ;
5-[4-[N-[6-hydroxy-2,5,7,8-tétraméthylchroman-2-yl-méthyl]-(2R)-pyrrolidine-2-méthoxy]phénylméthylène] thiazolidine-2,4-dione et ses sels ;
5-[4-[N-[2,3-dihydro-5-benzyloxy-2,2,4,6,7-pentaméthylbenzofuran-3-ylméthyl]-(2S)-pyrrolidine-2-méthoxy] phénylméthylène]thiazolidine-2,4-dione ;
5-[4-[N-[2,3-dihydro-5-benzyloxy-2,2,4,6,7-penta-méthylbenzofuran-3-yl-méthyl]-(2R)-pyrrolidine-2-méthoxy]phénylméthylène]thiazolidine-2,4-dione et ses sels ;
sel d'acide maléique de 5-[4-[N-[2,3-dihydro-5-benzyloxy-2,2,4,6,7-pentaméthylbenzofuran-3-ylméthyl(2S)-pyrrolidine-2-méthoxy]phénylméthylène]thiazolidine-2,4-dione ;
sel chlorhydrate de 5-[4-[N-[2,3-dihydro-5-benzyloxy-2,2,4,6,7-pentaméthylbenzofuran-3-ylméthyl-(2S)-pyrrolidine-2-méthoxy]phénylméthylène]thiazolidine-2,4-dione ;
5-[4-[N-[2,3-dihydro-5-benzyloxy-2,2,4,6,7-pentaméthylbenzofuran-3-ylméthyl]-(2S)-pyrrolidine-2-méthoxy] phénylméthyl]thiazolidine-2,4-dione ;
5-[4-[N-[2,3-dihydro-5-benzyloxy-2,2,4,6,7-pentaméthylbenzofuran-3-ylméthyl]-(2R)-pyrrolidine-2-méthoxy phényl méthyl]thiazolidine-2,4-dione et ses sels ;
sel d'acide maléique de 5-[4-[N-[2,3-dihydro-5-benzyloxy-2,2,4,6,7-pentaméthylbenzofuran-3-yl-méthyl-(2S)-pyrrolidine-2-méthoxy]phénylméthyl]thiazolidine-2,4-dione ;
sel chlorhydrate de 5-[4-[N-[2,3-dihydro-5-benzyloxy-2,2,4,6,7-pentaméthylbenzofuran-3-ylméthyl]-(2S)-pyrrolidine-2-méthoxy]phénylméthyl]thiazolidine-2,4-dione ;
5-[4-[N-[2,3-dihydro-5-hydroxy-2,2,4,6,7-pentaméthylbenzofuran-3-ylméthyl-(2S)-pyrrolidine-2-méthoxy] phénylméthyl]thiazolidine-2,4-dione ;
5-[4-[N-[2,3-dihydro-5-hydroxy-2,2,4,6,7-pentaméthylbenzofuran-3-ylméthyl]-(2R)-pyrrolidine-2-méthoxy] phénylméthyl]thiazolidine-2,4-dione et ses sels ;
sel chlorhydrate de 5-[4-[N-[2,3-dihydro-5-hydroxy-2,2,4,6,7-pentaméthylbenzofuran-3-ylméthyl]-(2S)-pyrrolidine-2-méthoxy]phénylméthyl]thiazolidine-2,4-dione ;
5-[4-[N-[2,3-dihydro-5-hydroxy-2,2,4,6,7-pentaméthylbenzofuran-3-ylméthyl]-(2S)-pyrrolidine-2-méthoxy]-phénylméthylène]thiazolidine-2,4-dione ;
5-[4-[N-[2,3-dihydro-5-hydroxy-2,2,4,6,7-pentaméthylbenzofuran-3-ylméthyl-(2R)-pyrrolidine-2-méthoxy]-phénylméthylène]thiazolidine-2,4-dione et ses sels ;
5-[4-[N-[5-benzyloxy-2-méthylbenzofuran-3-ylméthyl]-(2S)-pyrrolidine-2-méthoxy]phényl méthylène]thiazolidine-2,4-dione ;
sel de sodium de 5-[4-[N-[5-benzyloxy-2-méthylbenzofuran-3-ylméthyl]-(2S)-pyrrolidine-2-méthoxy]-phénylméthylène]thiazolidine-2,4-dione, et
5-[4-[N-[(5-benzyloxy-2-méthylbenzofuran-3-ylméthyl]-(2S)-pyrrolidine-2-méthoxy]phénylméthyl]thiazolidine-2,4-dione ;

une forme polymorphe 1 de la 5-[4-[N-[(2RS)-6-benzyloxy-2,5,7,8-tétraméthylchroman-2-ylméthyl)]-(2S)-pyrrolidine-2-méthoxy]phénylméthylène] thiazolidine-2,4-dione **caractérisée par** un point de fusion d'environ 186,6°C et $^1$H-RMN ($CDCl_3$, 200 MHz) : δ 1,21, 1,26 (2s, 3H), 1,5 - 2,05 (m, 6H), 2,08 - 2,22 (6s, 9H), 2,35 - 3,15 (m, 6H), 3,4 (m, 1H), 3,8 (m, 1H), 4,0 (m, 1H), 4,7 (s, 2H), 6,95 (m, 2H), 7,3 - 7,6 (m, 7H), 7,8 (s, 1H) ;
une forme polymorphe 2 de la 5-[4-[N-[(2RS)-6-benzyloxy-2,5,7,8-tétraméthylchroman-2-ylméthyl)]-(2S)-pyrrolidine-2-méthoxy]phénylméthylène]thiazolidine-2,4-dione **caractérisée par** un point de fusion d'environ 88°C et $^1$H-RMN ($CDCl_3$, 200 MHz) : δ 1,21, 1,26 (2s, 3H), 1,5 - 2,05 (m, 6H), 2,08 - 2,22 (6s, 9H), 2,35 - 3,15 (m, 6H), 3,4 (m, 1H), 3,8 (m, 1H), 4,0 (m, 1H), 4,7 (s, 2H), 6,95 (m, 2H), 7,3 - 7,6 (m, 7H), 7,8 (s, 1H) ; et
une forme polymorphe 3 de la 5-[4-[N-[(2RS)-6-benzyloxy-2,5,7,8-tétraméthylchroman-2-ylméthyl)]-(2S)-pyrrolidine-2-méthoxy]phénylméthylène]thiazolidine-2,4-dione **caractérisée par** un point de fusion d'environ 86°C ; et $[\alpha]_D^{24}$ = 17,3 (c, 1,0, $CDCl_3$) et 200 MHz δ 1,21, 1,26 (2s, 3H), 1,5 - 2,05 (m, 6H), 2,08 - 2,22 (6s, 9H), 2,35 - 3,15 (m, 6H), 3,4 (m, 1H), 3,8 (m, 1H), 4,0 (m, 1H), 4,7 (s, 2H), 6,95 (m, 2H), 7,3 - 7,6 (m, 7H), 7,8 (s, 1H).

**5.** Produit intermédiaire de formule (III)

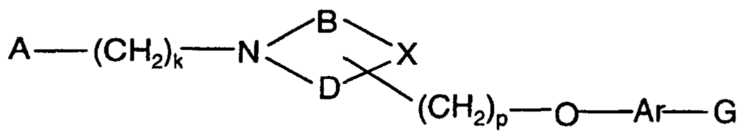

(III)

où A représente le dihydrobenzofuranyle, le benzofuranyle, le dihydrobenzopyranyle ou l'indolyle, le groupe représenté par A pouvant être substitué par un groupe hydroxy, alkyle en ($C_1$-$C_6$) ou alcoxy en ($C_1$-$C_6$), ledit groupe alcoxy pouvant lui-même être substitué par un groupe aryle ; B représente un groupe de liaison substitué ou non substitué entre N et X et B contient de 1 à 4 atomes de carbone ; D représente une liaison ou D contient de 1 à 4 atomes de carbone ; X représente $CH_2$ ou un hétéroatome sélectionné parmi l'azote ou le soufre, l'hétérocycle comprenant N, B, X et D étant un seul cycle saturé non substitué à cinq ou six éléments ; Ar représente le phénylène ou le naphtylène ; k est un nombre entier de 1 à 4 et p est un nombre entier de 0 à 1 et G représente -CHO, $-NO_2$, $-NH_2$, -CH=NHOH, $-CH_2NHOH$, $-CH_2N(OH)CONH_2$ ou $-CH_2CH$ (J) -COOR, où J représente un groupe hydroxy, ou un atome d'halogène et R représente H ou un groupe alkyle inférieur.

**6.** Procédé de préparation d'un produit intermédiaire de formule (III)

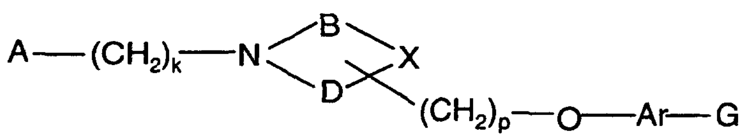

(III)

où A représente le dihydrobenzofuranyle, le benzofuranyle, le dihydrobenzopyranyle ou l'indolyle, le groupe représenté par A pouvant être substitué par un groupe hydroxy, alkyle en ($C_1$-$C_6$) ou alcoxy en ($C_1$-$C_6$) , ledit groupe alcoxy pouvant lui-même être substitué par un groupe aryle ; B contient de 1 à 4 atomes de carbone ; D représente une liaison ou D contient de 1 à 4 atomes de carbone ; X représente $CH_2$ ou un hétéroatome sélectionné parmi l'azote ou le soufre, l'hétérocycle comprenant N, B, X et D étant un seul cycle saturé non substitué à cinq ou six éléments ; Ar représente le phénylène ou le naphtylène ; k est un nombre entier de 1 à 4 et p est un nombre entier de 0 à 1 et G représente -CHO ou $-NO_2$, ledit procédé comprenant de :

a) faire réagir le composé de formule (IV)

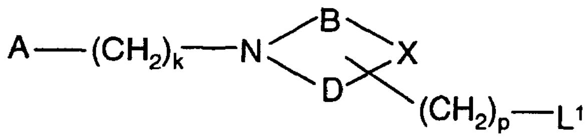

(IV)

où A, B, D, X, k et p sont tels que définis ci-dessus et $L^1$ est un atome d'halogène ou un groupe qui part, avec un composé de formule (V)

$$HO—Ar—G \quad (V)$$

où G est un groupe CHO ou $NO_2$ et Ar est tel que défini ci-dessus ;
b) faire réagir un composé de formule (VI)

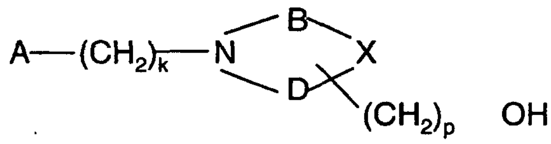

(VI)

où A, B, D, X, k et p sont tels que définis ci-dessus, avec un composé de formule (VII)

$$L_2—Ar—G \quad (VII)$$

où G est un groupe CHO ou $NO_2$ et Ar est tel que défini ci-dessus et $L^2$ représente un atome d'halogène ;
c) faire réagir un composé de formule (VI)

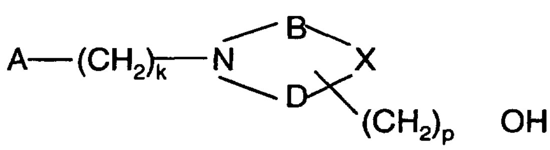

(VI)

où A, B, D, X, k et p sont tels que définis ci-dessus, avec un composé de formule (V)

$$HO—Ar—G \quad (V)$$

où G est un groupe CHO ou $NO_2$ et Ar est tel que défini ci-dessus ;
d) faire réagir un composé de formule (VIII)

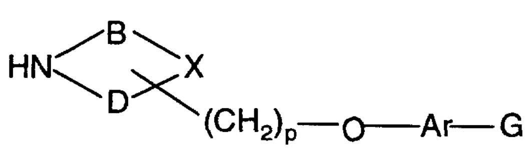

(VIII)

où B, D, X, Ar, k et p sont tels que définis ci-dessus, avec un composé de formule (IX)

$$A—(CH_2)_k—L^1 \qquad (IX)$$

où A et k sont tels que définis ci-dessus et $L^1$ est tel que défini en (a) ci-dessus.

**7.** Procédé de préparation de nouveaux dérivés d'azolidinedione de formule (I),

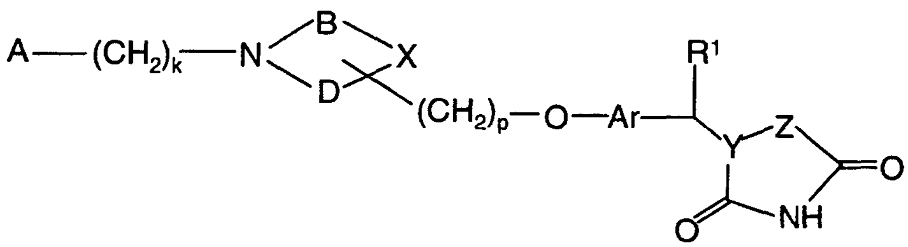

(I)

leurs formes tautomères, leurs stéréoisomères, leurs formes polymorphes, leurs sels pharmaceutiquement acceptables et leurs solvates pharmaceutiquement acceptables selon la revendication 1, où $R^1$ représente un hydrogène, Y représente un groupe CH et Z représente un atome de soufre ou d'oxygène et où A représente le dihydrobenzofuranyle, le benzofuranyle, le dihydrobenzopyranyle ou l'indolyle, le groupe représenté par A pouvant être substitué par un groupe hydroxy, alkyle en ($C_1$-$C_6$) ou alcoxy en ($C_1$-$C_6$), ledit groupe alcoxy pouvant lui-même être substitué par un groupe aryle ; B contient de 1 à 4 atomes de carbone ; D représente une liaison ou D contient de 1 à 4 atomes de carbone ; X représente $CH_2$ ou un hétéroatome sélectionné parmi l'azote ou le soufre, l'hétérocycle comprenant N, B, X et D étant un seul cycle saturé non substitué à cinq ou six éléments ; Ar représente le phénylène ou le naphtylène ; k est un nombre entier de 1 à 4 et p est un nombre entier de 0 à 1, comprenant de :

a) i) faire réagir un composé de formule (III)

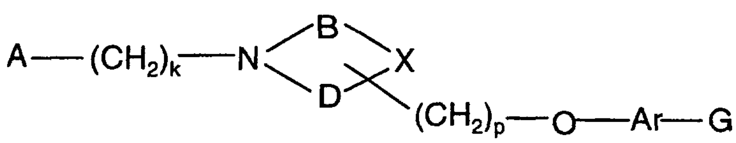

(III)

où G est un groupe CHO, avec de la 2,4-thiazolidinedione ou de la 2,4-oxazolidinedione, pour produire un composé de formule (XII)

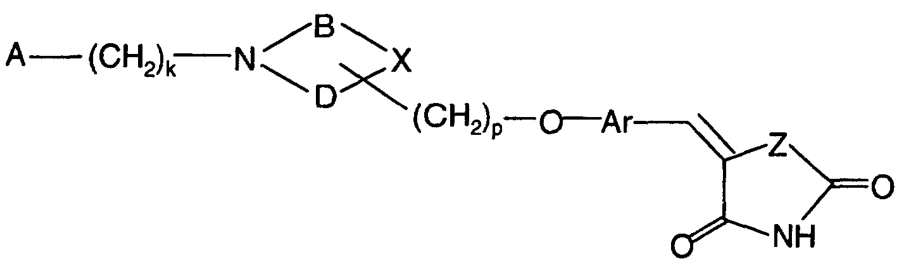

(XII)

où A, B, D, X, Ar, k, p et Z sont tels que définis ci-dessus, et d'éliminer l'eau formée pendant la réaction ;
(ii) réduire le composé de formule (XII) obtenu à l'étape (i) ci-dessus pour obtenir un composé de formule (XIII)

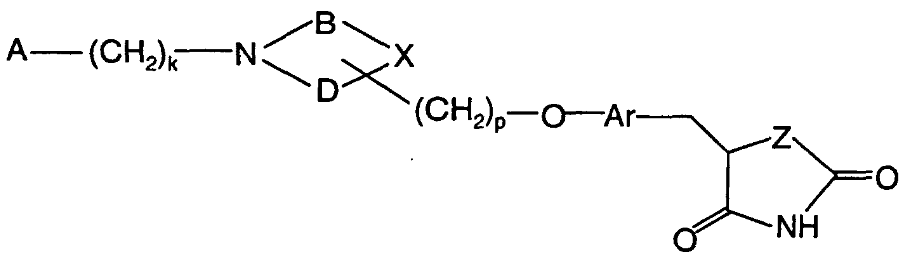

(XIII)

où A, B, D, X, Ar, k, p et Z sont tels que définis ci-dessus ;

b) procédé de préparation du composé de formule (I), comprenant de faire réagir un composé de formule (IV)

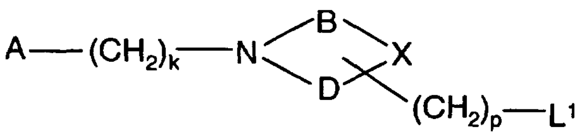

(IV)

où A, B, D, X, k et p sont tels que définis ci-dessus et $L^1$ est un atome d'halogène ou un groupe mobile, avec un composé de formule (XIV)

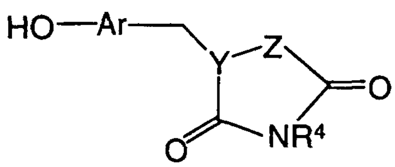

(XIV)

où Y, Z et Ar sont tels que définis ci-dessus et $R^4$ est un hydrogène ou un groupe protecteur d'azote qui est séparé après la réaction ;

c) procédé de préparation du composé de formule (I), comprenant de faire réagir un composé de formule (VI)

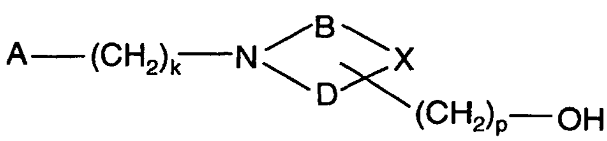

(VI)

où A, B, D, X, k et p sont tels que définis ci-dessus, avec un composé de formule (XIV)

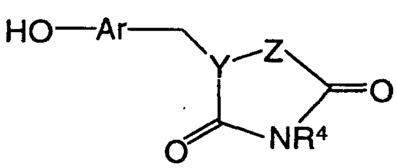

(XIV)

où Y, Z et Ar sont tels que définis ci-dessus et $R^4$ est un hydrogène ou un groupe protecteur d'azote, qui est séparé après la réaction ; ou

d) procédé de préparation du composé de formule (I), comprenant de faire réagir un composé de formule (XV)

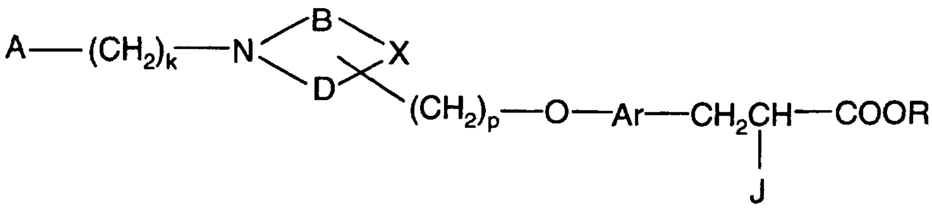

(XV)

où A, B, D, X, k et p sont tels que définis ci-dessus, J est un atome d'halogène ou un groupe hydroxy et R est un groupe alkyle inférieur, avec de la thiourée lorsque J est un atome d'halogène, ou de l'urée lorsque J est un groupe hydroxy, la réaction étant suivie d'un traitement avec un acide.

**8.** Composition pharmaceutique qui comprend un composé de formule (I) tel que défini dans l'une quelconque des revendications 1 à 4 et un véhicule, diluant, excipient ou solvate pharmaceutiquement acceptable.

**9.** Composition pharmaceutique selon la revendication 8, présentée sous forme de comprimé, de capsule, de poudre, de sirop, de solution ou de suspension.

**10.** Utilisation d'un composé selon l'une quelconque des revendications 1 à 4, pour la fabrication d'un médicament destiné à prévenir ou à traiter le diabète dû à une résistance à l'insuline ou une tolérance altérée au glucose ou des complications du diabète, ledit diabète étant dû à une résistance à l'insuline ou une tolérance altérée au glucose.

**11.** Utilisation selon la revendication 10, dans laquelle la complication est la dyslipidémie, l'hypertension, la maladie coronarienne, les troubles cardio-vasculaires, l'athérosclérose, l'obésité, le psoriasis, le syndrome des ovaires polykystiques (PCOS), les maladies rénales, la néphropathie diabétique, la glomérulonéphrite, la glomérulosclérose, le syndrome néphrotique, la néphrosclérose hypertensive, la maladie rénale de stade terminal, la microalbuminurie ou des troubles alimentaires.

**12.** Utilisation d'un composé selon l'une quelconque des revendications 1 à 4, pour diminuer le glucose sanguin, les triglycérides et les acides gras libres dans le sang.

**13.** Utilisation d'un composé selon l'une quelconque des revendications 1 à 4, pour la fabrication d'un médicament destiné à diminuer le glucose sanguin, les triglycérides et les acides gras libres dans le sang.